# EUROPEAN PATENT APPLICATION

(11) **EP 4 682 166 A1**
(43) Date of publication of application: **21.01.2026**
(21) Application number: 24788103.0
(22) Date of filing: 10.04.2024
(51) Int. Cl.: C07K 16/18, A61K 39/395

(54) **ANTI-NMI MONOCLONAL ANTIBODY OR ANTIGEN-BINDING FRAGMENT THEREOF, AND USE THEREOF**

(30) Priority: 10.04.2023 CN 202310383287
(71) Applicant: Guangzhou Enmai Biotechnology Co., Ltd, Guangzhou, Guangdong 510535 (CN)
(72) Inventor: LIU, Xiaotong, Guangdong 510535 (CN)
(74) Representative: Clark, Claudia
(86) International application number: PCT/CN2024/087014
(87) International publication number: WO 2024/213001

(57) **Abstract**

Provided are an anti-NMI monoclonal antibody or an antigen-binding fragment thereof, and the use thereof. The antibody or the antigen-binding fragment thereof has good binding activity to the NMI protein, has high affinity to the NMI protein, can be used for detecting the presence or level of NMI protein in a sample and performing diagnosis, auxiliary diagnosis or prognostic evaluation of diseases or conditions related to the abnormal high level and/or the activity of NMI, and is used for removing the NMI protein in the body fluid and preventing and treating diseases or conditions related to the abnormal high level and/or the activity of NMI.

## Description

### TECHNICAL FIELD

The present invention belongs to the field of antibody technology, and specifically relates to an anti-NMI monoclonal antibody or an antigen-binding fragment thereof, and a use thereof.

### BACKGROUND

When tissues are infected or damaged, a class of molecules called damage associated molecular patterns (DAMPs) are released. Such molecules are able to modulate the immune response and mobilize the inflammatory response, which in turn fight infection and promote tissue repair. However, when DAMPs cause an excessive or sustained inflammatory response, they may result in tissue and organ damages, leading to a variety of acute and chronic inflammatory diseases, including sepsis, pneumonia, rheumatoid arthritis, and multiple sclerosis.

In recent years, the inventors have discovered a novel molecule family of protein DAMPs (Damage associated molecular pattern): the IFP35 family proteins, which contains two homologous protein molecules, IFP35 and NMI (N-myc Interacting protein). In the absence of infection, damage, or interferon induction, intracellular NMI expression is very low. When the organism is infected, damaged, or induced by interferon, the expression of such DAMP molecules is significantly elevated and can be secreted into extracellular, blood, urine, and other body fluids. Such DAMP family of inflammatory factors is released by a variety of immune cells (macrophages, etc.) and has a pro-inflammatory effect, promoting the expression of pro-inflammatory factors, such as TNF and IL6, in a variety of disease models, e.g., *Salmonella* infections, LPS-induced septicemia, and APAP (acetaminophen)-induced liver damage disease models. The inventors also found that the release of such DAMP is much earlier (1 hour vs >3 hours) than the release of HMGB1 (a DAMP molecule identified earlier) in the case of infection induction. The study reveals that the receptor for such novel DAMP molecules on the cell surface can be Toll-like receptor 4 (TLR4), which promotes the release of large amounts of pro-inflammatory cytokines from immune cells, such as macrophages, and other cells (epithelial cells, neuronal cells, neuroglia, etc.) by binding to TLR4 to activate the intracellular NF-κB signaling pathway, thus promoting the elevation of the immune response. If such DAMP molecules are oversecreted , sepsis, some chronic inflammatory diseases or autoimmune diseases (e.g. arthritis, inflammatory bowel disease, psoriasis, various types of encephalitis, hepatitis, multiple sclerosis, Alzheimer's disease, lupus erythematosus, pneumonia, nephritis, neurogenic inflammation, etc.) are induced. The occurrence and development of these inflammatory diseases are closely related to the expression levels and secretion of such DAMPs (Zhikai Xiahou, Xiangli Wang, Juan Shen, Xiaoxiao Zhu, Feng Xu, Rong Hu, Deyin Guo, Henan Li, Yong Tian, Yingfang Liu*, and Huanhuan Liang* NMI and IFP35 serve as proinflammatory DAMPs during cellular infection and injury. Nat Commun. 2017; 8: 950. published online 2017 Oct 16. doi:. 10.1038/s41467-017-00930-9.; Yang Yu, Na Xu Qi Cheng, Fei Deng, Meiqin Liu, Airu Zhu, Yuan-Qin Min, Dan Zhu, Wenbo Huang, Xu Feng, Xizhong Jing, Ying Chen, Daoyuan Yue, Yawei Fan, Chang Shu, Qing Guan, Zifeng Yang, Jincun Zhao, Wenjun Song, Deyin Guo, Huanliang Liu, Jindong Zhao, Ping Lan*, Zhengli Shi*, Yingfang Liu1*, Xiaoping Chen* and Huanhuan Liang*. and Huanhuan Liang*. IFP35 as a promising biomarker and therapeutic target for the2 syndromes induced by SARS-CoV-2 or influenza virus. Cell Reports. 2021 Dec 21;37(12).110126. Cell Reports. doi: 10. 10 1 6/j.celrep.2021.110126; Xizhong Jing, Yongjie Yao, Danning Wu, Hao Hong, Feng Xu, Na Xu, Yingfang Liu*, Huanhuan Liang*. IFP35 family proteins promote neuroinflammation and multiple sclerosis. (Proc Natl Acad Sci USA. 2021 Aug 10;118(32):e2102642118. doi: 10.1073/pnas.2102642118.PMID: 34362845).

The development of a test kit for NMI is of great value in detecting the status and progress of various acute and chronic inflammatory diseases and autoimmune diseases caused by infections, determining the effectiveness of diagnosis and treatment, and determining the prognosis of the diseases, and other clinical testing needs, and the related basic medical research is also in urgent need of similar test reagents. Therefore, it is necessary to develop an anti-NMI monoclonal antibody or an antigen-binding fragment thereof.

### SUMMARY

An objective of a first aspect of the present invention is to provide an anti-NMI antibody or antigen-binding fragment thereof.

An objective of a second aspect of the present invention is to provide a recombinant protein.

An objective of a third aspect of the present invention is to provide a biological material related to the antibody or the antigen-binding fragment thereof of the first aspect or the recombinant protein of the second aspect of the present invention.

An objective of a fourth aspect of the present invention is to provide a conjugate comprising the antibody or the antigen-binding fragment thereof of the first aspect of the present invention.

An objective of a fifth aspect of the present invention is to provide use of the antibody or antigen-binding fragment thereof of the first aspect, the recombinant protein of the second aspect, the biological material of the third aspect, and/or the conjugate of the fourth aspect of the present invention in the preparation of a product.

An objective of a sixth aspect of the present invention is to provide a kit comprising the antibody or an antigen-binding fragment thereof of the first aspect of the present invention and/or the conjugate of the fourth aspect of the present invention.

An objective of a seventh aspect of the present invention is to provide a drug comprising the antibody or antigen-binding fragment thereof of the first aspect of the present invention and/or the conjugate of the fourth aspect of the present invention.

An objective of an eighth aspect of the present invention is to provide a method for preparing the antibody or antigen-binding fragment thereof of the first aspect of the present invention.

An objective of a ninth aspect of the present invention is to provide a method for detecting the presence or level of NMI.

An objective of a tenth aspect of the present invention is to provide a method for diagnosing, auxiliary diagnosing, or prognostic evaluating a diseases or condition related to abnormal high level and/or activity of NMI.

An objective of an eleventh aspect of the present invention is to provide a method for removing NMI protein from body fluids.

Technical solutions adopted in the present invention to achieve the above objectives are as follows.

The first aspect of the present invention provides an anti-NMI antibody or antigen-binding fragment thereof, wherein the antibody or antigen-binding fragment thereof is 94C4, B-8, 240B10-1, 98G10, or 39B4; the antibody or antigen-binding fragment thereof comprises a heavy chain variable region and a light chain variable region:
the heavy chain variable region of the 94C4 is any one of a1) to a2) (preferably a1):
   a1) which comprises CDR-H1, CDR-H2, and CDR-H3 of the heavy chain variable region with an amino acid sequence as shown in SEQ ID NO: 4;
   a2) which comprises CDR-H1, CDR-H2, and CDR-H3 of the heavy chain variable region with an amino acid sequence derived by substituting 1, 2, 3, 4, or 5 (preferably 1 or 2) amino acids of SEQ ID NO: 4 while having the same function as the protein as shown in SEQ ID NO: 4;
the amino acid that is substituted is an amino acid in CDR, and the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the light chain variable region of the 94C4 is any one of b1) to b2) (preferably b1):
   b1) which comprises CDR-L1, CDR-L2, and CDR-L3 of the light chain variable region with an amino acid sequence as shown in SEQ ID NO: 21;
   b2) which comprises CDR-L1, CDR-L2, and CDR-L3 of the light chain variable region with an amino acid sequence derived by substituting 1, 2, 3, 4, or 5 (preferably 1 or 2) amino acids of SEQ ID NO: 21 while having the same function as the protein as shown in SEQ ID NO: 21;
the amino acid that is substituted is an amino acid in CDR, and the amino acid that is substituted is selected from: an amino acid other than Y29, N30, Y32, S34, L46, Y49, R50, S52, T53, L54, S56, S94, A95, and D97 in SEQ ID NO: 21;
the heavy chain variable region of the B-8 is any one of c1) to c2) (preferably c1):
   c1) which comprises CDR-H1, CDR-H2, and CDR-H3 of the heavy chain variable region with an amino acid sequence as shown in SEQ ID NO: 32;
   c2) which comprises CDR-H1, CDR-H2, and CDR-H3 of the heavy chain variable region with an amino acid sequence derived by substituting 1, 2, 3, 4, or 5 (preferably 1 or 2) amino acids of SEQ ID NO: 32 while having the same function as the protein as shown in SEQ ID NO: 32;
the amino acid that is substituted is an amino acid in CDR, and the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the light chain variable region of the B-8 is any one of d1) to d2) (preferably d1):
   d1) which comprises CDR-L1, CDR-L2, and CDR-L3 of the light chain variable region with an amino acid sequence as shown in SEQ ID NO: 49;
   d2) which comprises CDR-L1, CDR-L2, and CDR-L3 of the light chain variable region with an amino acid sequence derived by substituting 1, 2, 3, 4, or 5 (preferably 1 or 2) amino acids of SEQ ID NO: 49 while having the same function as the protein as shown in SEQ ID NO: 49;
the amino acid that is substituted is an amino acid in CDR, and the amino acid that is substituted is selected from: an amino acid other than Y30, T31, N32, L92, Y93, S94, R95, 196, A97, D98, and R99 in SEQ ID NO: 49;
the heavy chain variable region of the 240B10-1 is any one of e1) to e2) (preferably e1):
   e1) which comprises CDR-H1, CDR-H2, and CDR-H3 of the heavy chain variable region with an amino acid sequence as shown in SEQ ID NO: 60;
   e2) which comprises CDR-H1, CDR-H2, and CDR-H3 of the heavy chain variable region with an amino acid sequence derived by substituting 1, 2, 3, 4, or 5 (preferably 1 or 2) amino acids of SEQ ID NO: 60 while having the same function as the protein as shown in SEQ ID NO: 60;
the amino acid that is substituted is an amino acid in CDR, and the amino acid that is substituted is selected from: an amino acid other than D27, L28, S29, S30, Y31, T32, S51, G52, G53, and G54 in SEQ ID NO: 60;
the light chain variable region of the 240B 10-1 is any one of f1) to f2) (preferably f1):
   f1) which comprises CDR-L1, CDR-L2, and CDR-L3 of the light chain variable region with an amino acid sequence as shown in SEQ ID NO: 77;
   f2) which comprises CDR-L1, CDR-L2, and CDR-L3 of the light chain variable region with an amino acid sequence derived by substituting 1, 2, 3, 4, or 5 (preferably 1 or 2) amino acids of SEQ ID NO: 77 while having the same function as the protein as shown in SEQ ID NO: 77;
the amino acid that is substituted is an amino acid in CDR, and the amino acid that is substituted is selected from: an amino acid other than Y29, N30, D32, Y33, S51, K54, C75, and Y76 in SEQ ID NO: 77;
the heavy chain variable region of the 98G10 is any one of g1) to g2) (preferably g1):
   g1) which comprises CDR-H1, CDR-H2, and CDR-H3 of the heavy chain variable region with an amino acid sequence as shown in SEQ ID NO: 88;
   g2) which comprises CDR-H1, CDR-H2, and CDR-H3 of the heavy chain variable region with an amino acid sequence derived by substituting 1, 2, 3, 4, or 5 (preferably 1 or 2) amino acids of SEQ ID NO: 88 while having the same function as the protein as shown in SEQ ID NO: 88;
the amino acid that is substituted is an amino acid in CDR, and the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the light chain variable region of the 98G10 is any one of h1) to h2) (preferably h1):
   h1) which comprises CDR-L1, CDR-L2, and CDR-L3 of the light chain variable region with an amino acid sequence as shown in SEQ ID NO: 105;
   h2) which comprises CDR-L1, CDR-L2, and CDR-L3 of the light chain variable region with an amino acid sequence derived by substituting 1, 2, 3, 4, or 5 (preferably 1 or 2) amino acids of SEQ ID NO: 105 while having the same function as the protein as shown in SEQ ID NO: 105;
the amino acid that is substituted is an amino acid in CDR, and the amino acid that is substituted is selected from: an amino acid other than K53 in SEQ ID NO: 105;
the heavy chain variable region of the 39B4 is any one of i1) to i2) (preferably i1):
   i1) which comprises CDR-H1, CDR-H2, and CDR-H3 of the heavy chain variable region with an amino acid sequence as shown in SEQ ID NO: 116;
   i2) which comprises CDR-H1, CDR-H2, and CDR-H3 of the heavy chain variable region with an amino acid sequence derived by substituting 1, 2, 3, 4, or 5 (preferably 1 or 2) amino acids of SEQ ID NO: 116 while having the same function as the protein as shown in SEQ ID NO: 116;
the amino acid that is substituted is an amino acid in CDR, and the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the light chain variable region of the 39B4 is any one of j1) to j2) (preferably j1):
   j1) which comprises CDR-L1, CDR-L2, and CDR-L3 of the light chain variable region with an amino acid sequence as shown in SEQ ID NO: 132;
   j2) which comprises CDR-L1, CDR-L2, and CDR-L3 of the light chain variable region with an amino acid sequence derived by substituting 1, 2, 3, 4, or 5 (preferably 1 or 2) amino acids of SEQ ID NO: 132 while having the same function as the protein as shown in SEQ ID NO: 132;
the amino acid that is substituted is an amino acid in CDR, and the amino acid that is substituted is selected from: an amino acid other than S28, N31, Y93, and S96 in SEQ ID NO: 132.

Preferably, the CDRs mentioned above refer to CDR-1, CDR-2 and/or CDR-3, taking the CDRs in the heavy chain variable region of 94C4 as an example: they refer to CDR-H1, CDR-H2 and/or CDR-H3; take the CDRs in the light chain variable region of 94C4 as an example: CDR-L1, CDR-L2 and/or CDR-L3.

Preferably, the two heavy chain variable regions and the two light chain variable regions of the above antibody or antigen-binding fragment thereof (94C4, B-8, 240B10-1, 98G10 or 39B4) can be arbitrarily combined: taking 94C4 as an example, the combinations of the heavy chain variable region and the light chain variable region can be: a1) and b1), a1) and b2) and so on.

Preferably, the combinations of the heavy chain variable region and the light chain variable region of the antibody or the antigen-binding fragment thereof (94C4, B-8, 240B10-1, 98G10 or 39B4) as mentioned above are preferably as follows in order: a1) and b1); c1) and d1); e1) and f1); g1) and h1); and i1) and j1).

Preferably, the above-mentioned amino acid sequences after substitution are related to the number and position of the amino acids that are substituted, as well as the number of amino acids in the CDRs, wherein the amino acid sequence after substitution also corresponds to the selection of amino acids after substitution. Thus, there are numerous amino acid sequences after substitution, which are all within the scope of protection of the present application, and hereinafter, each of the amino acid sequences will not be enumerated one by one.

The applicant seeking for protection of the amino acid sequence in which the above substitutions occur relative to a1), b1), c1), d1), e1), f1), g1), h1), i1), and j1) is due to the following: the applicant has found that the amino acids bound to/recognized by the antigen NMI are some amino acids in the CDRs of a1), b1), c1), d1), e1), f1), g1), h1), i1), and j1) (herein referred to as key amino acids, corresponding to sites other than those where substitutions occur in a2), b2), c2), d2), e2), f2), g2), h2), i2), and j2), respectively, e.g., F26, S29, S30, N31, A32, C49, 150, S51, S52, S54, S60, Y62, R100, D101, G102, D104, and L105 in a2)), therefore, changes (substitutions) to amino acids other than the key amino acids do not affect the function of the antibody or antigen-binding fragment thereof.

An anti-NMI antibody or antigen-binding fragment thereof, wherein the antibody or antigen-binding fragment thereof is 94C4, B-8, 240B10-1, 98G10, or 39B4; the antibody or antigen-binding fragment thereof comprises a heavy chain variable region and a light chain variable region:
the 94C4 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
   a111) SEQ ID NO: 5;
   a112) an amino acid sequence derived by substituting 1, 2, or 3 (preferably 1) amino acids of SEQ ID NO: 5 while having the same function as the protein as shown in SEQ ID NO: 5, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
   a211) SEQ ID NO: 6;
   a212) an amino acid sequence derived by substituting 1, 2, or 3 (preferably 1) amino acids of SEQ ID NO: 6 while having the same function as the protein as shown in SEQ ID NO: 6, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
   a311) SEQ ID NO: 7;
   a312) an amino acid sequence derived by substituting 1, 2, or 3 (preferably 1) amino acids of SEQ ID NO: 7 while having the same function as the protein as shown in SEQ ID NO: 7, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
   a411) SEQ ID NO: 22;
   a412) an amino acid sequence derived by substituting 1, 2, or 3 (preferably 1) amino acids of SEQ ID NO: 22 while having the same function as the protein as shown in SEQ ID NO: 22, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
   a511) SEQ ID NO: 23;
   a512) an amino acid sequence derived by substituting 1, 2, or 3 (preferably 1) amino acids of SEQ ID NO: 23 while having the same function as the protein as shown in SEQ ID NO: 23, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
   a611) SEQ ID NO: 24;
   a612) an amino acid sequence derived by substituting 1, 2, or 3 (preferably 1) amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the IMGT Definition Scheme,
the CDR-H1 is any one of a111) to a112):
   a111) SEQ ID NO: 8;
   a112) an amino acid sequence derived by substituting 1, 2, or 3 (preferably 1) amino acids of SEQ ID NO: 8 while having the same function as the protein as shown in SEQ ID NO: 8, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
   a211) SEQ ID NO: 9;
   a212) an amino acid sequence derived by substituting 1, 2, or 3 (preferably 1) amino acids of SEQ ID NO: 9 while having the same function as the protein as shown in SEQ ID NO: 9, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
   a311) SEQ ID NO: 10;
   a312) an amino acid sequence derived by substituting 1, 2, or 3 (preferably 1) amino acids of SEQ ID NO: 10 while having the same function as the protein as shown in SEQ ID NO: 10, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
   a411) SEQ ID NO: 25;
   a412) an amino acid sequence derived by substituting 1, 2, or 3 (preferably 1) amino acids of SEQ ID NO: 25 while having the same function as the protein as shown in SEQ ID NO: 25, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
   a511) RVS;
   a512) an amino acid sequence derived by substituting 1 or 2 (preferably 1) amino acids of RVS while having the same function as RVS, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
   a611) SEQ ID NO: 24;
   a612) an amino acid sequence derived by substituting 1, 2, or 3 (preferably 1) amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the Chothia Definition Scheme,
the CDR-H1 is any one of a111) to a112):
   a111) SEQ ID NO: 11;
   a112) an amino acid sequence derived by substituting 1, 2, or 3 (preferably 1) amino acids of SEQ ID NO: 11 while having the same function as the protein as shown in SEQ ID NO: 11, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
   a211) SEQ ID NO: 12;
   a212) an amino acid sequence derived by substituting 1, 2, or 3 (preferably 1) amino acids of SEQ ID NO: 12 while having the same function as the protein as shown in SEQ ID NO: 12, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
   a311) SEQ ID NO: 7;
   a312) an amino acid sequence derived by substituting 1, 2, or 3 (preferably 1) amino acids of SEQ ID NO: 7 while having the same function as the protein as shown in SEQ ID NO: 7, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
   a411) SEQ ID NO: 22;
   a412) an amino acid sequence derived by substituting 1, 2, or 3 (preferably 1) amino acids of SEQ ID NO: 22 while having the same function as the protein as shown in SEQ ID NO: 22, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
   a511) SEQ ID NO: 23;
   a512) an amino acid sequence derived by substituting 1, 2, or 3 (preferably 1) amino acids of SEQ ID NO: 23 while having the same function as the protein as shown in SEQ ID NO: 23, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
   a611) SEQ ID NO: 24;
   a612) an amino acid sequence derived by substituting 1, 2, or 3 (preferably 1) amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the Contact Definition Scheme,
the CDR-H1 is any one of a111) to a112):
   a111) SEQ ID NO: 13;
   a112) an amino acid sequence derived by substituting 1, 2, or 3 (preferably 1) amino acids of SEQ ID NO: 13 while having the same function as the protein as shown in SEQ ID NO: 13, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
   a211) SEQ ID NO: 14;
   a212) an amino acid sequence derived by substituting 1, 2, or 3 (preferably 1) amino acids of SEQ ID NO: 14 while having the same function as the protein as shown in SEQ ID NO: 14, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
   a311) SEQ ID NO: 15;
   a312) an amino acid sequence derived by substituting 1, 2, or 3 (preferably 1) amino acids of SEQ ID NO: 15 while having the same function as the protein as shown in SEQ ID NO: 15, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
   a411) SEQ ID NO: 26;
   a412) an amino acid sequence derived by substituting 1, 2, or 3 (preferably 1) amino acids of SEQ ID NO: 26 while having the same function as the protein as shown in SEQ ID NO: 26, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
   a511) SEQ ID NO: 27;
   a512) an amino acid sequence derived by substituting 1, 2, or 3 (preferably 1) amino acids of SEQ ID NO: 27 while having the same function as the protein as shown in SEQ ID NO: 27, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
   a611) SEQ ID NO: 28;
   a612) an amino acid sequence derived by substituting 1, 2, or 3 (preferably 1) amino acids of SEQ ID NO: 28 while having the same function as the protein as shown in SEQ ID NO: 28, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the Abm Definition Scheme,
the CDR-H1 is any one of a111) to a112):
   a111) SEQ ID NO: 16;
   a112) an amino acid sequence derived by substituting 1, 2, or 3 (preferably 1) amino acids of SEQ ID NO: 16 while having the same function as the protein as shown in SEQ ID NO: 16, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
   a211) SEQ ID NO: 17;
   a212) an amino acid sequence derived by substituting 1, 2, or 3 (preferably 1) amino acids of SEQ ID NO: 17 while having the same function as the protein as shown in SEQ ID NO: 17, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
   a311) SEQ ID NO: 7;
   a312) an amino acid sequence derived by substituting 1, 2, or 3 (preferably 1) amino acids of SEQ ID NO: 7 while having the same function as the protein as shown in SEQ ID NO: 7, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
   a411) SEQ ID NO: 22;
   a412) an amino acid sequence derived by substituting 1, 2, or 3 (preferably 1) amino acids of SEQ ID NO: 22 while having the same function as the protein as shown in SEQ ID NO: 22, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
   a511) SEQ ID NO: 23;
   a512) an amino acid sequence derived by substituting 1, 2, or 3 (preferably 1) amino acids of SEQ ID NO: 23 while having the same function as the protein as shown in SEQ ID NO: 23, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
   a611) SEQ ID NO: 24;
   a612) an amino acid sequence derived by substituting 1, 2, or 3 (preferably 1) amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
the B-8 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
   a111) SEQ ID NO: 33;
   a112) an amino acid sequence derived by substituting 1, 2, or 3 (preferably 1) amino acids of SEQ ID NO: 33 while having the same function as the protein as shown in SEQ ID NO: 33, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from Y33 in SEQ ID NO: 32);
the CDR-H2 is any one of a211) to a212):
   a211) SEQ ID NO: 34;
   a212) an amino acid sequence derived by substituting 1, 2, or 3 (preferably 1) amino acids of SEQ ID NO: 34 while having the same function as the protein as shown in SEQ ID NO: 34, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from Y61 in SEQ ID NO: 32);
the CDR-H3 is any one of a311) to a312):
   a311) SEQ ID NO: 35;
   a312) an amino acid sequence derived by substituting 1, 2, or 3 (preferably 1) amino acids of SEQ ID NO: 35 while having the same function as the protein as shown in SEQ ID NO: 35, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from F111 in SEQ ID NO: 32);
the CDR-L1 is any one of a411) to a412):
   a411) SEQ ID NO: 50;
   a412) an amino acid sequence derived by substituting 1, 2, or 3 (preferably 1) amino acids of SEQ ID NO: 50 while having the same function as the protein as shown in SEQ ID NO: 50, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
   a511) SEQ ID NO: 51;
   a512) an amino acid sequence derived by substituting 1, 2, or 3 (preferably 1) amino acids of SEQ ID NO: 51 while having the same function as the protein as shown in SEQ ID NO: 51, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
   a611) SEQ ID NO: 52;
   a612) an amino acid sequence derived by substituting 1, 2, or 3 (preferably 1) amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49 (the amino acids that are substituted are preferably selected from S91 and Y93 in SEQ ID NO: 49); or
as defined by the IMGT Definition Scheme,
the CDR-H1 is any one of a111) to a112):
   a111) SEQ ID NO: 36;
   a112) an amino acid sequence derived by substituting 1, 2, or 3 (preferably 1) amino acids of SEQ ID NO: 36 while having the same function as the protein as shown in SEQ ID NO: 36, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from Y33 in SEQ ID NO: 32);
the CDR-H2 is any one of a211) to a212):
   a211) SEQ ID NO: 37;
   a212) an amino acid sequence derived by substituting 1, 2, or 3 (preferably 1) amino acids of SEQ ID NO: 37 while having the same function as the protein as shown in SEQ ID NO: 37, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
   a311) SEQ ID NO: 38;
   a312) an amino acid sequence derived by substituting 1, 2, or 3 (preferably 1) amino acids of SEQ ID NO: 38 while having the same function as the protein as shown in SEQ ID NO: 38, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from F111 in SEQ ID NO: 32);
the CDR-L1 is any one of a411) to a412):
   a411) SEQ ID NO: 53;
   a412) an amino acid sequence derived by substituting 1, 2, or 3 (preferably 1) amino acids of SEQ ID NO: 53 while having the same function as the protein as shown in SEQ ID NO: 53, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
   a511) GAS;
   a512) an amino acid sequence derived by substituting 1 or 2 (preferably 1) amino acids of GAS while having the same function as GAS, wherein the amino acid that is substituted is selected from: an amino acid other than Y30, T31, N32, L92, Y93, S94, R95, I96, A97, D98, and R99 in SEQ ID NO: 49;
the CDR-L3 is any one of a611) to a612):
   a611) SEQ ID NO: 52;
   a612) an amino acid sequence derived by substituting 1, 2, or 3 (preferably 1) amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49 (the amino acid that are substituted are preferably selected from S91 and Y93 in SEQ ID NO: 49); or
as defined by the Chothia Definition Scheme,
the CDR-H1 is any one of a111) to a112):
   a111) SEQ ID NO: 39;
   a112) an amino acid sequence derived by substituting 1, 2, or 3 (preferably 1) amino acids of SEQ ID NO: 39 while having the same function as the protein as shown in SEQ ID NO: 39, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H2 is any one of a211) to a212):
   a211) SEQ ID NO: 40;
   a212) an amino acid sequence derived by substituting 1, 2, or 3 (preferably 1) amino acids of SEQ ID NO: 40 while having the same function as the protein as shown in SEQ ID NO: 40, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
   a311) SEQ ID NO: 35;
   a312) an amino acid sequence derived by substituting 1, 2, or 3 (preferably 1) amino acids of SEQ ID NO: 35 while having the same function as the protein as shown in SEQ ID NO: 35, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from F111 in SEQ ID NO: 32);
the CDR-L1 is any one of a411) to a412):
   a411) SEQ ID NO: 50;
   a412) an amino acid sequence derived by substituting 1, 2, or 3 (preferably 1) amino acids of SEQ ID NO: 50 while having the same function as the protein as shown in SEQ ID NO: 50, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
   a511) SEQ ID NO: 51;
   a512) an amino acid sequence derived by substituting 1, 2, or 3 (preferably 1) amino acids of SEQ ID NO: 51 while having the same function as the protein as shown in SEQ ID NO: 51, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
   a611) SEQ ID NO: 52;
   a612) an amino acid sequence derived by substituting 1, 2, or 3 (preferably 1) amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49 (the amino acids that are substituted are preferably selected from S91 and Y93 in SEQ ID NO: 49); or
as defined by the Contact Definition Scheme,
the CDR-H1 is any one of a111) to a112):
   a111) SEQ ID NO: 41;
   a112) an amino acid sequence derived by substituting 1, 2, or 3 (preferably 1) amino acids of SEQ ID NO: 41 while having the same function as the protein as shown in SEQ ID NO: 41, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from Y33 in SEQ ID NO: 32);
the CDR-H2 is any one of a211) to a212):
   a211) SEQ ID NO: 42;
   a212) an amino acid sequence derived by substituting 1, 2, or 3 (preferably 1) amino acids of SEQ ID NO: 42 while having the same function as the protein as shown in SEQ ID NO: 42, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
   a311) SEQ ID NO: 43;
   a312) an amino acid sequence derived by substituting 1, 2, or 3 (preferably 1) amino acids of SEQ ID NO: 43 while having the same function as the protein as shown in SEQ ID NO: 43, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from F111 in SEQ ID NO: 32);
the CDR-L1 is any one of a411) to a412):
   a411) SEQ ID NO: 54;
   a412) an amino acid sequence derived by substituting 1, 2, or 3 (preferably 1) amino acids of SEQ ID NO: 54 while having the same function as the protein as shown in SEQ ID NO: 54, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
   a511) SEQ ID NO: 55;
   a512) an amino acid sequence derived by substituting 1, 2, or 3 (preferably 1) amino acids of SEQ ID NO: 55 while having the same function as the protein as shown in SEQ ID NO: 55, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
   a611) SEQ ID NO: 56;
   a612) an amino acid sequence derived by substituting 1, 2, or 3 (preferably 1) amino acids of SEQ ID NO: 56 while having the same function as the protein as shown in SEQ ID NO: 56, wherein the amino acid that is substituted is selected from: an amino acid other than Y30, T31, N32, L92, Y93, S94, R95, 196, A97, D98, and R99 in SEQ ID NO: 49 (the amino acids that are substituted are preferably selected from S91 and Y93 in SEQ ID NO: 49); or
as defined by the Abm Definition Scheme,
the CDR-H1 is any one of a111) to a112):
   a111) SEQ ID NO: 44;
   a112) an amino acid sequence derived by substituting 1, 2, or 3 (preferably 1) amino acids of SEQ ID NO: 44 while having the same function as the protein as shown in SEQ ID NO: 44, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from Y33 in SEQ ID NO: 32);
the CDR-H2 is any one of a211) to a212):
   a211) SEQ ID NO: 45;
   a212) an amino acid sequence derived by substituting 1, 2, or 3 (preferably 1) amino acids of SEQ ID NO: 45 while having the same function as the protein as shown in SEQ ID NO: 45, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
   a311) SEQ ID NO: 35;
   a312) an amino acid sequence derived by substituting 1, 2, or 3 (preferably 1) amino acids of SEQ ID NO: 35 while having the same function as the protein as shown in SEQ ID NO: 35, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from F111 in SEQ ID NO: 32);
the CDR-L1 is any one of a411) to a412):
   a411) SEQ ID NO: 50;
   a412) an amino acid sequence derived by substituting 1, 2, or 3 (preferably 1) amino acids of SEQ ID NO: 50 while having the same function as the protein as shown in SEQ ID NO: 50, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
   a511) SEQ ID NO: 51;
   a512) an amino acid sequence derived by substituting 1, 2, or 3 (preferably 1) amino acids of SEQ ID NO: 51 while having the same function as the protein as shown in SEQ ID NO: 51, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
   a611) SEQ ID NO: 52;
   a612) an amino acid sequence derived by substituting 1, 2, or 3 (preferably 1) amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49 (the amino acids that are substituted are preferably selected from S91 and Y93 in SEQ ID NO: 49); or
the 240B10-1 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
   a111) SEQ ID NO: 61;
   a112) an amino acid sequence derived by substituting 1, 2, or 3 (preferably 1) amino acids of SEQ ID NO: 61 while having the same function as the protein as shown in SEQ ID NO: 61, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
   a211) SEQ ID NO: 62;
   a212) an amino acid sequence derived by substituting 1, 2, or 3 (preferably 1) amino acids of SEQ ID NO: 62 while having the same function as the protein as shown in SEQ ID NO: 62, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
   a311) SEQ ID NO: 63;
   a312) an amino acid sequence derived by substituting 1, 2, or 3 (preferably 1) amino acids of SEQ ID NO: 63 while having the same function as the protein as shown in SEQ ID NO: 63, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
   a411) SEQ ID NO: 78;
   a412) an amino acid sequence derived by substituting 1, 2, or 3 (preferably 1) amino acids of SEQ ID NO: 78 while having the same function as the protein as shown in SEQ ID NO: 78, wherein the amino acid that is substituted is selected from: an amino acid other than Y29, N30, D32, Y33, S51, K54, C75, and Y76 in SEQ ID NO: 77;
the CDR-L2 is any one of a511) to a512):
   a511) SEQ ID NO: 79;
   a512) an amino acid sequence derived by substituting 1, 2, or 3 (preferably 1) amino acids of SEQ ID NO: 79 while having the same function as the protein as shown in SEQ ID NO: 79, wherein the amino acid that is substituted is selected from: an amino acid other than Y29, N30, D32, Y33, S51, K54, C75, and Y76 in SEQ ID NO: 77;
the CDR-L3 is any one of a611) to a612):
   a611) SEQ ID NO: 80;
   a612) an amino acid sequence derived by substituting 1, 2, or 3 (preferably 1) amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
as defined by the IMGT Definition Scheme,
the CDR-H1 is any one of a111) to a112):
   a111) SEQ ID NO: 64;
   a112) an amino acid sequence derived by substituting 1, 2, or 3 (preferably 1) amino acids of SEQ ID NO: 64 while having the same function as the protein as shown in SEQ ID NO: 64, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
   a211) SEQ ID NO: 65;
   a212) an amino acid sequence derived by substituting 1, 2, or 3 (preferably 1) amino acids of SEQ ID NO: 65 while having the same function as the protein as shown in SEQ ID NO: 65, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
   a311) SEQ ID NO: 66;
   a312) an amino acid sequence derived by substituting 1, 2, or 3 (preferably 1) amino acids of SEQ ID NO: 66 while having the same function as the protein as shown in SEQ ID NO: 66, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
   a411) SEQ ID NO: 81;
   a412) an amino acid sequence derived by substituting 1, 2, or 3 (preferably 1) amino acids of SEQ ID NO: 81 while having the same function as the protein as shown in SEQ ID NO: 81, wherein the amino acid that is substituted is selected from: an amino acid other than Y29, N30, D32, Y33, S51, K54, C75, and Y76 in SEQ ID NO: 77;
the CDR-L2 is any one of a511) to a512):
   a511) SAS;
   a512) an amino acid sequence derived by substituting 1 or 2 (preferably 1) amino acids of SAS while having the same function as SAS, wherein the amino acid that is substituted is selected from: an amino acid other than Y29, N30, D32, Y33, S51, K54, C75, and Y76 in SEQ ID NO: 77;
the CDR-L3 is any one of a611) to a612):
   a611) SEQ ID NO: 80;
   a612) an amino acid sequence derived by substituting 1, 2, or 3 (preferably 1) amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
as defined by the Chothia Definition Scheme,
the CDR-H1 is any one of a111) to a112):
   a111) SEQ ID NO: 67;
   a112) an amino acid sequence derived by substituting 1, 2, or 3 (preferably 1) amino acids of SEQ ID NO: 67 while having the same function as the protein as shown in SEQ ID NO: 67, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
   a211) SEQ ID NO: 68;
   a212) an amino acid sequence derived by substituting 1, 2, or 3 (preferably 1) amino acids of SEQ ID NO: 68 while having the same function as the protein as shown in SEQ ID NO: 68, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
   a311) SEQ ID NO: 63;
   a312) an amino acid sequence derived by substituting 1, 2, or 3 (preferably 1) amino acids of SEQ ID NO: 63 while having the same function as the protein as shown in SEQ ID NO: 63, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
   a411) SEQ ID NO: 78;
   a412) an amino acid sequence derived by substituting 1, 2, or 3 (preferably 1) amino acids of SEQ ID NO: 78 while having the same function as the protein as shown in SEQ ID NO: 78, wherein the amino acid that is substituted is selected from: an amino acid other than Y29, N30, D32, Y33, S51, K54, C75, and Y76 in SEQ ID NO: 77;
the CDR-L2 is any one of a511) to a512):
   a511) SEQ ID NO: 79;
   a512) an amino acid sequence derived by substituting 1, 2, or 3 (preferably 1) amino acids of SEQ ID NO: 79 while having the same function as the protein as shown in SEQ ID NO: 79, wherein the amino acid that is substituted is selected from: an amino acid other than Y29, N30, D32, Y33, S51, K54, C75, and Y76 in SEQ ID NO: 77;
the CDR-L3 is any one of a611) to a612):
   a611) SEQ ID NO: 80;
   a612) an amino acid sequence derived by substituting 1, 2, or 3 (preferably 1) amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
as defined by the Contact Definition Scheme,
the CDR-H1 is any one of a111) to a112):
   a111) SEQ ID NO: 69;
   a112) an amino acid sequence derived by substituting 1, 2, or 3 (preferably 1) amino acids of SEQ ID NO: 69 while having the same function as the protein as shown in SEQ ID NO: 69, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
   a211) SEQ ID NO: 70;
   a212) an amino acid sequence derived by substituting 1, 2, or 3 (preferably 1) amino acids of SEQ ID NO: 70 while having the same function as the protein as shown in SEQ ID NO: 70, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
   a311) SEQ ID NO: 71;
   a312) an amino acid sequence derived by substituting 1, 2, or 3 (preferably 1) amino acids of SEQ ID NO: 71 while having the same function as the protein as shown in SEQ ID NO: 71, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
   a411) SEQ ID NO: 82;
   a412) an amino acid sequence derived by substituting 1, 2, or 3 (preferably 1) amino acids of SEQ ID NO: 82 while having the same function as the protein as shown in SEQ ID NO: 82, wherein the amino acid that is substituted is selected from: an amino acid other than Y29, N30, D32, Y33, S51, K54, C75, and Y76 in SEQ ID NO: 77;
the CDR-L2 is any one of a511) to a512):
   a511) SEQ ID NO: 83;
   a512) an amino acid sequence derived by substituting 1, 2, or 3 (preferably 1) amino acids of SEQ ID NO: 83 while having the same function as the protein as shown in SEQ ID NO: 83, wherein the amino acid that is substituted is selected from: an amino acid other than Y29, N30, D32, Y33, S51, K54, C75, and Y76 in SEQ ID NO: 77;
the CDR-L3 is any one of a611) to a612):
   a611) SEQ ID NO: 84;
   a612) an amino acid sequence derived by substituting 1, 2, or 3 (preferably 1) amino acids of SEQ ID NO: 84 while having the same function as the protein as shown in SEQ ID NO: 84, wherein the amino acid that is substituted is selected from: an amino acid other than Y29, N30, D32, Y33, S51, K54, C75, and Y76 in SEQ ID NO: 77; or
as defined by the Abm Definition Scheme,
the CDR-H1 is any one of a111) to a112):
   a111) SEQ ID NO: 72;
   a112) an amino acid sequence derived by substituting 1, 2, or 3 (preferably 1) amino acids of SEQ ID NO: 72 while having the same function as the protein as shown in SEQ ID NO: 72, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
   a211) SEQ ID NO: 73;
   a212) an amino acid sequence derived by substituting 1, 2, or 3 (preferably 1) amino acids of SEQ ID NO: 73 while having the same function as the protein as shown in SEQ ID NO: 73, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
   a311) SEQ ID NO: 63;
   a312) an amino acid sequence derived by substituting 1, 2, or 3 (preferably 1) amino acids of SEQ ID NO: 63 while having the same function as the protein as shown in SEQ ID NO: 63, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
   a411) SEQ ID NO: 78;
   a412) an amino acid sequence derived by substituting 1, 2, or 3 (preferably 1) amino acids of SEQ ID NO: 78 while having the same function as the protein as shown in SEQ ID NO: 78, wherein the amino acid that is substituted is selected from: an amino acid other than Y29, N30, D32, Y33, S51, K54, C75, and Y76 in SEQ ID NO: 77;
the CDR-L2 is any one of a511) to a512):
   a511) SEQ ID NO: 79;
   a512) an amino acid sequence derived by substituting 1, 2, or 3 (preferably 1) amino acids of SEQ ID NO: 79 while having the same function as the protein as shown in SEQ ID NO: 79, wherein the amino acid that is substituted is selected from: an amino acid other than Y29, N30, D32, Y33, S51, K54, C75, and Y76 in SEQ ID NO: 77;
the CDR-L3 is any one of a611) to a612):
   a611) SEQ ID NO: 80;
   a612) an amino acid sequence derived by substituting 1, 2, or 3 (preferably 1) amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
the 98G10 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
   a111) SEQ ID NO: 89;
   a112) an amino acid sequence derived by substituting 1, 2, or 3 (preferably 1) amino acids of SEQ ID NO: 89 while having the same function as the protein as shown in SEQ ID NO: 89, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from W34 in SEQ ID NO: 88);
the CDR-H2 is any one of a211) to a212):
   a211) SEQ ID NO: 90;
   a212) an amino acid sequence derived by substituting 1, 2, or 3 (preferably 1) amino acids of SEQ ID NO: 90 while having the same function as the protein as shown in SEQ ID NO: 90, wherein the amino acid that is substituted is selected from: an amino acid other than F29, S30, A31, Y53, G55, S56, S57, G58, S59, Y61, Y102, N104, S107, A108, G109, D110, 1111, and Y112 in SEQ ID NO: 88;
the CDR-H3 is any one of a311) to a312):
   a311) SEQ ID NO: 91;
   a312) an amino acid sequence derived by substituting 1, 2, or 3 (preferably 1) amino acids of SEQ ID NO: 91 while having the same function as the protein as shown in SEQ ID NO: 91, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from Y112 in SEQ ID NO: 88);
the CDR-L1 is any one of a411) to a412):
   a411) SEQ ID NO: 106;
   a412) an amino acid sequence derived by substituting 1, 2, or 3 (preferably 1) amino acids of SEQ ID NO: 106 while having the same function as the protein as shown in SEQ ID NO: 106, wherein the amino acid that is substituted is selected from: an amino acid other than K53 in SEQ ID NO: 105;
the CDR-L2 is any one of a511) to a512):
   a511) SEQ ID NO: 107;
   a512) an amino acid sequence derived by substituting 1, 2, or 3 (preferably 1) amino acids of SEQ ID NO: 107 while having the same function as the protein as shown in SEQ ID NO: 107, wherein the amino acid that is substituted is selected from: an amino acid other than K53 in SEQ ID NO: 105;
the CDR-L3 is any one of a611) to a612):
   a611) SEQ ID NO: 108;
   a612) an amino acid sequence derived by substituting 1, 2, or 3 (preferably 1) amino acids of SEQ ID NO: 108 while having the same function as the protein as shown in SEQ ID NO: 108, wherein the amino acid that is substituted is selected from: an amino acid other than K53 in SEQ ID NO: 105 (the amino acid that is substituted is preferably selected from Y93 in SEQ ID NO: 108); or
as defined by the IMGT Definition Scheme,
the CDR-H1 is any one of a111) to a112):
   a111) SEQ ID NO: 92;
   a112) an amino acid sequence derived by substituting 1, 2, or 3 (preferably 1) amino acids of SEQ ID NO: 92 while having the same function as the protein as shown in SEQ ID NO: 92, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from W34 in SEQ ID NO: 88);
the CDR-H2 is any one of a211) to a212):
   a211) SEQ ID NO: 93;
   a212) an amino acid sequence derived by substituting 1, 2, or 3 (preferably 1) amino acids of SEQ ID NO: 93 while having the same function as the protein as shown in SEQ ID NO: 93, wherein the amino acid that is substituted is selected from: an amino acid other than F29, S30, A31, Y53, G55, S56, S57, G58, S59, Y61, Y102, N104, S107, A108, G109, D110, 1111, and Y112 in SEQ ID NO: 88;
the CDR-H3 is any one of a311) to a312):
   a311) SEQ ID NO: 94;
   a312) an amino acid sequence derived by substituting 1, 2, or 3 (preferably 1) amino acids of SEQ ID NO: 94 while having the same function as the protein as shown in SEQ ID NO: 94, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from Y112 in SEQ ID NO: 88);
the CDR-L1 is any one of a411) to a412):
   a411) SEQ ID NO: 109;
   a412) an amino acid sequence derived by substituting 1, 2, or 3 (preferably 1) amino acids of SEQ ID NO: 109 while having the same function as the protein as shown in SEQ ID NO: 109, wherein the amino acid that is substituted is selected from: an amino acid other than K53 in SEQ ID NO: 105;
the CDR-L2 is any one of a511) to a512):
   a511) TAS;
   a512) the amino acid sequence derived by substituting 1 or 2 (preferably 1) amino acids of TAS while having the same function as TAS, wherein the amino acid that is substituted is selected from: an amino acid other than K53 in SEQ ID NO: 105;
the CDR-L3 is any one of a611) to a612):
   a611) SEQ ID NO: 108;
   a612) an amino acid sequence derived by substituting 1, 2, or 3 (preferably 1) amino acids of SEQ ID NO: 108 while having the same function as the protein as shown in SEQ ID NO: 108, wherein the amino acid that is substituted is selected from: an amino acid other than K53 in SEQ ID NO: 105 (the amino acid that is substituted is preferably selected from Y93 in SEQ ID NO: 108); or
as defined by the Chothia Definition Scheme,
the CDR-H1 is any one of a111) to a112):
   a111) SEQ ID NO: 95;
   a112) an amino acid sequence derived by substituting 1, 2, or 3 (preferably 1) amino acids of SEQ ID NO: 95 while having the same function as the protein as shown in SEQ ID NO: 95, wherein the amino acid that is substituted is selected from: an amino acid other than F29, S30, A31, Y53, G55, S56, S57, G58, S59, Y61, Y102, N104, S107, A108, G109, D110, 1111, and Y112 in SEQ ID NO: 88;
the CDR-H2 is any one of a211) to a212):
   a211) SEQ ID NO: 96;
   a212) an amino acid sequence derived by substituting 1, 2, or 3 (preferably 1) amino acids of SEQ ID NO: 96 while having the same function as the protein as shown in SEQ ID NO: 96, wherein the amino acid that is substituted is selected from: an amino acid other than F29, S30, A31, Y53, G55, S56, S57, G58, S59, Y61, Y102, N104, S107, A108, G109, D110, 1111, and Y112 in SEQ ID NO: 88;
the CDR-H3 is any one of a311) to a312):
   a311) SEQ ID NO: 91;
   a312) an amino acid sequence derived by substituting 1, 2, or 3 (preferably 1) amino acids of SEQ ID NO: 91 while having the same function as the protein as shown in SEQ ID NO: 91, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from Y112 in SEQ ID NO: 88);
the CDR-L1 is any one of a411) to a412):
   a411) SEQ ID NO: 106;
   a412) an amino acid sequence derived by substituting 1, 2, or 3 (preferably 1) amino acids of SEQ ID NO: 106 while having the same function as the protein as shown in SEQ ID NO: 106, wherein the amino acid that is substituted is selected from: an amino acid other than K53 in SEQ ID NO: 105;
the CDR-L2 is any one of a511) to a512):
   a511) SEQ ID NO: 107;
   a512) an amino acid sequence derived by substituting 1, 2, or 3 (preferably 1) amino acids of SEQ ID NO: 107 while having the same function as the protein as shown in SEQ ID NO: 107, wherein the amino acid that is substituted is selected from: an amino acid other than K53 in SEQ ID NO: 105;
the CDR-L3 is any one of a611) to a612):
   a611) SEQ ID NO: 108;
   a612) an amino acid sequence derived by substituting 1, 2, or 3 (preferably 1) amino acids of SEQ ID NO: 108 while having the same function as the protein as shown in SEQ ID NO: 108, wherein the amino acid that is substituted is selected from: an amino acid other than K53 in SEQ ID NO: 105 (the amino acid that is substituted is preferably selected from Y93 in SEQ ID NO: 108); or
as defined by the Contact Definition Scheme,
the CDR-H1 is any one of a111) to a112):
   a111) SEQ ID NO: 97;
   a112) an amino acid sequence derived by substituting 1, 2, or 3 (preferably 1) amino acids of SEQ ID NO: 97 while having the same function as the protein as shown in SEQ ID NO: 97, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from W34 in SEQ ID NO: 88);
the CDR-H2 is any one of a211) to a212):
   a211) SEQ ID NO: 98;
   a212) an amino acid sequence derived by substituting 1, 2, or 3 (preferably 1) amino acids of SEQ ID NO: 98 while having the same function as the protein as shown in SEQ ID NO: 98, wherein the amino acid that is substituted is selected from: an amino acid other than F29, S30, A31, Y53, G55, S56, S57, G58, S59, Y61, Y102, N104, S107, A108, G109, D110, 1111, and Y112 in SEQ ID NO: 88;
the CDR-H3 is any one of a311) to a312):
   a311) SEQ ID NO: 99;
   a312) an amino acid sequence derived by substituting 1, 2, or 3 (preferably 1) amino acids of SEQ ID NO: 99 while having the same function as the protein as shown in SEQ ID NO: 99, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from Y112 in SEQ ID NO: 88);
the CDR-L1 is any one of a411) to a412):
   a411) SEQ ID NO: 110;
   a412) an amino acid sequence derived by substituting 1, 2, or 3 (preferably 1) amino acids of SEQ ID NO: 110 while having the same function as the protein as shown in SEQ ID NO: 110, wherein the amino acid that is substituted is selected from: an amino acid other than K53 in SEQ ID NO: 105;
the CDR-L2 is any one of a511) to a512):
   a511) SEQ ID NO: 111;
   a512) an amino acid sequence derived by substituting 1, 2, or 3 (preferably 1) amino acids of SEQ ID NO: 111 while having the same function as the protein as shown in SEQ ID NO: 111, wherein the amino acid that is substituted is selected from: an amino acid other than K53 in SEQ ID NO: 105;
the CDR-L3 is any one of a611) to a612):
   a611) SEQ ID NO: 112;
   a612) an amino acid sequence derived by substituting 1, 2, or 3 (preferably 1) amino acids of SEQ ID NO: 112 while having the same function as the protein as shown in SEQ ID NO: 112, wherein the amino acid that is substituted is selected from: an amino acid other than K53 in SEQ ID NO: 105 (the amino acid that is substituted is preferably selected from Y93 in SEQ ID NO: 108); or
as defined by the Abm Definition Scheme,
the CDR-H1 is any one of a111) to a112):
   a111) SEQ ID NO: 100;
   a112) an amino acid sequence derived by substituting 1, 2, or 3 (preferably 1) amino acids of SEQ ID NO: 100 while having the same function as the protein as shown in SEQ ID NO: 100, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from W34 in SEQ ID NO: 88);
the CDR-H2 is any one of a211) to a212):
   a211) SEQ ID NO: 101;
   a212) an amino acid sequence derived by substituting 1, 2, or 3 (preferably 1) amino acids of SEQ ID NO: 101 while having the same function as the protein as shown in SEQ ID NO: 101, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H3 is any one of a311) to a312):
   a311) SEQ ID NO: 91;
   a312) an amino acid sequence derived by substituting 1, 2, or 3 (preferably 1) amino acids of SEQ ID NO: 91 while having the same function as the protein as shown in SEQ ID NO: 91, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88 (the amino acid that is substituted is preferably selected from Y112 in SEQ ID NO: 88);
the CDR-L1 is any one of a411) to a412):
   a411) SEQ ID NO: 106;
   a412) an amino acid sequence derived by substituting 1, 2, or 3 (preferably 1) amino acids of SEQ ID NO: 106 while having the same function as the protein as shown in SEQ ID NO: 106, wherein the amino acid that is substituted is selected from: an amino acid other than K53 in SEQ ID NO: 105;
the CDR-L2 is any one of a511) to a512):
   a511) SEQ ID NO: 107;
   a512) an amino acid sequence derived by substituting 1, 2, or 3 (preferably 1) amino acids of SEQ ID NO: 107 while having the same function as the protein as shown in SEQ ID NO: 107, wherein the amino acid that is substituted is selected from: an amino acid other than K53 in SEQ ID NO: 105;
the CDR-L3 is any one of a611) to a612):
   a611) SEQ ID NO: 108;
   a612) an amino acid sequence derived by substituting 1, 2, or 3 (preferably 1) amino acids of SEQ ID NO: 108 while having the same function as the protein as shown in SEQ ID NO: 108, wherein the amino acid that is substituted is selected from: an amino acid other than K53 in SEQ ID NO: 105 (the amino acid that is substituted is preferably selected from Y93 in SEQ ID NO: 108); or
the 39B4 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
   a111) SEQ ID NO: 117;
   a112) the amino acid sequence derived by substituting 1, 2, or 3 (preferably 1) amino acids of SEQ ID NO: 117 while having the same function as the protein as shown in SEQ ID NO: 117, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
   a211) SEQ ID NO: 118;
   a212) an amino acid sequence derived by substituting 1, 2, or 3 (preferably 1) amino acids of SEQ ID NO: 118 while having the same function as the protein as shown in SEQ ID NO: 118, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
   a311) SEQ ID NO: 119;
   a312) an amino acid sequence derived by substituting 1, 2, or 3 (preferably 1) amino acids of SEQ ID NO: 119 while having the same function as the protein as shown in SEQ ID NO: 119, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
   a411) SEQ ID NO: 133;
   a412) an amino acid sequence derived by substituting 1, 2, or 3 (preferably 1) amino acids of SEQ ID NO: 133 while having the same function as the protein as shown in SEQ ID NO: 133, wherein the amino acid that is substituted is selected from: an amino acid other than S28, N31, Y93, and S96 in SEQ ID NO: 132;
the CDR-L2 is any one of a511) to a512):
   a511) SEQ ID NO: 134;
   a512) an amino acid sequence derived by substituting 1, 2, or 3 (preferably 1) amino acids of SEQ ID NO: 134 while having the same function as the protein as shown in SEQ ID NO: 134, wherein the amino acid that is substituted is selected from: an amino acid other than S28, N31, Y93, and S96 in SEQ ID NO: 132;
the CDR-L3 is any one of a611) to a612):
   a611) SEQ ID NO: 135;
   a612) an amino acid sequence derived by substituting 1, 2, or 3 (preferably 1) amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132; or
as defined by the IMGT Definition Scheme,
the CDR-H1 is any one of a111) to a112):
   a111) SEQ ID NO: 120;
   a112) an amino acid sequence derived by substituting 1, 2, or 3 (preferably 1) amino acids of SEQ ID NO: 120 while having the same function as the protein as shown in SEQ ID NO: 120, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
   a211) SEQ ID NO: 121;
   a212) an amino acid sequence derived by substituting 1, 2, or 3 (preferably 1) amino acids of SEQ ID NO: 121 while having the same function as the protein as shown in SEQ ID NO: 121, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
   a311) SEQ ID NO: 122;
   a312) an amino acid sequence derived by substituting 1, 2, or 3 (preferably 1) amino acids of SEQ ID NO: 122 while having the same function as the protein as shown in SEQ ID NO: 122, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
   a411) SEQ ID NO: 136;
   a412) an amino acid sequence derived by substituting 1, 2, or 3 (preferably 1) amino acids of SEQ ID NO: 136 while having the same function as the protein as shown in SEQ ID NO: 136, wherein the amino acid that is substituted is selected from: an amino acid other than S28, N31, Y93, and S96 in SEQ ID NO: 132;
the CDR-L2 is any one of a511) to a512):
   a511) SAS;
   a512) an amino acid sequence derived by substituting 1 or 2 (preferably 1) amino acids of SAS while having the same function as SAS, wherein the amino acid that is substituted is selected from: an amino acid other than S28, N31, Y93, and S96 in SEQ ID NO: 132;
the CDR-L3 is any one of a611) to a612):
   a611) SEQ ID NO: 135;
   a612) an amino acid sequence derived by substituting 1, 2, or 3 (preferably 1) amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132; or
as defined by the Chothia Definition Scheme,
the CDR-H1 is any one of a111) to a112):
   a111) SEQ ID NO: 120;
   a112) an amino acid sequence derived by substituting 1, 2, or 3 (preferably 1) amino acids of SEQ ID NO: 120 while having the same function as the protein as shown in SEQ ID NO: 120, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
   a211) SEQ ID NO: 123;
   a212) an amino acid sequence derived by substituting 1, 2, or 3 (preferably 1) amino acids of SEQ ID NO: 123 while having the same function as the protein as shown in SEQ ID NO: 123, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
   a311) SEQ ID NO: 119;
   a312) an amino acid sequence derived by substituting 1, 2, or 3 (preferably 1) amino acids of SEQ ID NO: 119 while having the same function as the protein as shown in SEQ ID NO: 119, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
   a411) SEQ ID NO: 133;
   a412) an amino acid sequence derived by substituting 1, 2, or 3 (preferably 1) amino acids of SEQ ID NO: 133 while having the same function as the protein as shown in SEQ ID NO: 133, wherein the amino acid that is substituted is selected from: an amino acid other than S28, N31, Y93, and S96 in SEQ ID NO: 132;
the CDR-L2 is any one of a511) to a512):
   a511) SEQ ID NO: 134;
   a512) an amino acid sequence derived by substituting 1, 2, or 3 (preferably 1) amino acids of SEQ ID NO: 134 while having the same function as the protein as shown in SEQ ID NO: 134, wherein the amino acid that is substituted is selected from: an amino acid other than S28, N31, Y93, and S96 in SEQ ID NO: 132;
the CDR-L3 is any one of a611) to a612):
   a611) SEQ ID NO: 135;
   a612) an amino acid sequence derived by substituting 1, 2, or 3 (preferably 1) amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132; or
as defined by the Contact Definition Scheme,
the CDR-H1 is any one of a111) to a112):
   a111) SEQ ID NO: 117;
   a112) an amino acid sequence derived by substituting 1, 2, or 3 (preferably 1) amino acids of SEQ ID NO: 117 while having the same function as the protein as shown in SEQ ID NO: 117, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
   a211) SEQ ID NO: 124;
   a212) an amino acid sequence derived by substituting 1, 2, or 3 (preferably 1) amino acids of SEQ ID NO: 124 while having the same function as the protein as shown in SEQ ID NO: 124, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
   a311) SEQ ID NO: 125;
   a312) an amino acid sequence derived by substituting 1, 2, or 3 (preferably 1) amino acids of SEQ ID NO: 125 while having the same function as the protein as shown in SEQ ID NO: 125, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
   a411) SEQ ID NO: 137;
   a412) an amino acid sequence derived by substituting 1, 2, or 3 (preferably 1) amino acids of SEQ ID NO: 137 while having the same function as the protein as shown in SEQ ID NO: 137, wherein the amino acid that is substituted is selected from: an amino acid other than S28, N31, Y93, and S96 in SEQ ID NO: 132;
the CDR-L2 is any one of a511) to a512):
   a511) SEQ ID NO: 138;
   a512) an amino acid sequence derived by substituting 1, 2, or 3 (preferably 1) amino acids of SEQ ID NO: 138 while having the same function as the protein as shown in SEQ ID NO: 138, wherein the amino acid that is substituted is selected from: an amino acid other than S28, N31, Y93, and S96 in SEQ ID NO: 132;
the CDR-L3 is any one of a611) to a612):
   a611) SEQ ID NO: 139;
   a612) an amino acid sequence derived by substituting 1, 2, or 3 (preferably 1) amino acids of SEQ ID NO: 139 while having the same function as the protein as shown in SEQ ID NO: 139, wherein the amino acid that is substituted is selected from: an amino acid other than S28, N31, Y93, and S96 in SEQ ID NO: 132; or
as defined by the Abm Definition Scheme,
the CDR-H1 is any one of a111) to a112):
   a111) SEQ ID NO: 126;
   a112) an amino acid sequence derived by substituting 1, 2, or 3 (preferably 1) amino acids of SEQ ID NO: 126 while having the same function as the protein as shown in SEQ ID NO: 126, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
   a211) SEQ ID NO: 127;
   a212) an amino acid sequence derived by substituting 1, 2, or 3 (preferably 1) amino acids of SEQ ID NO: 127 while having the same function as the protein as shown in SEQ ID NO: 127, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
   a311) SEQ ID NO: 128;
   a312) an amino acid sequence derived by substituting 1, 2, or 3 (preferably 1) amino acids of SEQ ID NO: 128 while having the same function as the protein as shown in SEQ ID NO: 128, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
   a411) SEQ ID NO: 133;
   a412) an amino acid sequence derived by substituting 1, 2, or 3 (preferably 1) amino acids of SEQ ID NO: 133 while having the same function as the protein as shown in SEQ ID NO: 133, wherein the amino acid that is substituted is selected from: an amino acid other than S28, N31, Y93, and S96 in SEQ ID NO: 132;
the CDR-L2 is any one of a511) to a512):
   a511) SEQ ID NO: 134;
   a512) an amino acid sequence derived by substituting 1, 2, or 3 (preferably 1) amino acids of SEQ ID NO: 134 while having the same function as the protein as shown in SEQ ID NO: 134, wherein the amino acid that is substituted is selected from: an amino acid other than S28, N31, Y93, and S96 in SEQ ID NO: 132;
the CDR-L3 is any one of a611) to a612):
   a611) SEQ ID NO: 135;
   a612) an amino acid sequence derived by substituting 1, 2, or 3 (preferably 1) amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132.

Preferably, the CDR-H1, CDR-H2, CDR-H3, CDR-L1, CDR-L2 and CDR-L3 of the above-mentioned antibody or antigen-binding fragment thereof (94C4, B-8, 240B10-1, 98G10, or 39B4) defined by different definition schemes can be arbitrarily combined: taking 94C4 defined by Kabat Definition Scheme as an example, the combinations of CDR-H1, CDR-H2, CDR-H3, CDR-L1, CDR-L2 and CDR-L3 can be: a111), a211), a311), a411), a511) and a611); a112), a211), a311), a411), a511) and a611); and so on, which will not be enumerated here.

Preferably, the substituted amino acid or the inserted amino acid may be any amino acid present in the prior art, including but not limited to: alanine, cysteine, aspartic acid, glutamic acid, phenylalanine, glycine, histidine, isoleucine, lysine, leucine, methionine, asparagine, proline, glutamine, arginine, serine, threonine, valine, tryptophan, tyrosine, 2-aminoadipic acid, 3-aminoadipic acid, β-alanine, 2-aminobutyric acid, 4-aminobutyric acid, 6-aminohexanoic acid, 2-aminoheptanoic acid, 2-aminoisobutyric acid, 3-aminoisobutyric acid, 2-aminoheptanedioic acid, 2,4-diaminobutyric acid, lysine, 2,2'-diaminoheptanedioic acid, 2,3-diaminopropionic acid, N-ethyl glycine, N-ethylaspartic acid, hydroxylysine, allo-hydroxylysine, 3-hydroxyproline, 4-hydroxyproline, iso-lysine, allo-isoleucine, sarcosine, N-methyl-isoleucine, 6-N-methyllysine, N-methyl-valine, n-valine, n-leucine, and ornithine.

Preferably, the above-mentioned amino acid sequences after substitution are related to the number and position of the amino acids that are substituted, as well as the number of amino acids in the corresponding CDRs (CDR-H1, CDR-H2, CDR-H3, CDR-L1, CDR-L2 and/or CDR-L3), wherein the amino acid sequence after substitution also corresponds to the selection of amino acids after substitution. Thus, there are numerous amino acid sequences after substitution, which are all within the scope of protection of the present application, and hereinafter, each of the amino acid sequences will not be enumerated one by one.

The applicant seeking for protection of the amino acid sequence (i.e., a112), a212), a312), a412), a512), and a612)) in which the above substitutions occur relative to a111), a211), a311), a411), a511), and a611) is due to the following: the applicant has found that the amino acids bound to/recognized by the antigen NMI are some amino acids in the a111), a211), a311), a411), a511), and a611) (herein referred to as key amino acids), therefore, substitutions of amino acids other than these amino acids (the key amino acids) do not affect the function of the antibody or antigen-binding fragment thereof.

Preferably, the amino acid sequences of CDR-H1, CDR-H2, CDR-H3, CDR-L1, CDR-L2, and CDR-L3 of the 94C4 are as shown in SEQ ID NOs: 5, 6, 7, 22, 23, and 24 in order, and the CDRs are defined by the Kabat Definition Scheme; or
the amino acid sequences of CDR-H1, CDR-H2, CDR-H3, CDR-L1, and CDR-L3 of the 94C4 are as shown in SEQ ID NOs: 8, 9, 10, 25, and 24 in order and the amino acid sequence of CDR-L2 is RVS, and the CDRs are defined by the IMGT Definition Scheme; or
the amino acid sequences of CDR-H1, CDR-H2, CDR-H3, CDR-L1, CDR-L2, and CDR-L3 of the 94C4 are as shown in SEQ ID NOs: 11, 12, 7, 22, 23, and 24 in order, and the CDRs are defined by the Chothia Definition Scheme; or
the amino acid sequences of CDR-H1, CDR-H2, CDR-H3, CDR-L1, CDR-L2, and CDR-L3 of the 94C4 are as shown in SEQ ID NOs: 13, 14, 15, 26, 27, and 28 in order, and the CDRs are defined by the Contact Definition Scheme; or
the amino acid sequences of CDR-H1, CDR-H2, CDR-H3, CDR-L1, CDR-L2, and CDR-L3 of the 94C4 are as shown in SEQ ID NOs: 16, 17, 7, 22, 23, and 24 in order, and the CDRs are defined by the Abm Definition Scheme.

Preferably, the amino acid sequences of CDR-H1, CDR-H2, CDR-H3, CDR-L1, CDR-L2, and CDR-L3 of the 240B10-1 are as shown in SEQ ID NOs: 61, 62, 63, 78, 79, and 80 in order, and the CDRs are defined by the Kabat Definition Scheme; or
the amino acid sequences of CDR-H1, CDR-H2, CDR-H3, CDR-L1, and CDR-L3 of the 240B10-1 are as shown in SEQ ID NOs: 64, 65, 66, 81, and 80 in order and the amino acid sequence of CDR-L2 is SAS, and the CDRs are defined by the IMGT Definition Scheme; or
the amino acid sequences of CDR-H1, CDR-H2, CDR-H3, CDR-L1, CDR-L2, and CDR-L3 of the 240B10-1 are as shown in SEQ ID NOs: 67, 68, 63, 78, 79, and 80 in order, and the CDRs are defined by the Chothia Definition Scheme; or
the amino acid sequences of CDR-H1, CDR-H2, CDR-H3, CDR-L1, CDR-L2, and CDR-L3 of the 240B10-1 are as shown in SEQ ID NOs: 69, 70, 71, 82, 83, and 84 in order, and the CDRs are defined by the Contact Definition Scheme; or
the amino acid sequences of CDR-H1, CDR-H2, CDR-H3, CDR-L1, CDR-L2, and CDR-L3 of the 240B10-1 are as shown in SEQ ID NOs: 72, 73, 63, 78, 79, and 80 in order, and the CDRs are defined by the Abm Definition Scheme.

Preferably, the amino acid sequences of CDR-H1, CDR-H2, CDR-H3, CDR-L1, CDR-L2, and CDR-L3 of the 39B4 are as shown in SEQ ID NOs: 117, 118, 119, 133, 134, and 135 in order, and the CDRs are defined by the Kabat Definition Scheme; or
the amino acid sequences of CDR-H1, CDR-H2, CDR-H3, CDR-L1, and CDR-L3 of the 39B4 are as shown in SEQ ID NOs: 120, 121, 122, 136, and 135 in order and the amino acid sequence of CDR-L2 is SAS, and the CDRs are defined by the IMGT Definition Scheme; or
the amino acid sequences of CDR-H1, CDR-H2, CDR-H3, CDR-L1, CDR-L2, and CDR-L3 of the 39B4 are as shown in SEQ ID NOs: 120, 123, 119, 133, 134, and 135 in order, and the CDRs are defined by the Chothia Definition Scheme; or
the amino acid sequences of CDR-H1, CDR-H2, CDR-H3, CDR-L1, CDR-L2, and CDR-L3 of the 39B4 are as shown in SEQ ID NOs: 117, 124, 125, 137, 138, and 139 in order, and the CDRs are defined by the Contact Definition Scheme; or
the amino acid sequences of CDR-H1, CDR-H2, CDR-H3, CDR-L1, CDR-L2, and CDR-L3 of the 39B4 are as shown in SEQ ID NOs: 126, 127, 128, 133, 134, and 135 in order, and the CDRs are defined by the Abm Definition Scheme.

Preferably,
m1) the amino acid sequences of CDR-H1, CDR-H2, CDR-H3, CDR-L1, CDR-L2, and CDR-L3 of the B-8 are as shown in SEQ ID NOs: 33, 34, 35, 50, 51, and 52 in order, and the CDRs are defined by the Kabat Definition Scheme (corresponding to B-8 in Table 17-8); or
m2) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m1): Y33E in SEQ ID NO: 32 (i.e., Y at position 33 of SEQ ID NO: 32 is mutated to E (i.e., Y at position 33 is substituted by E, same applies below), Y at position 4 of CDR-H1 is mutated to E, other CDRs are unchanged, corresponding to B-8-3 mutant in Table 17-8), and the CDRs are defined by the Kabat Definition Scheme; or
m3) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m1): Y61E in SEQ ID NO: 32 (i.e., Y at position 61 of SEQ ID NO: 32 is mutated to E, Y at position 12 of CDR-H2 is mutated to E, other CDRs are unchanged, corresponding to B-8-4 mutant in Table 17-8), and the CDRs are defined by the Kabat Definition Scheme; or
m4) the amino acid sequences of CDR-H1, CDR-H2, CDR-H3, CDR-L1, CDR-L2, and CDR-L3 of the B-8 have the following mutations compared to m1): F111A in SEQ ID NO: 32 (i.e., F at position 111 of SEQ ID NO: 32 is mutated to A, F at position 13 of CDR-H3 is mutated to A, other CDRs are unchanged, corresponding to B-8-5 mutant in Table 17-8), and the CDRs are defined by the Kabat Definition Scheme; or
m5) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m1): Y93E in SEQ ID NO: 49 (i.e., Y at position 93 of SEQ ID NO: 49 is mutated to E, Y at position 5 of CDR-L3 is mutated to E, other CDRs are unchanged, corresponding to B-8-1 mutant in Table 17-8), and the CDRs are defined by the Kabat Definition Scheme; or
m6) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m1): S91R in SEQ ID NO: 49 (i.e., S at position 91 of SEQ ID NO: 49 is mutated to R, S at position 3 of CDR-L3 is mutated to R, other CDRs are unchanged, corresponding to B-8-2 mutant in Table 17-8), and the CDRs are defined by the Kabat Definition Scheme; or
m7) the amino acid sequences of CDR-H1, CDR-H2, CDR-H3, CDR-L1, and CDR-L3 of the B-8 are as shown in SEQ ID NOs: 36, 37, 38, 53, and 52 in order, and the amino acid sequence of CDR-L2 is GAS, and the CDRs are defined by the IMGT Definition Scheme (corresponding to B-8, and B-8-4 in Table 17-8); or
m8) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m7): Y33E in SEQ ID NO: 32 (i.e., Y at position 33 of SEQ ID NO: 32 is mutated to E, Y at position 9 of CDR-H1 is mutated to E, other CDRs are unchanged, corresponding to B-8-3 mutant in Table 17-8), and the CDRs are defined by the IMGT Definition Scheme; or
m9) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m7): F111A in SEQ ID NO: 32 (i.e., F at position 111 of SEQ ID NO: 32 is mutated to A, F at position 15 of CDR-H3 is mutated to A, other CDRs are unchanged, corresponding to B-8-5 mutant in Table 17-8), and the CDRs are defined by the IMGT Definition Scheme; or
m10) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m7): Y93E in SEQ ID NO: 49 (i.e., Y at position 93 of SEQ ID NO: 49 is mutated to E, Y at position 5 of CDR-L3 is mutated to E, other CDRs are unchanged, corresponding to B-8-1 mutant in Table 17-8), and the CDRs are defined by the IMGT Definition Scheme; or
m11) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m7): S91R in SEQ ID NO: 49 (i.e., S at position 91 of SEQ ID NO: 49 is mutated to R, S at position 3 of CDR-L3 is mutated to R, other CDRs are unchanged, corresponding to B-8-2 mutant in Table 17-8), and the CDRs are defined by the IMGT Definition Scheme; or
m12) the amino acid sequences of CDR-H1, CDR-H2, CDR-H3, CDR-L1, CDR-L2, and CDR-L3 of the B-8 are as shown in SEQ ID NOs: 50, 51, 52, 67, 68, and 63 in order, and the CDRs are defined by the Chothia Definition Scheme (corresponding to B-8, B-8-3, and B-8-4 in Table 17-8); or
m13) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m12): F111A in SEQ ID NO: 32 (i.e., F at position **111** of SEQ ID NO: 32 is mutated to A, F at position 13 of CDR-H3 is mutated to A, other CDRs are unchanged, corresponding to B-8-5 mutant in Table 17-8), and the CDRs are defined by the Chothia Definition Scheme; or
m14) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m12): Y93E in SEQ ID NO: 49 (i.e., Y at position 93 of SEQ ID NO: 49 is mutated to E, Y at position 5 of CDR-L3 is mutated to E, other CDRs are unchanged, corresponding to B-8-1 mutant in Table 17-8), and the CDRs are defined by the Chothia Definition Scheme; or
m15) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m12): S91R in SEQ ID NO: 49 (i.e., S at position 91 of SEQ ID NO: 49 is mutated to R, S at position 3 of CDR-L3 is mutated to R, other CDRs are unchanged, corresponding to B-8-2 mutant in Table 17-8), and the CDRs are defined by the Chothia Definition Scheme; or
m16) the amino acid sequences of CDR-H1, CDR-H2, CDR-H3, CDR-L1, CDR-L2, and CDR-L3 of the B-8 are as shown in SEQ ID NOs: 41, 42, 43, 54, 55, and 56 in order, and the CDRs are defined by the Contact Definition Scheme (corresponding to B-8, and B-8-4 in Table 17-8); or
m17) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m16): Y33E in SEQ ID NO: 32 (i.e., Y at position 33 of SEQ ID NO: 32 is mutated to E, Y at position 5 of CDR-H1 is mutated to E, other CDRs are unchanged, corresponding to B-8-3 mutant in Table 17-8), and the CDRs are defined by the Contact Definition Scheme; or
m18) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m16): F111A in SEQ ID NO: 32 (i.e., F at position 111 of SEQ ID NO: 32 is mutated to A, F at position 15 of CDR-H3 is mutated to A, other CDRs are unchanged, corresponding to B-8-5 mutant in Table 17-8), and the CDRs are defined by the Contact Definition Scheme; or
m19) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m16): Y93E in SEQ ID NO: 49 (i.e., Y at position 93 of SEQ ID NO: 49 is mutated to E, Y at position 5 of CDR-L3 is mutated to E, other CDRs are unchanged, corresponding to B-8-1 mutant in Table 17-8), and the CDRs are defined by the Contact Definition Scheme; or
m20) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m16): S91R in SEQ ID NO: 49 (i.e., S at position 91 of SEQ ID NO: 49 is mutated to R, S at position 3 of CDR-L3 is mutated to R, other CDRs are unchanged, corresponding to B-8-2 mutant in Table 17-8), and the CDRs are defined by the Contact Definition Scheme; or
m21) the amino acid sequences of CDR-H1, CDR-H2, CDR-H3, CDR-L1, CDR-L2, and CDR-L3 of the B-8 are as shown in SEQ ID NOs: 44, 45, 35, 50, 51, and 52 in order, and the CDRs are defined by the Abm Definition Scheme (corresponding to B-8, and B-8-4 in Table 17-8); or
m22) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m21): Y33E in SEQ ID NO: 32 (i.e., Y at position 33 of SEQ ID NO: 32 is mutated to E, Y at position 9 of CDR-H1 is mutated to E, other CDRs are unchanged, corresponding to B-8-3 mutant in Table 17-8), and the CDRs are defined by the Abm Definition Scheme; or
m23) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m21): F111A in SEQ ID NO: 32 (i.e., F at position 111 of SEQ ID NO: 32 is mutated to A, F at position 13 of CDR-H3 is mutated to A, other CDRs are unchanged, corresponding to B-8-5 mutant in Table 17-8), and the CDRs are defined by the Abm Definition Scheme; or
m24) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m21): Y93E in SEQ ID NO: 49 (i.e., Y at position 93 of SEQ ID NO: 49 is mutated to E, Y at position 5 of CDR-L3 is mutated to E, other CDRs are unchanged, corresponding to B-8-1 mutant in Table 17-8), and the CDRs are defined by the Abm Definition Scheme; or
m25) the amino acid sequences of CDR-H1, CDR-H2, CDR-H3, CDR-L1, CDR-L2, and CDR-L3 of the B-8 have the following mutations compared to CDR-H1, CDR-H2, CDR-H3, CDR-L1, CDR-L2, and CDR-L3 of m21): S91R in SEQ ID NO: 49 (i.e., S at position 91 of SEQ ID NO: 49 is mutated to R, S at position 3 of CDR-L3 is mutated to R, other CDRs are unchanged, corresponding to B-8-2 mutant in Table 17-8), and the CDRs are defined by the Abm Definition Scheme.

Preferably,
n1) the amino acid sequences of CDR-H1, CDR-H2, CDR-H3, CDR-L1, CDR-L2, and CDR-L3 of the 98G10 are as shown in SEQ ID NOs: 89, 90, 91, 106, 107, and 108 in order, and the CDRs are defined by the Kabat Definition Scheme (corresponding to 98G10 in Table 17-6); or
n2) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34H in SEQ ID NO: 88 (i.e., W at position 34 of SEQ ID NO: 88 is mutated to H, W at position 4 of CDR-H1 is mutated to H, other CDRs are unchanged, corresponding to 98G10-H-1 mutant in Table 17-6), and the CDRs are defined by the Kabat Definition Scheme; or
n3) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34Y in SEQ ID NO: 88 (i.e., W at position 34 of SEQ ID NO: 88 is mutated to Y, W at position 4 of CDR-H1 is mutated to Y, other CDRs are unchanged, corresponding to 98G10-H-2 mutant in Table 17-6), and the CDRs are defined by the Kabat Definition Scheme; or
n4) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): Y112R in SEQ ID NO: 88 (i.e., Y at position 112 of SEQ ID NO: 88 is mutated to R, Y at position 13 of CDR-H3 is mutated to R, other CDRs are unchanged, corresponding to 98G10-H-3 mutant in Table 17-6), and the CDRs are defined by the Kabat Definition Scheme; or
n5) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): Y112S in SEQ ID NO: 88 (i.e., Y at position 112 of SEQ ID NO: 88 is mutated to S, Y at position 13 of CDR-H3 is mutated to S, other CDRs are unchanged, corresponding to 98G10-H-4 mutant in Table 17-6), and the CDRs are defined by the Kabat Definition Scheme; or
n6) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): Y112E in SEQ ID NO: 88 (i.e., Y at position 112 of SEQ ID NO: 88 is mutated to E, Y at position 13 of CDR-H3 is mutated to E, other CDRs are unchanged, corresponding to 98G10-H-5 mutant in Table 17-6), and the CDRs are defined by the Kabat Definition Scheme; or
n7) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): Y112Q in SEQ ID NO: 88 (i.e., Y at position 112 of SEQ ID NO: 88 is mutated to Q, Y at position 13 of CDR-H3 is mutated to Q, other CDRs are unchanged, corresponding to 98G10-H-6 mutant in Table 17-6), and the CDRs are defined by the Kabat Definition Scheme; or
n8) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34R and Y112R in SEQ ID NO: 88 (i.e., W at position 34 of SEQ ID NO: 88 is mutated to R, Y at position 112 of SEQ ID NO: 88 is mutated to R, W at position 4 of CDR-H1 is mutated to R, Y at position 13 of CDR-H3 is mutated to R, other CDRs are unchanged, corresponding to 98G10-H-7 mutant in Table 17-6), and the CDRs are defined by the Kabat Definition Scheme; or
n9) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34D and Y112R in SEQ ID NO: 88 (i.e., W at position 34 of SEQ ID NO: 88 is mutated to D, Y at position 112 of SEQ ID NO: 88 is mutated to R, W at position 4 of CDR-H1 is mutated to D, Y at position 13 of CDR-H3 is mutated to R, other CDRs are unchanged, corresponding to 98G10-H-8 mutant in Table 17-6), and the CDRs are defined by the Kabat Definition Scheme; or
n10) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34H and Y112L in SEQ ID NO: 88 (i.e., W at position 34 of SEQ ID NO: 88 is mutated to H, Y at position 112 of SEQ ID NO: 88 is mutated to L, W at position 4 of CDR-H1 is mutated to H, Y at position 13 of CDR-H3 is mutated to L, other CDRs are unchanged, corresponding to 98G10-H-9 mutant in Table 17-6), and the CDRs are defined by the Kabat Definition Scheme; or
n11) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34T and Y112R in SEQ ID NO: 88 (i.e., W at position 34 of SEQ ID NO: 88 is mutated to T, Y at position 112 of SEQ ID NO: 88 is mutated to R, W at position 4 of CDR-H1 is mutated to T, Y at position 13 of CDR-H3 is mutated to R, other CDRs are unchanged, corresponding to 98G10-H-10 mutant in Table 17-6), and the CDRs are defined by the Kabat Definition Scheme; or
n12) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34H and Y112R in SEQ ID NO: 88 (i.e., W at position 34 of SEQ ID NO: 88 is mutated to H, Y at position 112 of SEQ ID NO: 88 is mutated to R, W at position 4 of CDR-H1 is mutated to H, Y at position 13 of CDR-H3 is mutated to R, other CDRs are unchanged, corresponding to 98G10-H-11 mutant in Table 17-6), and the CDRs are defined by the Kabat Definition Scheme; or
n13) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34H and Y112S in SEQ ID NO: 88 (i.e., W at position 34 of SEQ ID NO: 88 is mutated to H, Y at position 112 of SEQ ID NO: 88 is mutated to S, W at position 4 of CDR-H1 is mutated to H, Y at position 13 of CDR-H3 is mutated to S, other CDRs are unchanged, corresponding to 98G10-H-12 mutant in Table 17-6), and the CDRs are defined by the Kabat Definition Scheme; or
n14) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34K and Y112S in SEQ ID NO: 88 (i.e., W at position 34 of SEQ ID NO: 88 is mutated to K, Y at position 112 of SEQ ID NO: 88 is mutated to S, W at position 4 of CDR-H1 is mutated to K, Y at position 13 of CDR-H3 is mutated to S, other CDRs are unchanged, corresponding to 98G10-H-13 mutant in Table 17-6), and the CDRs are defined by the Kabat Definition Scheme; or
n15) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): W34D and Y112S in SEQ ID NO: 88 (i.e., W at position 34 of SEQ ID NO: 88 is mutated to D, Y at position 112 of SEQ ID NO: 88 is mutated to S, W at position 4 of CDR-H1 is mutated to D, Y at position 13 of CDR-H3 is mutated to S, other CDRs are unchanged, corresponding to 98G10-H-14 mutant in Table 17-6), and the CDRs are defined by the Kabat Definition Scheme; or
n16) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): Y93R in SEQ ID NO: 105 (i.e., Y at position 93 of SEQ ID NO: 105 is mutated to R, Y at position 5 of CDR-L3 is mutated to R, other CDRs are unchanged, corresponding to 98G10-L-1 mutant in Table 17-6), and the CDRs are defined by the Kabat Definition Scheme; or
n17) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): Y93S in SEQ ID NO: 105 (i.e., Y at position 93 of SEQ ID NO: 105 is mutated to S, Y at position 5 of CDR-L3 is mutated to S, other CDRs are unchanged, corresponding to 98G10-L-2 mutant in Table 17-6), and the CDRs are defined by the Kabat Definition Scheme; or
n18) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n1): Y93Q in SEQ ID NO: 105 (i.e., Y at position 93 of SEQ ID NO: 105 is mutated to Q, Y at position 5 of CDR-L3 is mutated to Q, other CDRs are unchanged, corresponding to 98G10-L-3 mutant in Table 17-6), and the CDRs are defined by the Kabat Definition Scheme; or
n20) the amino acid sequences of CDR-H1, CDR-H2, CDR-H3, CDR-L1, and CDR-L3 of the 98G10 are as shown in SEQ ID NOs: 92, 93, 94, 109, and 108 in order, and the amino acid sequence of CDR-L2 is TAS, and the CDRs are defined by the IMGT Definition Scheme (corresponding to 98G10 in Table 17-6); or
n21) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34H in SEQ ID NO: 88 (i.e., W at position 34 of SEQ ID NO: 88 is mutated to H, W at position 9 of CDR-H1 is mutated to H, other CDRs are unchanged, corresponding to 98G10-H-1 mutant in Table 17-6), and the CDRs are defined by the IMGT Definition Scheme; or
n22) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34Y in SEQ ID NO: 88 (i.e., W at position 34 of SEQ ID NO: 88 is mutated to Y, W at position 9 of CDR-H1 is mutated to Y, other CDRs are unchanged, corresponding to 98G10-H-2 mutant in Table 17-6), and the CDRs are defined by the IMGT Definition Scheme; or
n23) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): Y112R in SEQ ID NO: 88 (i.e., Y at position 112 of SEQ ID NO: 88 is mutated to R, Y at position 15 of CDR-H3 is mutated to R, other CDRs are unchanged, corresponding to 98G10-H-3 mutant in Table 17-6), and the CDRs are defined by the IMGT Definition Scheme; or
n24) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): Y112S in SEQ ID NO: 88 (i.e., Y at position 112 of SEQ ID NO: 88 is mutated to S, Y at position 15 of CDR-H3 is mutated to S, other CDRs are unchanged, corresponding to 98G10-H-4 mutant in Table 17-6), and the CDRs are defined by the IMGT Definition Scheme; or
n25) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): Y112E in SEQ ID NO: 88 (i.e., Y at position 112 of SEQ ID NO: 88 is mutated to E, Y at position 15 of CDR-H3 is mutated to E, other CDRs are unchanged, corresponding to 98G10-H-5 mutant in Table 17-6), and the CDRs are defined by the IMGT Definition Scheme; or
n26) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): Y112Q in SEQ ID NO: 88 (i.e., Y at position 112 of SEQ ID NO: 88 is mutated to Q, Y at position 15 of CDR-H3 is mutated to Q, other CDRs are unchanged, corresponding to 98G10-H-6 mutant in Table 17-6), and the CDRs are defined by the IMGT Definition Scheme; or
n27) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34R and Y112R in SEQ ID NO: 88 (i.e., W at position 34 of SEQ ID NO: 88 is mutated to R, Y at position 112 of SEQ ID NO: 88 is mutated to R, W at position 9 of CDR-H1 is mutated to R, Y at position 15 of CDR-H3 is mutated to R, other CDRs are unchanged, corresponding to 98G10-H-7 mutant in Table 17-6), and the CDRs are defined by the IMGT Definition Scheme; or
n28) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34D and Y112R in SEQ ID NO: 88 (i.e., W at position 34 of SEQ ID NO: 88 is mutated to D, Y at position 112 of SEQ ID NO: 88 is mutated to R, W at position 9 of CDR-H1 is mutated to D, Y at position 15 of CDR-H3 is mutated to R, other CDRs are unchanged, corresponding to 98G10-H-8 mutant in Table 17-6), and the CDRs are defined by the IMGT Definition Scheme; or
n29) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34H and Y112L in SEQ ID NO: 88 (i.e., W at position 34 of SEQ ID NO: 88 is mutated to H, Y at position 112 of SEQ ID NO: 88 is mutated to L, W at position 9 of CDR-H1 is mutated to H, Y at position 15 of CDR-H3 is mutated to L, other CDRs are unchanged, corresponding to 98G10-H-9 mutant in Table 17-6), and the CDRs are defined by the IMGT Definition Scheme; or
n30) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34T and Y112R in SEQ ID NO: 88 (i.e., W at position 34 of SEQ ID NO: 88 is mutated to T, Y at position 112 of SEQ ID NO: 88 is mutated to R, W at position 9 of CDR-H1 is mutated to T, Y at position 15 of CDR-H3 is mutated to R, other CDRs are unchanged, corresponding to 98G10-H-10 mutant in Table 17-6), and the CDRs are defined by the IMGT Definition Scheme; or
n31) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34H and Y112R in SEQ ID NO: 88 (i.e., W at position 34 of SEQ ID NO: 88 is mutated to H, Y at position 112 of SEQ ID NO: 88 is mutated to R, W at position 9 of CDR-H1 is mutated to H, Y at position 15 of CDR-H3 is mutated to R, other CDRs are unchanged, corresponding to 98G10-H-11 mutant in Table 17-6), and the CDRs are defined by the IMGT Definition Scheme; or
n32) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34H and Y112S in SEQ ID NO: 88 (i.e., W at position 34 of SEQ ID NO: 88 is mutated to H, Y at position 112 of SEQ ID NO: 88 is mutated to S, W at position 9 of CDR-H1 is mutated to H, Y at position 15 of CDR-H3 is mutated to S, other CDRs are unchanged, corresponding to 98G10-H-12 mutant in Table 17-6), and the CDRs are defined by the IMGT Definition Scheme; or
n33) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34K and Y112S in SEQ ID NO: 88 (i.e., W at position 34 of SEQ ID NO: 88 is mutated to K, Y at position 112 of SEQ ID NO: 88 is mutated to S, W at position 9 of CDR-H1 is mutated to K, Y at position 15 of CDR-H3 is mutated to S, other CDRs are unchanged, corresponding to 98G10-H-13 mutant in Table 17-6), and the CDRs are defined by the IMGT Definition Scheme; or
n34) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): W34D and Y112S in SEQ ID NO: 88 (i.e., W at position 34 of SEQ ID NO: 88 is mutated to D, Y at position 112 of SEQ ID NO: 88 is mutated to S, W at position 9 of CDR-H1 is mutated to D, Y at position 15 of CDR-H3 is mutated to S, other CDRs are unchanged, corresponding to 98G10-H-14 mutant in Table 17-6), and the CDRs are defined by the IMGT Definition Scheme; or
n35) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): Y93R in SEQ ID NO: 105 (i.e., Y at position 93 of SEQ ID NO: 105 is mutated to R, Y at position 5 of CDR-L3 is mutated to R, other CDRs are unchanged, corresponding to 98G10-L-1 mutant in Table 17-6), and the CDRs are defined by the IMGT Definition Scheme; or
n36) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): Y93S in SEQ ID NO: 105 (i.e., Y at position 93 of SEQ ID NO: 105 is mutated to S, Y at position 5 of CDR-L3 is mutated to S, other CDRs are unchanged, corresponding to 98G10-L-2 mutant in Table 17-6), and the CDRs are defined by the IMGT Definition Scheme; or
n37) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n20): Y93Q in SEQ ID NO: 105 (i.e., Y at position 93 of SEQ ID NO: 105 is mutated to Q, Y at position 5 of CDR-L3 is mutated to Q, other CDRs are unchanged, corresponding to 98G10-L-3 mutant in Table 17-6), and the CDRs are defined by the IMGT Definition Scheme; or
n39) the amino acid sequences of CDR-H1, CDR-H2, CDR-H3, CDR-L1, CDR-L2, and CDR-L3 of the 98G10 are as shown in SEQ ID NOs: 95, 96, 91, 106, 107, and 108 in order, and the CDRs are defined by the Chothia Definition Scheme (corresponding to 98G10, 98G10-H-1, and 98G10-H-2 in Table 17-6); or
n40) the amino acid sequences of CDR-H1, CDR-H2, CDR-H3, CDR-L1, CDR-L2, and CDR-L3 of the 98G10 have the following mutations compared to n39): Y112R in SEQ ID NO: 88 (i.e., Y at position 112 of SEQ ID NO: 88 is mutated to R, Y at position 13 of CDR-H3 is mutated to R, other CDRs are unchanged, corresponding to 98G10-H-3, 98G10-H-7, 98G10-H-8, 98G10-H-10, and 98G10-H-11 mutants in Table 17-6), and the CDRs are defined by the Chothia Definition Scheme; or
n41) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n39): Y112S in SEQ ID NO: 88 (i.e., Y at position 112 of SEQ ID NO: 88 is mutated to S, Y at position 13 of CDR-H3 is mutated to S, other CDRs are unchanged, corresponding to 98G10-H-4, 98G10-H-12, 98G10-H-13, and 98G10-H-14 mutants in Table 17-6), and the CDRs are defined by the Chothia Definition Scheme; or
n42) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n39): Y112E in SEQ ID NO: 88 (i.e., Y at position 112 of SEQ ID NO: 88 is mutated to E, Y at position 13 of CDR-H3 is mutated to E, other CDRs are unchanged, corresponding to 98G10-H-5 mutant in Table 17-6), and the CDRs are defined by the Chothia Definition Scheme; or
n43) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n39): Y112Q in SEQ ID NO: 88 (i.e., Y at position 112 of SEQ ID NO: 88 is mutated to Q, Y at position 13 of CDR-H3 is mutated to Q, other CDRs are unchanged, corresponding to 98G10-H-6 mutant in Table 17-6), and the CDRs are defined by the Chothia Definition Scheme; or
n44) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n39): Y112L in SEQ ID NO: 88 (i.e., Y at position 112 of SEQ ID NO: 88 is mutated to L, Y at position 13 of CDR-H3 is mutated to L, other CDRs are unchanged, corresponding to 98G10-H-9 mutant in Table 17-6), and the CDRs are defined by the Chothia Definition Scheme; or
n45) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n39): Y93R in SEQ ID NO: 105 (i.e., Y at position 93 of SEQ ID NO: 105 is mutated to R, Y at position 5 of CDR-L3 is mutated to R, other CDRs are unchanged, corresponding to 98G10-L-1 mutant in Table 17-6), and the CDRs are defined by the Chothia Definition Scheme; or
n46) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n39): Y93S in SEQ ID NO: 105 (i.e., Y at position 93 of SEQ ID NO: 105 is mutated to S, Y at position 5 of CDR-L3 is mutated to S, other CDRs are unchanged, corresponding to 98G10-L-2 mutant in Table 17-6), and the CDRs are defined by the Chothia Definition Scheme; or
n47) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n39): Y93Q in SEQ ID NO: 105 (i.e., Y at position 93 of SEQ ID NO: 105 is mutated to Q, Y at position 5 of CDR-L3 is mutated to Q, other CDRs are unchanged, corresponding to 98G10-L-3 mutant in Table 17-6), and the CDRs are defined by the Chothia Definition Scheme; or
n48) the amino acid sequences of CDR-H1, CDR-H2, CDR-H3, CDR-L1, CDR-L2, and CDR-L3 of the 98G10 are as shown in SEQ ID NOs: 97, 98, 99, 110, **111,** and 112 in order, and the CDRs are defined by the Contact Definition Scheme (corresponding to 98G10 in Table 17-6); or
n49) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34H in SEQ ID NO: 88 (i.e., W at position 34 of SEQ ID NO: 88 is mutated to H, W at position 5 of CDR-H1 is mutated to H, other CDRs are unchanged, corresponding to 98G10-H-1 mutant in Table 17-6), and the CDRs are defined by the Contact Definition Scheme; or
n50) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34Y in SEQ ID NO: 88 (i.e., W at position 34 of SEQ ID NO: 88 is mutated to Y, W at position 5 of CDR-H1 is mutated to Y, other CDRs are unchanged, corresponding to 98G10-H-2 mutant in Table 17-6), and the CDRs are defined by the Contact Definition Scheme; or
n51) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): Y112R in SEQ ID NO: 88 (i.e., Y at position 112 of SEQ ID NO: 88 is mutated to R, Y at position 15 of CDR-H3 is mutated to R, other CDRs are unchanged, corresponding to 98G10-H-3 mutant in Table 17-6), and the CDRs are defined by the Contact Definition Scheme; or
n52) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): Y112S in SEQ ID NO: 88 (i.e., Y at position 112 of SEQ ID NO: 88 is mutated to S, Y at position 15 of CDR-H3 is mutated to S, other CDRs are unchanged, corresponding to 98G10-H-4 mutant in Table 17-6), and the CDRs are defined by the Contact Definition Scheme; or
n53) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): Y112E in SEQ ID NO: 88 (i.e., Y at position 112 of SEQ ID NO: 88 is mutated to E, Y at position 15 of CDR-H3 is mutated to E, other CDRs are unchanged, corresponding to 98G10-H-5 mutant in Table 17-6), and the CDRs are defined by the Contact Definition Scheme; or
n54) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): Y112Q in SEQ ID NO: 88 (i.e., Y at position 112 of SEQ ID NO: 88 is mutated to Q, Y at position 15 of CDR-H3 is mutated to Q, other CDRs are unchanged, corresponding to 98G10-H-6 mutant in Table 17-6), and the CDRs are defined by the Contact Definition Scheme; or
n55) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34R and Y112R in SEQ ID NO: 88 (i.e., W at position 34 of SEQ ID NO: 88 is mutated to R, Y at position 112 of SEQ ID NO: 88 is mutated to R, W at position 5 of CDR-H1 is mutated to R, Y at position 15 of CDR-H3 is mutated to R, other CDRs are unchanged, corresponding to 98G10-H-7 mutant in Table 17-6), and the CDRs are defined by the Contact Definition Scheme; or
n56) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34D and Y112R in SEQ ID NO: 88 (i.e., W at position 34 of SEQ ID NO: 88 is mutated to D, Y at position 112 of SEQ ID NO: 88 is mutated to R, W at position 5 of CDR-H1 is mutated to D, Y at position 15 of CDR-H3 is mutated to R, other CDRs are unchanged, corresponding to 98G10-H-8 mutant in Table 17-6), and the CDRs are defined by the Contact Definition Scheme; or
n57) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34H and Y112L in SEQ ID NO: 88 (i.e., W at position 34 of SEQ ID NO: 88 is mutated to H, Y at position 112 of SEQ ID NO: 88 is mutated to L, W at position 5 of CDR-H1 is mutated to H, Y at position 15 of CDR-H3 is mutated to L, other CDRs are unchanged, corresponding to 98G10-H-9 mutant in Table 17-6), and the CDRs are defined by the Contact Definition Scheme; or
n58) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34T and Y112R in SEQ ID NO: 88 (i.e., W at position 34 of SEQ ID NO: 88 is mutated to T, Y at position 112 of SEQ ID NO: 88 is mutated to R, W at position 5 of CDR-H1 is mutated to T, Y at position 15 of CDR-H3 is mutated to R, other CDRs are unchanged, corresponding to 98G10-H-10 mutant in Table 17-6), and the CDRs are defined by the Contact Definition Scheme; or
n59) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34H and Y112R in SEQ ID NO: 88 (i.e., W at position 34 of SEQ ID NO: 88 is mutated to H, Y at position 112 of SEQ ID NO: 88 is mutated to R, W at position 5 of CDR-H1 is mutated to H, Y at position 15 of CDR-H3 is mutated to R, other CDRs are unchanged, corresponding to 98G10-H-11 mutant in Table 17-6), and the CDRs are defined by the Contact Definition Scheme; or
n60) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34H and Y112S in SEQ ID NO: 88 (i.e., W at position 34 of SEQ ID NO: 88 is mutated to H, Y at position 112 of SEQ ID NO: 88 is mutated to S, W at position 5 of CDR-H1 is mutated to H, Y at position 15 of CDR-H3 is mutated to S, other CDRs are unchanged, corresponding to 98G10-H-12 mutant in Table 17-6), and the CDRs are defined by the Contact Definition Scheme; or
n61) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34K and Y112S in SEQ ID NO: 88 (i.e., W at position 34 of SEQ ID NO: 88 is mutated to K, Y at position 112 of SEQ ID NO: 88 is mutated to S, W at position 5 of CDR-H1 is mutated to K, Y at position 15 of CDR-H3 is mutated to S, other CDRs are unchanged, corresponding to 98G10-H-13 mutant in Table 17-6), and the CDRs are defined by the Contact Definition Scheme; or
n62) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): W34D and Y112S in SEQ ID NO: 88 (i.e., W at position 34 of SEQ ID NO: 88 is mutated to D, Y at position 112 of SEQ ID NO: 88 is mutated to S, W at position 5 of CDR-H1 is mutated to D, Y at position 15 of CDR-H3 is mutated to S, other CDRs are unchanged, corresponding to 98G10-H-14 mutant in Table 17-6), and the CDRs are defined by the Contact Definition Scheme; or
n63) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): Y93R in SEQ ID NO: 105 (i.e., Y at position 93 of SEQ ID NO: 105 is mutated to R, Y at position 5 of CDR-L3 is mutated to R, other CDRs are unchanged, corresponding to 98G10-L-1 mutant in Table 17-6), and the CDRs are defined by the Contact Definition Scheme; or
n64) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): Y93S in SEQ ID NO: 105 (i.e., Y at position 93 of SEQ ID NO: 105 is mutated to S, Y at position 5 of CDR-L3 is mutated to S, other CDRs are unchanged, corresponding to 98G10-L-2 mutant in Table 17-6), and the CDRs are defined by the Contact Definition Scheme; or
n65) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n48): Y93Q in SEQ ID NO: 105 (i.e., Y at position 93 of SEQ ID NO: 105 is mutated to Q, Y at position 5 of CDR-L3 is mutated to Q, other CDRs are unchanged, corresponding to 98G10-L-3 mutant in Table 17-6), and the CDRs are defined by the Contact Definition Scheme; or
n66) the amino acid sequences of CDR-H1, CDR-H2, CDR-H3, CDR-L1, CDR-L2, and CDR-L3 of the 98G10 are as shown in SEQ ID NO: 100, 101, 91, 106, 107, and 108 in order, and the CDRs are defined by the Abm Definition Scheme (corresponding to 98G10 in Table 17-6); or
n67) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34H in SEQ ID NO: 88 (i.e., W at position 34 of SEQ ID NO: 88 is mutated to H, W at position 9 of CDR-H1 is mutated to H, other CDRs are unchanged, corresponding to 98G10-H-1 mutant in Table 17-6), and the CDRs are defined by the Abm Definition Scheme; or
n68) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34Y in SEQ ID NO: 88 (i.e., W at position 34 of SEQ ID NO: 88 is mutated to Y, W at position 9 of CDR-H1 is mutated to Y, other CDRs are unchanged, corresponding to 98G10-H-2 mutant in Table 17-6), and the CDRs are defined by the Abm Definition Scheme; or
n69) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): Y112R in SEQ ID NO: 88 (i.e., Y at position 112 of SEQ ID NO: 88 is mutated to R, Y at position 13 of CDR-H3 is mutated to R, other CDRs are unchanged, corresponding to 98G10-H-3 mutant in Table 17-6), and the CDRs are defined by the Abm Definition Scheme; or
n70) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): Y112S in SEQ ID NO: 88 (i.e., Y at position 112 of SEQ ID NO: 88 is mutated to S, Y at position 13 of CDR-H3 is mutated to S, other CDRs are unchanged, corresponding to 98G10-H-4 mutant in Table 17-6), and the CDRs are defined by the Abm Definition Scheme; or
n71) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): Y112E in SEQ ID NO: 88 (i.e., Y at position 112 of SEQ ID NO: 88 is mutated to E, Y at position 13 of CDR-H3 is mutated to E, other CDRs are unchanged, corresponding to 98G10-H-5 mutant in Table 17-6), and the CDRs are defined by the Abm Definition Scheme; or
n72) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): Y112Q in SEQ ID NO: 88 (i.e., Y at position 112 of SEQ ID NO: 88 is mutated to Q, Y at position 13 of CDR-H3 is mutated to Q, other CDRs are unchanged, corresponding to 98G10-H-6 mutant in Table 17-6), and the CDRs are defined by the Abm Definition Scheme; or
n73) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34R and Y112R in SEQ ID NO: 88 (i.e., W at position 34 of SEQ ID NO: 88 is mutated to R, Y at position 112 of SEQ ID NO: 88 is mutated to R, W at position 9 of CDR-H1 is mutated to R, Y at position 13 of CDR-H3 is mutated to R, other CDRs are unchanged, corresponding to 98G10-H-7 mutant in Table 17-6), and the CDRs are defined by the Abm Definition Scheme; or
n74) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34D and Y112R in SEQ ID NO: 88 (i.e., W at position 34 of SEQ ID NO: 88 is mutated to D, Y at position 112 of SEQ ID NO: 88 is mutated to R, W at position 9 of CDR-H1 is mutated to D, Y at position 13 of CDR-H3 is mutated to R, other CDRs are unchanged, corresponding to 98G10-H-8 mutant in Table 17-6), and the CDRs are defined by the Abm Definition Scheme; or
n75) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34H and Y112L in SEQ ID NO: 88 (i.e., W at position 34 of SEQ ID NO: 88 is mutated to H, Y at position 112 of SEQ ID NO: 88 is mutated to L, W at position 9 of CDR-H1 is mutated to H, Y at position 13 of CDR-H3 is mutated to L, other CDRs are unchanged, corresponding to 98G10-H-9 mutant in Table 17-6), and the CDRs are defined by the Abm Definition Scheme; or
n76) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34T and Y112R in SEQ ID NO: 88 (i.e., W at position 34 of SEQ ID NO: 88 is mutated to T, Y at position 112 of SEQ ID NO: 88 is mutated to R, W at position 9 of CDR-H1 is mutated to T, Y at position 13 of CDR-H3 is mutated to R, other CDRs are unchanged, corresponding to 98G10-H-10 mutant in Table 17-6), and the CDRs are defined by the Abm Definition Scheme; or
n77) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34H and Y112R in SEQ ID NO: 88 (i.e., W at position 34 of SEQ ID NO: 88 is mutated to H, Y at position 112 of SEQ ID NO: 88 is mutated to R, W at position 9 of CDR-H1 is mutated to H, Y at position 13 of CDR-H3 is mutated to R, other CDRs are unchanged, corresponding to 98G10-H-11 mutant in Table 17-6), and the CDRs are defined by the Abm Definition Scheme; or
n78) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34H and Y112S in SEQ ID NO: 88 (i.e., W at position 34 of SEQ ID NO: 88 is mutated to H, Y at position 112 of SEQ ID NO: 88 is mutated to S, W at position 9 of CDR-H1 is mutated to H, Y at position 13 of CDR-H3 is mutated to S, other CDRs are unchanged, corresponding to 98G10-H-12 mutant in Table 17-6), and the CDRs are defined by the Abm Definition Scheme; or
n79) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34K and Y112S in SEQ ID NO: 88 (i.e., W at position 34 of SEQ ID NO: 88 is mutated to K, Y at position 112 of SEQ ID NO: 88 is mutated to S, W at position 9 of CDR-H1 is mutated to K, Y at position 13 of CDR-H3 is mutated to S, other CDRs are unchanged, corresponding to 98G10-H-13 mutant in Table 17-6), and the CDRs are defined by the Abm Definition Scheme; or
n80) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): W34D and Y112S in SEQ ID NO: 88 (i.e., W at position 34 of SEQ ID NO: 88 is mutated to D, Y at position 112 of SEQ ID NO: 88 is mutated to S, W at position 9 of CDR-H1 is mutated to D, Y at position 13 of CDR-H3 is mutated to S, other CDRs are unchanged, corresponding to 98G10-H-14 mutant in Table 17-6), and the CDRs are defined by the Abm Definition Scheme; or
n81) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): Y93R in SEQ ID NO: 105 (i.e., Y at position 93 of SEQ ID NO: 105 is mutated to R, Y at position 5 of CDR-L3 is mutated to R, other CDRs are unchanged, corresponding to 98G10-L-1 mutant in Table 17-6), and the CDRs are defined by the Abm Definition Scheme; or
n82) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): Y93S in SEQ ID NO: 105 (i.e., Y at position 93 of SEQ ID NO: 105 is mutated to S, Y at position 5 of CDR-L3 is mutated to S, other CDRs are unchanged, corresponding to 98G10-L-2 mutant in Table 17-6), and the CDRs are defined by the Abm Definition Scheme; or
n83) the amino acid sequences of CDR-H1, CDR-H2, CDR-H3, CDR-L1, CDR-L2, and CDR-L3 of the 98G10 have the following mutations compared to CDR-H1, CDR-H2, CDR-H3, CDR-L1, CDR-L2, and CDR-L3 of n66): Y93Q in SEQ ID NO: 105 (i.e., Y at position 93 of SEQ ID NO: 105 is mutated to Q, Y at position 5 of CDR-L3 is mutated to Q, other CDRs are unchanged, corresponding to 98G10-L-3 mutant in Table 17-6), and the CDRs are defined by the Abm Definition Scheme.

Preferably, the amino acid sequence of the heavy chain variable region of the 94C4 comprises:
a1111) SEQ ID NO: 4; or
a2111) an amino acid sequence derived by substituting and/or deleting and/or adding one or several amino acids of the amino acid sequence described in a1111) while having the same function as the protein of the amino acid sequence described in a1111); or
a3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in a1111) while having the same function as the protein of the amino acid sequence described in a1111); and/or
the amino acid sequence of the light chain variable region of the 94C4 comprises:
   b1111) SEQ ID NO: 21; or
   b2111) an amino acid sequence derived by substituting and/or deleting and/or adding one or several amino acids of the amino acid sequence described in b1111) while having the same function as the protein of the amino acid sequence described in b1111); or
   b3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in b1111) while having the same function as the protein of the amino acid sequence described in b1111).

Preferably, the amino acid sequence of the heavy chain variable region of the B-8 comprises:
c1111) SEQ ID NO: 32; or
c2111) an amino acid sequence derived by substituting and/or deleting and/or adding one or several amino acids of the amino acid sequence described in c1111) while having the same function as the protein of the amino acid sequence described in c1111); or
c3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in c1111) while having the same function as the protein of the amino acid sequence described in c1111); and/or
the amino acid sequence of the light chain variable region of the B-8 comprises:
   d1111) SEQ ID NO: 49; or
   d2111) an amino acid sequence derived by substituting and/or deleting and/or adding one or several amino acids of the amino acid sequence described in d1111) while having the same function as the protein of the amino acid sequence described in d1111); or
   d3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in d1111) while having the same function as the protein of the amino acid sequence described in d1111).

Preferably, the amino acid sequence of the heavy chain variable region of the B-8 comprises:
c1111) SEQ ID NO: 32; or
c2111) an amino acid sequence derived by substituting and/or deleting and/or adding one or several amino acids of the amino acid sequence described in c1111) while having the same function as the protein of the amino acid sequence described in c1111); or
c3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in c1111) while having the same function as the protein of the amino acid sequence described in c1111); and/or
the amino acid sequence of the light chain variable region of the B-8 comprises:
   d1111) an amino acid sequence having the following mutation compared to SEQ ID NO: 49: Y93E; or
   d2111) an amino acid sequence derived by substituting and/or deleting and/or adding one or several amino acids of the amino acid sequence described in d1111) while having the same function as the protein of the amino acid sequence described in d1111); or
   d3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in d1111) while having the same function as the protein of the amino acid sequence described in d1111).

Preferably, the amino acid sequence of the heavy chain variable region of the B-8 comprises:
c1111) SEQ ID NO: 32; or
c2111) an amino acid sequence derived by substituting and/or deleting and/or adding one or several amino acids of the amino acid sequence described in c1111) while having the same function as the protein of the amino acid sequence described in c1111); or
c3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in c1111) while having the same function as the protein of the amino acid sequence described in c1111); and/or
the amino acid sequence of the light chain variable region of the B-8 comprises:
   d1111) an amino acid sequence having the following mutation compared to SEQ ID NO: 49: S91R; or
   d2111) an amino acid sequence derived by substituting and/or deleting and/or adding one or several amino acids of the amino acid sequence described in d1111) while having the same function as the protein of the amino acid sequence described in d1111); or
   d3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in d1111) while having the same function as the protein of the amino acid sequence described in d1111).

Preferably, the amino acid sequence of the heavy chain variable region of the B-8 comprises:
c1111) an amino acid sequence having the following mutation compared to SEQ ID NO: 32: Y33E; or
c2111) an amino acid sequence derived by substituting and/or deleting and/or adding one or several amino acids of the amino acid sequence described in c1111) while having the same function as the protein of the amino acid sequence described in c1111); or
c3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in c1111) while having the same function as the protein of the amino acid sequence described in c1111); and/or
the amino acid sequence of the light chain variable region of the B-8 comprises:
   d1111) SEQ ID NO: 49; or
   d2111) an amino acid sequence derived by substituting and/or deleting and/or adding one or several amino acids of the amino acid sequence described in d1111) while having the same function as the protein of the amino acid sequence described in d1111); or
   d3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in d1111) while having the same function as the protein of the amino acid sequence described in d1111).

Preferably, the amino acid sequence of the heavy chain variable region of the B-8 comprises:
c1111) an amino acid sequence having the following mutation compared to SEQ ID NO: 32: Y61E; or
c2111) an amino acid sequence derived by substituting and/or deleting and/or adding one or several amino acids of the amino acid sequence described in c1111) while having the same function as the protein of the amino acid sequence described in c1111); or
c3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in c1111) while having the same function as the protein of the amino acid sequence described in c1111); and/or
the amino acid sequence of the light chain variable region of the B-8 comprises:
   d1111) SEQ ID NO: 49; or
   d2111) an amino acid sequence derived by substituting and/or deleting and/or adding one or several amino acids of the amino acid sequence described in d1111) while having the same function as the protein of the amino acid sequence described in d1111); or
   d3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in d1111) while having the same function as the protein of the amino acid sequence described in d1111).

Preferably, the amino acid sequence of the heavy chain variable region of the B-8 comprises:
c1111) an amino acid sequence having the following mutation compared to SEQ ID NO: 32: F111A; or
c2111) an amino acid sequence derived by substituting and/or deleting and/or adding one or several amino acids of the amino acid sequence described in c1111) while having the same function as the protein of the amino acid sequence described in c1111); or
c3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in c1111) while having the same function as the protein of the amino acid sequence described in c1111); and/or
the amino acid sequence of the light chain variable region of the B-8 comprises:
   d1111) SEQ ID NO: 49; or
   d2111) an amino acid sequence derived by substituting and/or deleting and/or adding one or several amino acids of the amino acid sequence described in d1111) while having the same function as the protein of the amino acid sequence described in d1111); or
   d3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in d1111) while having the same function as the protein of the amino acid sequence described in d1111).

Preferably, the amino acid sequence of the heavy chain variable region of the 240B10-1 comprises:
e1111) SEQ ID NO: 60; or
e2111) an amino acid sequence derived by substituting and/or deleting and/or adding one or several amino acids of the amino acid sequence described in e1111) while having the same function as the protein of the amino acid sequence described in e1111); or
e3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in e1111) while having the same function as the protein of the amino acid sequence described in e1111); and/or
the amino acid sequence of the light chain variable region of the 240B10-1 comprises:
   f1111) SEQ ID NO: 77; or
   f2111) an amino acid sequence derived by substituting and/or deleting and/or adding one or several amino acids of the amino acid sequence described in f1111) while having the same function as the protein of the amino acid sequence described in f1111); or
   f3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in f1111) while having the same function as the protein of the amino acid sequence described in f1111).

Preferably, the amino acid sequence of the heavy chain variable region of the 98G10 comprises:
g1111) SEQ ID NO: 88; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutation compared to SEQ ID NO: 88: W34H; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutation compared to SEQ ID NO: 88: W34Y; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutation compared to SEQ ID NO: 88: Y112R; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutation compared to SEQ ID NO: 88: Y112S; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutation compared to SEQ ID NO: 88: Y112E; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutation compared to SEQ ID NO: 88: Y112Q; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34R and Y112R; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34D and Y112R; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34H and Y112L; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34T and Y112R; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34H and Y112R; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34H and Y112S; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34K and Y112S; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) an amino acid sequence having the following mutations compared to SEQ ID NO: 88: W34D and Y112S; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) SEQ ID NO: 105; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) SEQ ID NO: 88; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) an amino acid sequence having the following mutation compared to SEQ ID NO: 105: Y93R; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) SEQ ID NO: 88; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) an amino acid sequence having the following mutation compared to SEQ ID NO: 105: Y93S; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 98G10 comprises:
g1111) SEQ ID NO: 88; or
g2111) an amino acid sequence derived by substituting and/or deleting and/or adding one or several amino acids of the amino acid sequence described in g1111) while having the same function as the protein of the amino acid sequence described in g1111); or
g3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in g1111) while having the same function as the protein of the amino acid sequence described in g1111); and/or
the amino acid sequence of the light chain variable region of the 98G10 comprises:
   h1111) an amino acid sequence having the following mutation compared to SEQ ID NO: 105: Y93Q; or
   h2111) an amino acid sequence derived by substituting and/or deleting and/or adding one or several amino acids of the amino acid sequence described in h1111) while having the same function as the protein of the amino acid sequence described in h1111); or
   h3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in h1111) while having the same function as the protein of the amino acid sequence described in h1111).

Preferably, the amino acid sequence of the heavy chain variable region of the 39B4 comprises:
i1111) SEQ ID NO: 116; or
i2111) an amino acid sequence derived by substituting and/or deleting and/or adding one or several amino acids of the amino acid sequence described in i1111) while having the same function as the protein of the amino acid sequence described in i1111); or
i3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in i1111) while having the same function as the protein of the amino acid sequence described in i1111); and/or
the amino acid sequence of the light chain variable region of the 39B4 comprises:
   j1111) SEQ ID NO: 132; or
   j2111) an amino acid sequence derived by substituting and/or deleting and/or adding one or several amino acids of the amino acid sequence described in j1111) while having the same function as the protein of the amino acid sequence described in j1111); or
   j3111) an amino acid sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the amino acid sequence described in j1111) while having the same function as the protein of the amino acid sequence described in j1111).

Preferably, the antibody or antigen-binding fragment thereof comprises at least one of a full-length antibody, Fab, Fab', F(ab')2, Fv, scFv, a bispecific antibody, and a multispecific antibody.

Preferably, the antibody is a fully human antibody or a humanized antibody.

Preferably, the heavy chain variable region of the antibody or antigen-binding fragment thereof further comprises a heavy chain signal peptide; and/or
the light chain variable region of the antibody or antigen-binding fragment thereof further comprises a light chain signal peptide.

Preferably, an amino acid sequence of the heavy chain signal peptide of the 94C4 comprises:
a11111) the amino acid sequence consisting of amino acids at positions 1 to 19 in SEQ ID NO: 3; or
a21111) an amino acid sequence derived by substituting and/or deleting and/or adding one or several amino acids of the amino acid sequence described in a11111) while having the same function as the protein of the amino acid sequence described in a11111); or
a31111) an amino acid sequence having 99%, 98%, 97%, 96%, 95%, 94%, or 93% homology to the amino acid sequence described in a11111) while having the same function as the protein of the amino acid sequence described in a11111).

Preferably, an amino acid sequence of the light chain signal peptide of the 94C4 comprises:
b11111) the amino acid sequence consisting of amino acids at positions 1 to 23 in SEQ ID NO: 20; or
b21111) an amino acid sequence derived by substituting and/or deleting and/or adding one or several amino acids of the amino acid sequence described in b11111) while having the same function as the protein of the amino acid sequence described in b11111); or
b31111) an amino acid sequence having 99%, 98%, 97%, 96%, 95%, 94%, or 93% homology to the amino acid sequence described in b11111) while having the same function as the protein of the amino acid sequence described in b11111).

Preferably, an amino acid sequence of the heavy chain signal peptide of the B-8 comprises:
c11111) the amino acid sequence consisting of amino acids at positions 1 to 19 in SEQ ID NO: 31; or
c21111) an amino acid sequence derived by substituting and/or deleting and/or adding one or several amino acids of the amino acid sequence described in c11111) while having the same function as the protein of the amino acid sequence described in c11111); or
c31111) an amino acid sequence having 99%, 98%, 97%, 96%, 95%, 94%, or 93% homology to the amino acid sequence described in c11111) while having the same function as the protein of the amino acid sequence described in c11111).

Preferably, an amino acid sequence of the light chain signal peptide of the B-8 comprises:
d11111) the amino acid sequence consisting of amino acids at positions 1 to 22 in SEQ ID NO: 48; or
d21111) an amino acid sequence derived by substituting and/or deleting and/or adding one or several amino acids of the amino acid sequence described in d11111) while having the same function as the protein of the amino acid sequence described in d11111); or
d31111) an amino acid sequence having 99%, 98%, 97%, 96%, 95%, 94%, or 93% homology to the amino acid sequence described in d11111) while having the same function as the protein of the amino acid sequence described in d11111).

Preferably, an amino acid sequence of the heavy chain signal peptide of the 240B 10-1 comprises:
e11111) the amino acid sequence consisting of amino acids at positions 1 to 19 in SEQ ID NO: 59; or
e21111) an amino acid sequence derived by substituting and/or deleting and/or adding one or several amino acids of the amino acid sequence described in e11111) while having the same function as the protein of the amino acid sequence described in e11111); or
e31111) an amino acid sequence having 99%, 98%, 97%, 96%, 95%, 94%, or 93% homology to the amino acid sequence described in e11111) while having the same function as the protein of the amino acid sequence described in e11111).

Preferably, an amino acid sequence of the light chain signal peptide of the 240B10-1 comprises:
f11111) the amino acid sequence consisting of amino acids at positions 1 to 23 in SEQ ID NO: 76; or
f21111) an amino acid sequence derived by substituting and/or deleting and/or adding one or several amino acids of the amino acid sequence described in f11111) while having the same function as the protein of the amino acid sequence described in f11111); or
f31111) an amino acid sequence having 99%, 98%, 97%, 96%, 95%, 94%, or 93% homology to the amino acid sequence described in f11111) while having the same function as the protein of the amino acid sequence described in f11111).

Preferably, an amino acid sequence of the heavy chain signal peptide of the 98G10 comprises:
g11111) the amino acid sequence consisting of amino acids at positions 1 to 19 in SEQ ID NO: 87; or
g21111) an amino acid sequence derived by substituting and/or deleting and/or adding one or several amino acids of the amino acid sequence described in g11111) while having the same function as the protein of the amino acid sequence described in g11111); or
g31111) an amino acid sequence having 99%, 98%, 97%, 96%, 95%, 94%, or 93% homology to the amino acid sequence described in g11111) while having the same function as the protein of the amino acid sequence described in g11111).

Preferably, an amino acid sequence of the light chain signal peptide of the 98G10 comprises:
h11111) the amino acid sequence consisting of amino acids at positions 1 to 22 in SEQ ID NO: 104; or
h21111) an amino acid sequence derived by substituting and/or deleting and/or adding one or several amino acids of the amino acid sequence described in h11111) while having the same function as the protein of the amino acid sequence described in h11111); or
h31111) an amino acid sequence having 99%, 98%, 97%, 96%, 95%, 94%, or 93% homology to the amino acid sequence described in h11111) while having the same function as the protein of the amino acid sequence described in h11111).

Preferably, an amino acid sequence of the heavy chain signal peptide of the 39B4 comprises:
i11111) the amino acid sequence consisting of amino acids at positions 1 to 19 in SEQ ID NO: 115; or
i21111) an amino acid sequence derived by substituting and/or deleting and/or adding one or several amino acids of the amino acid sequence described in i11111) while having the same function as the protein of the amino acid sequence described in i11111); or
i31111) an amino acid sequence having 99%, 98%, 97%, 96%, 95%, 94%, or 93% homology to the amino acid sequence described in i11111) while having the same function as the protein of the amino acid sequence described in i11111).

Preferably, an amino acid sequence of the light chain signal peptide of the 39B4 comprises:
j11111) the amino acid sequence consisting of amino acids at positions 1 to 22 in SEQ ID NO: 131; or
j21111) an amino acid sequence derived by substituting and/or deleting and/or adding one or several amino acids of the amino acid sequence described in j11111) while having the same function as the protein of the amino acid sequence described in j11111); or
j31111) an amino acid sequence having 99%, 98%, 97%, 96%, 95%, 94%, or 93% homology to the amino acid sequence described in j11111) while having the same function as the protein of the amino acid sequence described in j11111).

Preferably, the amino acid sequence of the NMI comprises:
k1) the amino acid sequence with the accession number of Gene ID: 9111; or
k2) an amino acid sequence derived by substituting and/or deleting and/or adding one or several amino acids of the amino acid sequence described in k1) while having the same function as the protein of the amino acid sequence described in k1); or
k3) an amino acid sequence having 99%, 98%, 97%, 96%, 95%, 94%, or 93% homology to the amino acid sequence described in k1) while having the same function as the protein of the amino acid sequence described in k1).

The second aspect of the present invention provides a recombinant protein, comprising: the antibody or antigen-binding fragment thereof of the first aspect of the present invention; and
optionally, a tag sequence for facilitating expression and/or purification.

Preferably, the tag sequence is selected from at least one of the following groups: a His tag, a GGGS sequence, and a FLAG tag.

The third aspect of the present invention provides a biological material related to the antibody or antigen-binding fragment thereof of the first aspect of the present invention, or the recombinant protein of the second aspect of the present invention, the biological material comprises at least one of l1) to l16):
11) a nucleic acid molecule encoding the antibody or antigen-binding fragment thereof of the first aspect of the present invention, or the recombinant protein of the second aspect of the present invention;
l2) an expression cassette comprising the nucleic acid molecule of 11);
l3) a vector comprising the nucleic acid molecule of l1);
l4) a vector comprising the expression cassette of l2);
l5) a transgenic cell line comprising the nucleic acid molecule of 11);
l6) a transgenic cell line comprising the expression cassette of l2);
l7) a transgenic cell line comprising the vector of l3);
l8) a transgenic cell line comprising the vector of l4);
l9) a microorganism comprising the nucleic acid molecule of 11);
l10) a microorganism comprising the expression cassette of l2);
l11) a microorganism comprising the vector of l3);
l12) a microorganism comprising the vector of l4);
l13) a virus comprising the nucleic acid molecule of l1);
l14) a virus comprising the expression cassette of l2);
l15) a virus comprising the vector of l3);
l16) a virus comprising the vector of l4).

Preferably, the transgenic cell line does not contain a propagation material.

Preferably, the nucleic acid molecule encoding the antibody or antigen-binding fragment thereof of the first aspect of the present invention comprises a nucleic acid molecule encoding the heavy chain variable region of the antibody or antigen-binding fragment thereof of the first aspect of the present invention, and a nucleic acid molecule encoding the light chain variable region of the antibody or antigen-binding fragment thereof of the first aspect of the present invention.

Preferably, a nucleotide sequence of the nucleic acid molecule encoding the heavy chain variable region of the antibody or antigen binding fragment thereof of 94C4 of the first aspect of the present invention comprises:
m1) the nucleotide sequence as shown in SEQ ID NO: 1 or 2; or
m2) a nucleotide sequence derived by substituting and/or deleting and/or adding one or several nucleotides of the nucleotide sequence described in m1) while having the same function as the nucleic acid molecule of the nucleotide sequence described in m1); or
m3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in m1) while having the same function as the nucleic acid molecule of the nucleotide sequence described in m1); and/or
a nucleotide sequence of the nucleic acid molecule encoding the light chain variable region of the antibody or antigen binding fragment thereof of 94C4 of the first aspect of the present invention comprises:
   n1) the nucleotide sequence as shown in SEQ ID NO: 18 or 19; or
   n2) a nucleotide sequence derived by substituting and/or deleting and/or adding one or several nucleotides of the nucleotide sequence described in n1) while having the same function as the nucleic acid molecule of the nucleotide sequence described in n1); or
   n3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in n1) while having the same function as the nucleic acid molecule of the nucleotide sequence described in n1).

Preferably, a nucleotide sequence of the nucleic acid molecule encoding the heavy chain variable region of the antibody or antigen binding fragment thereof of B-8 of the first aspect of the present invention comprises:
o1) the nucleotide sequence as shown in SEQ ID NO: 29 or 30; or
o2) a nucleotide sequence derived by substituting and/or deleting and/or adding one or several nucleotides of the nucleotide sequence described in o1) while having the same function as the nucleic acid molecule of the nucleotide sequence described in o1); or
o3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in o1) while having the same function as the nucleic acid molecule of the nucleotide sequence described in o1); and/or
a nucleotide sequence of the nucleic acid molecule encoding the light chain variable region of the antibody or antigen binding fragment thereof of B-8 of the first aspect of the present invention comprises:
   p1) the nucleotide sequence as shown in SEQ ID NO: 46 or 47; or
   p2) a nucleotide sequence derived by substituting and/or deleting and/or adding one or several nucleotides of the nucleotide sequence described in p1) while having the same function as the nucleic acid molecule of the nucleotide sequence described in p1); or
   p3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in p1) while having the same function as the nucleic acid molecule of the nucleotide sequence described in p1).

Preferably, a nucleotide sequence of the nucleic acid molecule encoding the heavy chain variable region of the antibody or antigen binding fragment thereof of 240B10-1 of the first aspect of the present invention comprises:
q1) the nucleotide sequence as shown in SEQ ID NO: 57 or 58; or
q2) a nucleotide sequence derived by substituting and/or deleting and/or adding one or several nucleotides of the nucleotide sequence described in q1) while having the same function as the nucleic acid molecule of the nucleotide sequence described in q1); or
q3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in q1) while having the same function as the nucleic acid molecule of the nucleotide sequence described in q1); and/or
a nucleotide sequence of the nucleic acid molecule encoding the light chain variable region of the antibody or antigen binding fragment thereof of 240B10-1 of the first aspect of the present invention comprises:
   r1) the nucleotide sequence as shown in SEQ ID NO: 74 or 75; or
   r2) a nucleotide sequence derived by substituting and/or deleting and/or adding one or several nucleotides of the nucleotide sequence described in r1) while having the same function as the nucleic acid molecule of the nucleotide sequence described in r1); or
   r3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in r1) while having the same function as the nucleic acid molecule of the nucleotide sequence described in r1).

Preferably, a nucleotide sequence of the nucleic acid molecule encoding the heavy chain variable region of the antibody or antigen binding fragment thereof of 98G10 of the first aspect of the present invention comprises:
s1) the nucleotide sequence as shown in SEQ ID NO: 85 or 86; or
s2) a nucleotide sequence derived by substituting and/or deleting and/or adding one or several nucleotides of the nucleotide sequence described in s1) while having the same function as the nucleic acid molecule of the nucleotide sequence described in s1); or
s3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in s1) while having the same function as the nucleic acid molecule of the nucleotide sequence described in s1); and/or
a nucleotide sequence of the nucleic acid molecule encoding the light chain variable region of the antibody or antigen binding fragment thereof of 98G10 of the first aspect of the present invention comprises:
   t1) the nucleotide sequence as shown in SEQ ID NO: 102 or 103; or
   t2) a nucleotide sequence derived by substituting and/or deleting and/or adding one or several nucleotides of the nucleotide sequence described in t1) while having the same function as the nucleic acid molecule of the nucleotide sequence described in t1); or
   t3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in t1) while having the same function as the nucleic acid molecule of the nucleotide sequence described in t1).

Preferably, a nucleotide sequence of the nucleic acid molecule encoding the heavy chain variable region of the antibody or antigen binding fragment thereof of 39B4 of the first aspect of the present invention comprises:
u1) the nucleotide sequence as shown in SEQ ID NO: 113 or 114; or
u2) a nucleotide sequence derived by substituting and/or deleting and/or adding one or several nucleotides of the nucleotide sequence described in u1) while having the same function as the nucleic acid molecule of the nucleotide sequence described in u1); or
u3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in u1) while having the same function as the nucleic acid molecule of the nucleotide sequence described in u1); and/or
a nucleotide sequence of the nucleic acid molecule encoding the light chain variable region of the antibody or antigen binding fragment thereof of 39B4 of the first aspect of the present invention comprises:
   v1) the nucleotide sequence as shown in SEQ ID NO: 129 or 130; or
   v2) a nucleotide sequence derived by substituting and/or deleting and/or adding one or several nucleotides of the nucleotide sequence described in v1) while having the same function as the nucleic acid molecule of the nucleotide sequence described in v1); or
   v3) a nucleotide sequence having 99%, 98%, 97%, 96%, 95%, 94%, 93%, 92%, 91%, 90%, 89%, 88%, 87%, 86%, 85%, 84%, 83%, 82%, 81%, 80%, 70%, 60%, 50%, 40%, 35%, or at least 30% homology to the nucleotide sequence described in v1) while having the same function as the nucleic acid molecule of the nucleotide sequence described in v1).

The fourth aspect of the present invention provides a conjugate comprising: at least one of the antibody or antigen-binding fragment thereof of the first aspect of the present invention and the recombinant protein of the second aspect of the present invention;
and a conjugated moiety, the conjugated moiety comprises at least one of a detectable marker, a drug, a toxin, and a cytokine.

Preferably, the conjugated moiety comprises a detectable marker.

Preferably, the conjugated moiety comprises at least one of a drug, a toxin, and a cytokine.

Preferably, the detectable marker is selected from the group consisting of microparticles (e.g., colloidal gold and colored latex), fluorescent labels (e.g., fluorescein (e.g., 5-fluorescein, 6-carboxyfluorescein, 3'6-carboxyfluorescein, 5(6)-carboxyfluorescein, 6-hexachloro-fluorescein, 6-tetrachloro-fluorescein, fluorescein isothiocyanate, etc.)), rhodamine, phycoerythrin, phycocyanin, allophycocyanin, o-phthalaldehyde, and fluorescamin), redox molecular labels (e.g., ferrocene, ruthenium complexes, viologen, quinone, It ion, Cs ion, diimide, 1,4-benzoquinone, and hydroquinone), chemiluminescent labels (e.g., acridinium ester, luminol, isoluminol, phenanthridinium ester, etc.), radioactive labels (e.g., 3H, 14C, 32P, 33P, 35S, 90Y, 99Tc, 111In, 125I, 131I, 177Lu, 166Ho, and 153Sm), enzyme labels (e.g., β-glucuronidase, β-glucosidase, urease, peroxidase (horseradish peroxidase) or alkaline phosphatase, acetylcholinesterase, glucose oxidase, hexokinase and GDPase, RNase, glucose oxidase and luciferase, fructose phosphokinase, phosphoenolpyruvate carboxylase, aspartate aminotransferase, phosphoenolpyruvate decarboxylase, and β-lactamase), ligand labels (e.g., biotin and derivatives thereof), or any combination thereof.

The fifth aspect of the present invention provides use of the antibody or antigen-binding fragment thereof of the first aspect, the recombinant protein of the second aspect, the biological material of the third aspect, and/or the conjugate of the fourth aspect of the present invention in the preparation of a product;
the product comprises at least one of a drug, a reagent, a test strip, a test plate, a kit, a test chip, an adsorbent, and a medical device.

Preferably, the drug may prevent and treat a disease or condition related to abnormal high level and/or activity of NMI.

Preferably, a subject to which the drug is administered is a mammal.

Preferably, the reagent, test strip, test plate, test chip or kit has at least one of the functions w1) to w3):
w1) detecting the presence or level of NMI protein in a sample;
w2) diagnosing, auxiliary diagnosing, or prognostic evaluating a diseases or condition related to abnormal high level and/or activity of NMI;
w3) drug screening, the drug is used for preventing and treating a disease or condition related to abnormal high level and/or activity of NMI.

Preferably, the reagent, test strip, test plate, test chip or kit is used for one or more detection methods selected from the following groups: radio-labeled immunoassay (RIA), enzyme immunoassay (EIA or ELISA), fluorescence immunoassay (FIA), luminescence immunoassay, Western Blot method, physicochemical method, and biochip method.

Preferably, a test sample of the reagent, test strip, test plate, test chip or kit is selected from at least one of body fluids, tissues, cells, and excreta of a subject to be tested.

Preferably, the subject to be tested is a mammal.

Preferably, the adsorbent or the medical device is used for removing NMI protein from body fluids and reducing occurrence of abnormal immune responses caused by NMI protein.

Preferably, the body fluids comprise serum, plasma, whole blood, urine, sputum, pleural fluid, cerebrospinal fluid, joint fluid, ascites, saliva, tear fluid, lymph fluid, body cavity effusions, and the like.

Preferably, the body fluids are from a mammal.

Preferably, the disease or condition related to abnormal high level and/or activity of NMI comprises at least one of inflammation, infection, septicemia, organ damage and autoimmune disease; further comprising at least one of septicemia and autoimmune disease.

Preferably, the autoimmune disease comprises at least one of arthritis, systemic lupus erythematosus, ankylosing spondylitis, psoriasis, neurological disorders, vitiligo, asthma, inflammatory bowel disease, peritonitis, lung injury, pneumonia, nephritis, neurological inflammation, and hepatitis.

Preferably, the neurological disorders comprise at least one of amyotrophic lateral sclerosis, Alzheimer's disease, Parkinson's disease, multiple sclerosis, encephalitis, brain tumor, traumatic brain injury, and immune-mediated encephalopathy.

Preferably, the encephalitis comprises at least one of fungal encephalitis, bacterial encephalitis, viral encephalitis, and anti-N-methyl-D-aspartate receptor encephalitis.

Preferably, the mammal is human.

Preferably, the antibody or antigen-binding fragment thereof of the first aspect of the present invention may be one, two, three, four or five of 94C4, B-8, 240B10-1, 98G10, and 39B4.

The sixth aspect of the present invention provides a product, the product comprises at least one of x1) to x3):
x1) the antibody or antigen-binding fragment thereof of the first aspect of the present invention;
x2) the recombinant protein of the second aspect of the present invention;
x3) the conjugate of the fourth aspect of the present invention;
the product comprises at least one of a reagent, a test strip, a test plate, a kit, a test chip, an adsorbent, and a medical device.

Preferably, the reagent, test strip, test plate, test chip or kit has at least one of the functions w1) to w3):
w1) detecting the presence or level of NMI protein in a sample;
w2) diagnosing, auxiliary diagnosing, or prognostic evaluating a diseases or condition related to abnormal high level and/or activity of NMI;
w3) drug screening, the drug is used for preventing and treating a disease or condition related to abnormal high level and/or activity of NMI.

Preferably, the reagent, test strip, test plate, test chip or kit is used for one or more detection methods selected from the following groups: radio-labeled immunoassay (RIA), enzyme immunoassay (EIA or ELISA), fluorescence immunoassay (FIA), luminescence immunoassay, Western Blot method, physicochemical method, and biochip method.

Preferably, a test sample of the reagent, test strip, test plate, test chip or kit is selected from at least one of body fluids, tissues, cells, and excreta of a subject to be tested.

Preferably, the subject to be tested is a mammal.

Preferably, the adsorbent or the medical device is used for removing NMI protein from body fluids and reducing occurrence of abnormal immune responses caused by NMI protein.

Preferably, the body fluids comprise serum, plasma, whole blood, urine, sputum, pleural fluid, cerebrospinal fluid, joint fluid, ascites, saliva, tear fluid, lymph fluid, body cavity effusions, and the like.

Preferably, the body fluids are from a mammal.

Preferably, the disease or condition related to abnormal high level and/or activity of NMI comprises at least one of inflammation, infection, septicemia, organ damage and autoimmune disease; further comprising at least one of septicemia and autoimmune disease.

Preferably, the autoimmune disease comprises at least one of arthritis, systemic lupus erythematosus, ankylosing spondylitis, psoriasis, neurological disorders, vitiligo, asthma, inflammatory bowel disease, peritonitis, lung injury, pneumonia, nephritis, neurological inflammation, and hepatitis.

Preferably, the neurological disorders comprise at least one of amyotrophic lateral sclerosis, Alzheimer's disease, Parkinson's disease, multiple sclerosis, encephalitis, brain tumor, traumatic brain injury, and immune-mediated encephalopathy.

Preferably, the encephalitis comprises at least one of fungal encephalitis, bacterial encephalitis, viral encephalitis, and anti-N-methyl-D-aspartate receptor encephalitis.

Preferably, the mammal is human.

Preferably, the antibody or antigen-binding fragment thereof of the first aspect of the present invention may be one, two, three, four or five of 94C4, B-8, 240B10-1, 98G10, and 39B4.

Preferably, a double-antibody sandwich ELISA kit, comprising: a coating antibody and a detection antibody, at least one of the coating antibody and the detection antibody comprises the antibody or antigen-binding fragment thereof of the first aspect of the present invention.

Preferably, the coating antibody and the detection antibody are directed against different antigenic epitopes of NMI, respectively.

Preferably, the coating antibody is bound to a solid phase carrier.

Preferably, the solid phase carrier comprises at least one of magnetic particles, latex particles and a microplate; preferably, the solid phase carrier is a microplate; further preferably a microtiter plate; yet further preferably a high-binding microtiter plate made of polystyrene.

Preferably, the detection antibody is labeled with a detectable marker.

Preferably, the detectable marker is selected from at least one of enzyme labels (e.g., β-glucuronidase, β-glucosidase, urease, peroxidase (horseradish peroxidase) or alkaline phosphatase, acetylcholinesterase, glucose oxidase, hexokinase and GDPase, RNase, glucose oxidase and luciferase, fructose phosphokinase, phosphoenolpyruvate carboxylase, aspartate aminotransferase, phosphoenolpyruvate decarboxylase, and β-lactamase), ligand labels (e.g., biotin and derivatives thereof); further preferably, the detectable marker is a ligand label; yet further preferably biotin.

Preferably, when the detectable marker is selected from ligand (e.g. biotin) labels, the double-antibody sandwich ELISA kit further comprises an enzyme-labeled receptor (e.g. horseradish peroxidase-labeled streptavidin).

Preferably, the coating antibody and the detection antibody are selected from any two of the antibodies or antigen-binding fragments thereof of the first aspect of the present invention; further preferably, the coating antibody comprises B-8 and the detection antibody comprises 94C4; or the coating antibody comprises 94C4 and the detection antibody comprises B-8.

Preferably, the double-antibody sandwich ELISA kit further comprises at least one of a sample diluent, a washing solution, a blocking solution, a chromogenic solution, a stop solution, and a calibrator.

In the present application, the sample buffer, washing solution, blocking solution, chromogenic solution and stop solution are commonly used reagents in the art, and those skilled in the art can routinely select and use them according to the needs.

Preferably, the sample dilution is PBS comprising BSA and Tween-20.

Preferably, the washing solution is PBS comprising Tween-20.

Preferably, the blocking solution is PBS comprising BSA.

Preferably, the chromogenic solution is a TMB chromogenic solution.

Preferably, the stop solution is sulfuric acid.

Preferably, the coating antibody bound to the solid phase carrier has a coating concentration of 0.25 µg/mL to 5 µg/mL; preferably 0.25 µg/mL to 1 µg/mL.

Preferably, the detection antibody is used at a concentration of 0.25 µg/mL to 1 µg/mL.

Preferably, the enzyme-labeled receptor is used at a concentration of 50 ng/mL to 200 ng/mL.

Preferably, when using the kit, an incubation time for the sample with the coating antibody is 45min to 1.5h.

A chemiluminescent assay kit, comprising a coating antibody and a detection antibody, at least one of the coating antibody and the detection antibody comprises the antibody or antigen-binding fragment thereof of the first aspect of the present invention.

Preferably, the coating antibody and the detection antibody are directed against different antigenic epitopes of NMI, respectively.

Preferably, the coating antibody is bound to a solid phase carrier.

Preferably, the solid phase carrier comprises at least one of magnetic particles, latex particles and a microplate; further preferably, the solid phase carrier is magnetic particles.

Preferably, the detection antibody is labeled with a detectable marker.

Preferably, the detectable marker is selected from chemiluminescent labels (e.g., acridinium ester, luminol, isoluminol, phenanthridinium ester, etc.); further preferably selected from acridinium ester.

Preferably, the coating antibody and the detection antibody are selected from any two of the antibodies or antigen-binding fragments thereof of the first aspect of the present invention; further preferably, the coating antibody comprises B-8 and the detection antibody comprises 98G10; or the coating antibody comprises 98G10 and the detection antibody comprises B-8.

Preferably, the chemiluminescent assay kit further comprises at least one of a reaction buffer, an NMI calibrator and a luminescent substrate; further comprising a reaction buffer, an NMI calibrator and a luminescent substrate.

Preferably, the luminescent substrate comprises a pre-excitation solution and an excitation solution.

An immunochromatographic test strip, comprising a base plate; and a sample pad, a marker pad, a chromatographic membrane and an absorbent pad that are partially overlapping and adhered to the base plate; wherein the marker pad is coated with a marker-labeled anti-NMI antibody I, the chromatographic membrane is provided with a test line, the test line is coated with an anti-NMI antibody II, at least one of the anti-NMI antibody I and the anti-NMI antibody II comprises the antibody or antigen-binding fragment thereof of the first aspect of the present invention.

Preferably, the anti-NMI antibody I and the anti-NMI antibody II are directed against different antigenic epitopes of NMI, respectively.

Preferably, the anti-NMI antibody I and the anti-NMI antibody II are selected from any two of the antibodies or antigen-binding fragments thereof of the first aspect of the present invention; further preferably, the anti-NMI antibody I comprises B-8 and the anti-NMI antibody II comprises 94C4; or the anti-NMI antibody I comprises 94C4 and the anti-NMI antibody II comprises B-8.

Preferably, the chromatographic membrane may be a conventional NC membrane (nitrocellulose membrane).

Preferably, the marker is colloidal gold.

Preferably, the chromatographic membrane is further provided with a quality control line.

Preferably, the quality control line is coated with an anti-NMI antibody I; further preferably coated with a goat anti-rabbit IgG antibody.

Preferably, an adsorbent, comprising a carrier matrix and the antibody or antigen-binding fragment thereof of the first aspect of the present invention.

Preferably, the antibody or antigen-binding fragment thereof is attached to the carrier matrix.

Preferably, the carrier matrix is selected from at least one of agarose gel particles, cellulose gel particles, dextran gel particles, magnetic particles, silica gel particles, activated carbon, resin particles, Protein A agarose particles, and Protein G agarose particles.

Preferably, a medical device, comprising at least one of the antibody or antigen-binding fragment thereof of the first aspect of the present invention and the above-mentioned adsorbent.

The seventh aspect of the present invention provides a drug, the drug comprises at least one of y1) to y4):
y1) the antibody or antigen-binding fragment thereof of the first aspect of the present invention;
y2) the recombinant protein of the second aspect of the present invention;
y3) the biological material of the third aspect of the present invention;
y4) the conjugate of the fourth aspect of the present invention.

Preferably, the drug further comprises a pharmaceutically acceptable carrier.

Preferably, the drug may prevent and treat a disease or condition related to abnormal high level and/or activity of NMI.

Preferably, the disease or condition related to abnormal high level and/or activity of NMI comprises at least one of inflammation, infection, septicemia, organ damage, and autoimmune disease; further comprising at least one of septicemia and autoimmune disease.

Preferably, the autoimmune disease comprises at least one of arthritis, systemic lupus erythematosus, ankylosing spondylitis, psoriasis, neurological disorders, vitiligo, asthma, inflammatory bowel disease, peritonitis, lung injury, pneumonia, nephritis, neurological inflammation, and hepatitis.

Preferably, the neurological disorders comprise at least one of amyotrophic lateral sclerosis, Alzheimer's disease, Parkinson's disease, multiple sclerosis, encephalitis, brain tumor, traumatic brain injury, and immune-mediated encephalopathy.

Preferably, the encephalitis comprises at least one of fungal encephalitis, bacterial encephalitis, viral encephalitis, and anti-N-methyl-D-aspartate receptor encephalitis.

Preferably, a subject to which the drug is administered is a mammal.

Preferably, the mammal is human.

Preferably, the antibody or antigen-binding fragment thereof of the first aspect of the present invention may be one, two, three, four or five of 94C4, B-8, 240B10-1, 98G10, and 39B4.

The eighth aspect of the present invention provides a method for preparing the antibody or antigen-binding fragment thereof of the first aspect, or the recombinant protein of the second aspect of the present invention, which are obtained by culturing a transgenic cell line, a microorganism or a virus of the third aspect of the present invention.

The ninth aspect of the present invention provides a method for detecting the presence or level of NMI, comprising the step of using the reagent, test strip, test plate, kit or test chip of the sixth aspect of the present invention.

The tenth aspect of the present invention provides a method for diagnosing, auxiliary diagnosing, or prognostic evaluating a diseases or condition related to abnormal high level and/or activity of NMI, comprising the step of using the reagent, test strip, test plate, kit or test chip of the sixth aspect of the present invention.

Preferably, the method comprises the following step of: detecting the presence or level of NMI in a test sample using the reagent, test strip, test plate, kit or test chip of the sixth aspect of the present invention.

Preferably, the method further comprises the following step of: performing diagnosis, auxiliary diagnosis or prognostic evaluation of the disease or condition related to abnormal high level and/or activity of NMI based on the presence or level of NMI in the test sample.

Preferably, the test sample is selected from at least one of body fluids, tissues, cells, and excreta of a subject to be tested.

Preferably, the subject to be tested is a mammal.

The eleventh aspect of the present invention provides a method for removing NMI protein from body fluids, comprising the step of using the adsorbent or medical device of the sixth aspect of the present invention.

Preferably, the method comprises the following step of: treating the body fluids with the adsorbent or medical device of the sixth aspect of the present invention, thereby removing the NMI protein from the body fluids.

The beneficial effects of the present invention are as follows.

The present invention provides an anti-NMI antibody or antigen-binding fragment thereof, the antibody or antigen-binding fragment thereof demonstrates good binding activity to NMI protein and high affinity for NMI protein, which can be used for detecting the presence or level of NMI protein in a sample, diagnosing, auxiliary diagnosing, or prognostic evaluating a disease or condition related to abnormal high level and/or activity of NMI, for removing the NMI protein from the body fluids to prevent and treat the disease or condition related to abnormal high level and/or activity of NMI.

### BRIEF DESCRIPTION OF DRAWINGS

FIG. 1 is a graph of EC₅₀ results for the 23 positive monoclonal antibodies in Example 1.
FIG. 2 is a graph of EC₅₀ results for the 8 positive monoclonal antibodies in Example 1.
FIG. 3 is a graph of SDS-PAGE results after preparation of monoclonal antibodies B8 and 94C4 in Example 2.
FIG. 4 is a graph of the results of the double-antibody sandwich ELISA assay in Example 2 for detecting the specificity of the paired antibodies.
FIG. 5 is a graph of EC₅₀ results for the monoclonal antibodies 94C4, B-8, 240B10-1, 98G10, and 39B4 in Example 2.
FIG. 6 is a graph of Western blot results of specific detection of monoclonal antibodies 94C4, B-8, 240B10-1, 98G10, and 39B4 in Example 2.
FIG. 7 is a graph of the standard curve in Example 5 obtained by using the kit of 1) in Example 3.
FIG. 8 is a schematic diagram of a colloidal gold test strip for detecting NMI in Example 9.
FIG. 9 is an interaction model and critical sites between the monoclonal antibody 39B4 and NMI as predicted by AlphaFold2 in Example 2.
FIG. 10 is an interaction model and critical sites between the monoclonal antibody 94C4 and NMI as predicted by AlphaFold2 in Example 2.
FIG. 11 is an interaction model and critical sites between the monoclonal antibody 240B10-1 and NMI as predicted by AlphaFold2 in Example 2.
FIG. 12 is an interaction model and critical sites between the monoclonal antibody B-8 and NMI as predicted by AlphaFold2 in Example 2.
FIG. 13 is an interaction model and critical sites between the monoclonal antibody 98G10 and NMI as predicted by AlphaFold2 in Example 2.

### DETAILED DESCRIPTION

The following is a further detailed description of the present invention by means of specific examples, which are given to enable a more thorough understanding of the present invention and to enable the scope of the present invention to be conveyed in full to those skilled in the art.

It should be understood that these examples are used only to illustrate the present invention and are not intended to limit the scope of the present invention.

It should be noted that certain words are used in the specification and claims to refer to specific components. It should be appreciated by those skilled in the art that the skilled person may refer to the same component by different terms. The specification and claims do not use differences in nomenclature as a way of distinguishing components, but rather differences in function of the components as a criterion for differentiation. As the term "comprises" or "includes" is open-ended throughout the specification and claims, it should be interpreted to mean "includes but is not limited to". The specification subsequently describes preferred embodiments for carrying out the present application, however, the description is intended to be a general principle of the specification and is not intended to limit the scope of the present application. The scope of protection of the present application shall be as defined in the appended claims.

Scientific and technical terms referred to in this specification have the same meanings as those commonly understood by those skilled in the art, and in the event of a conflict the definitions in this specification shall prevail.

In general, the terms used in this specification have the following meanings.

In this specification, "monoclonal antibody" denotes an antibody derived from a population of substantially homologous antibodies, i.e., the individual antibodies constituting the population are identical and/or bind to the same epitope, except for possible variant antibodies (e.g., containing naturally occurring mutations or mutations produced during the production of the monoclonal antibody product). Such variants are typically present in trace amounts. Unlike polyclonal antibody products that typically include different antibodies directed against different determinants (epitopes), monoclonal antibody products have each monoclonal antibody directed against a single determinant on an antigen. Thus, the modifier "monoclonal" indicates the characterization of the antibody as being derived from a substantially homologous population of antibodies and should not be interpreted as requiring the production of the antibody by any particular method. For example, monoclonal antibodies to be used in accordance with the present invention may be prepared by a variety of techniques, the techniques include, but are not limited to, hybridoma methods, recombinant DNA methods, phage display methods, and methods using genetically engineered animals comprising all or a portion of the human immunoglobulin locus, and such methods and other exemplary methods of preparing monoclonal antibodies are described herein.

In this specification, "affinity" refers to the total strength of non-covalent interactions between a single binding site of a molecule (e.g., an antibody) and its binding partner (e.g., an antigen). Unless otherwise indicated, "binding affinity" as used in this specification denotes an intrinsic binding affinity that reflects a 1:1 interaction between members of a binding pair (e.g., antibody and antigen). The affinity of molecule X for its partner Y can usually be expressed by the equilibrium dissociation constant (KD). Affinity can be measured by common methods known in the art.

In this specification, human NMI represents a protein of human origin.

In this specification, "anti-human NMI monoclonal antibody" denotes a monoclonal antibody that binds to human NMI protein with sufficient affinity such that the monoclonal antibody can be used to identify/detect human NMI protein.

In this specification, "enzyme-linked immunosorbent assay (EILSA)" refers to an assay that utilizes the ability of an antibody molecule to bind specifically to an antigen molecule, binding a free impurity protein to a target protein bound to a solid-phase carrier, and qualitatively or quantitatively analyzing it using a special marker. The principle lies in that: antigens or antibodies are physically adsorbed to the surface of the solid phase and maintain their immunoreactivity; antigens or antibodies can form enzyme conjugates with enzymes through covalent bonds while maintaining their respective immunologic activities or enzymatic activities; the binding of the enzyme conjugate to the corresponding antigen or antibody enables the occurrence of an immune reaction to be determined by the chromogenic reaction of the added substrate, and the intensity of the chromogenic reaction is proportional to the amount of the corresponding antigen or antibody in the specimen. Depending on the substance to be detected and the conditions available for detection, different types of assays can be designed, with the double antibody sandwich method being the most commonly used method for detecting antigens. It comprises adsorbing antisera containing a known antibody into small wells on a microtiter plate and washing for once; adding an antigen to be detected, and if the two are specific, binding occurs and then washing away the excess antibody; adding an enzyme-linked antibody that reacts specifically with the antigen to be detected to form a "sandwich"; adding a substrate of this enzyme, and if the production of a colored enzymatic product is visible, it demonstrates the presence of the corresponding antigen.

In this specification, "avidin" is a glycoprotein consisting of four subunits per molecule, which can bind closely to four biotin molecules. Streptavidin, which is extracted from streptomycin, is used more often.

In this specification, "biotin" is also referred to as vitamin H. Derivatives made chemically, biotin-hydroxy succinimide (BNHS) can form biotinylated products with many types of large and small molecules such as proteins, sugars and enzymes. The combination of avidin and biotin is not an immune reaction, but has strong specificity and high affinity, and the two are extremely stable once combined. Since 1 avidin molecule has binding sites for 4 biotin molecules, more biotinylated molecules can be attached to form a lattice-like complex. Thus, combining avidin and biotin with an ELISA can greatly improve the sensitivity of the ELISA.

The biotin-avidin system is used in ELISA in various forms, either for indirect coating or for amplification of final reaction. The enzyme-labeled antibody in a routine ELISA can also be replaced by a biotinylated antibody, which is then attached to an avidin-enzyme conjugate to amplify the reaction signal.

Experimental methods for which specific conditions are not indicated in the following examples are generally in accordance with conventional conditions, or in accordance with conditions recommended by the manufacturer. The materials, reagents, and the like used in the examples, if not otherwise specified, are reagents and materials obtained commercially.

The biotin-labeled monoclonal antibody in the example was prepared as follows: 2.0 mg of an antibody was taken, 27 µL of 10 mM biotin solution was added, mixed, and rotated and mixed by protecting from light for 18±2 hours in a freezer at 2~8°C; the coupling solution was transferred to a 50 kD ultrafiltration centrifuge tube and centrifuged at 8000 rpm for 10 min, filtrate was discarded; 200 µL of PBS buffer was added, and centrifuged at 8000 rpm for 10 min, the operation was repeated for five times; the filter membrane of centrifuge tube was inverted and centrifuged at 3000 rpm for 1 min, the concentrated biotin-labeled antibody was collected, 100 µL of PBS buffer was added and left to stand for 3 min, the filter membrane of centrifuge tube was inverted and centrifuged at 3000 rpm for 1 min again; the concentration of biotin-labeled antibody was detected by Nanodrop and set to 2 mg/mL.

### Example 1. Screening and determination of anti-NMI monoclonal antibodies

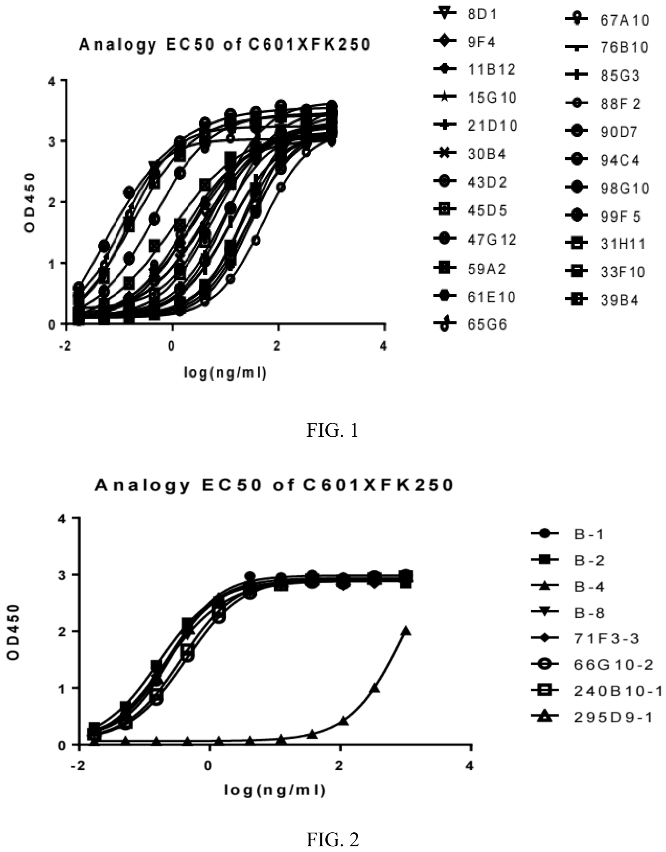

Rabbits were immunized with human NMI (Gene ID: 9111, unless otherwise specified below, its corresponding accession number is Gene ID: 9111) protein, antibodies were prepared from hybridoma cells, and rabbit monoclonal antibodies were screened by ELISA assay (for the specific method, refer to the Example section of the Patent Document CN115698058A), with the differences only in that: in the present Example, the coating protein was human NMI; the secondary antibody was horseradish peroxidase-conjugated anti-rabbit IgG Fc secondary antibody, diluted at 1:30,000), in which the indirect Elisa assay results of each positive monoclonal antibody were shown in Tables 1-4 and FIGs. 1-2, and the monoclonal antibodies with clone numbers 94C4, 39B4, 8D1, 65G6, 98G10, 295D9-1, B-8, 240B10-1, B-2, and 71F3-3 were selected for epitope analysis, the results were shown in Table 5: 39B4, 8D1, 65G6, and 295D9-1 shared the same epitope; 240B10-1 and B-2 shared the same epitope; the epitope of 71F3-3 was unknown; and the epitopes of the remaining monoclonal antibodies were all distinct from one another.

**Table 1. Results of the relative affinity of 23 positive monoclonal antibodies determined by the concentration-response relationship curve**

| Dilution (Dilution ratio) | Antibody Name | | | | | | | |
|---|---|---|---|---|---|---|---|---|
| | 8D1 | 9F4 | 11B12 | 15G10 | 21D10 | 30B4 | 43D2 | 45D5 |
| no-dilution (stock solution) | 3.343 | 3.295 | 3.149 | 3.230 | 3.124 | 3.134 | 3.137 | 3.225 |
| 1/3 | 3.288 | 3.168 | 2.968 | 3.149 | 3.070 | 3.200 | 2.995 | 3.222 |
| 1/9 | 3.171 | 3.134 | 2.630 | 3.042 | 2.877 | 2.988 | 2.749 | 2.956 |
| 1/27 | 3.208 | 2.816 | 1.807 | 2.845 | 2.683 | 2.909 | 2.111 | 2.805 |
| 1/81 | 3.199 | 2.562 | 0.961 | 2.259 | 1.910 | 2.390 | 1.238 | 2.303 |
| 1/243 | 3.084 | 1.861 | 0.468 | 1.555 | 1.132 | 1.594 | 0.594 | 1.356 |
| 1/729 | 2.867 | 1.246 | 0.279 | 1.086 | 0.604 | 1.070 | 0.360 | 0.823 |
| 1/2187 | 2.561 | 0.893 | 0.179 | 0.750 | 0.410 | 0.777 | 0.220 | 0.493 |
| 1/6561 | 1.653 | 0.434 | 0.102 | 0.329 | 0.246 | 0.407 | 0.126 | 0.232 |
| 1/19683 | 0.810 | 0.203 | 0.076 | 0.171 | 0.133 | 0.205 | 0.090 | 0.133 |
| 1/59049 | 0.373 | 0.118 | 0.067 | 0.108 | 0.093 | 0.122 | 0.073 | 0.099 |
| 0 | 0.069 | 0.072 | 0.064 | 0.073 | 0.067 | 0.071 | 0.068 | 0.068 |
| Concentration of supernatant (supernatant concentration, µg/mL) | 7.064 | 1.013 | 0.426 | 1.070 | 1.322 | 1.572 | 0.236 | 0.492 |
| EC50 (ng/mL) | 1.036 | 2.691 | 12.990 | 4.582 | 10.815 | 6.079 | 4.855 | 2.744 |
| R² | 0.9968 | 0.9979 | 0.9993 | 0.9972 | 0.9982 | 0.9954 | 0.9993 | 0.9976 |

| Dilution (Dilution ratio) | Antibody Name | | | | | | | |
|---|---|---|---|---|---|---|---|---|
| | 47G12 | 59A2 | 61E10 | 65G6 | 67A10 | 76B10 | 85G3 | 88F2 |
| no-dilution (stock solution) | 3.110 | 3.160 | 3.163 | 3.074 | 3.008 | 3.046 | 3.086 | 3.004 |
| 1/3 | 2.979 | 3.063 | 3.054 | 2.980 | 2.949 | 3.055 | 2.966 | 2.817 |
| 1/9 | 2.553 | 3.010 | 2.978 | 3.007 | 2.932 | 2.798 | 2.717 | 2.240 |
| 1/27 | 1.834 | 2.882 | 2.845 | 3.008 | 2.790 | 2.300 | 2.374 | 1.373 |
| 1/81 | 1.208 | 2.722 | 2.558 | 3.096 | 2.651 | 1.661 | 1.689 | 0.735 |
| 1/243 | 0.509 | 2.277 | 1.957 | 2.928 | 1.970 | 0.901 | 0.885 | 0.369 |
| 1/729 | 0.346 | 1.621 | 1.331 | 2.846 | 1.413 | 0.464 | 0.485 | 0.211 |
| 1/2187 | 0.234 | 1.282 | 0.771 | 2.552 | 0.924 | 0.335 | 0.344 | 0.173 |
| 1/6561 | 0.138 | 0.666 | 0.342 | 1.848 | 0.384 | 0.179 | 0.169 | 0.101 |
| 1/19683 | 0.104 | 0.314 | 0.180 | 1.024 | 0.184 | 0.109 | 0.098 | 0.078 |
| 1/59049 | 0.095 | 0.167 | 0.109 | 0.498 | 0.114 | 0.092 | 0.075 | 0.070 |
| 0 | 0.081 | 0.076 | 0.069 | 0.074 | 0.068 | 0.074 | 0.064 | 0.066 |

| Concentration of supernatant (supernatant concentration, µg/mL) | 0.141 | 2.120 | 1.075 | 8.880 | 1.560 | 0.536 | 0.167 | N/A |
|---|---|---|---|---|---|---|---|---|
| EC50 (ng/mL) | 3.938 | 2.013 | 2.253 | 0.981 | 2.474 | 6.620 | 1.921 | N/A |
| R² | 0.9986 | 0.9973 | 0.9995 | 0.9982 | 0.9975 | 0.9990 | 0.9987 | 0.9993 |

| Dilution (Dilution ratio) | Antibody Name | | | | | | | |
|---|---|---|---|---|---|---|---|---|
| | 90D7 | 94C4 | 98G10 | 99F5 | 31H11 | 33F10 | 39B4 | - |
| no-dilution (stock solution) | 3.570 | 3.462 | 3.449 | 3.482 | 3.404 | 3.492 | 3.404 | - |
| 1/3 | 3.490 | 3.505 | 3.448 | 3.371 | 3.180 | 3.227 | 3.434 | - |
| 1/9 | 3.460 | 3.576 | 3.390 | 3.148 | 2.917 | 2.768 | 3.412 | - |
| 1/27 | 3.227 | 3.468 | 3.316 | 2.766 | 2.214 | 1.939 | 3.347 | - |
| 1/81 | 2.347 | 3.433 | 3.295 | 1.900 | 1.276 | 1.069 | 3.284 | - |
| 1/243 | 1.486 | 3.286 | 2.970 | 1.036 | 0.626 | 0.501 | 3.095 | - |
| 1/729 | 0.896 | 2.977 | 2.475 | 0.562 | 0.338 | 0.277 | 2.764 | - |
| 1/2187 | 0.714 | 2.476 | 1.720 | 0.288 | 0.245 | 0.166 | 2.322 | - |
| 1/6561 | 0.405 | 2.154 | 1.075 | 0.215 | 0.225 | 0.116 | 1.610 | - |
| 1/19683 | 0.200 | 1.279 | 0.528 | 0.146 | 0.125 | 0.089 | 0.758 | - |
| 1/59049 | 0.153 | 0.585 | 0.237 | 0.094 | 0.083 | 0.175 | 0.415 | - |
| 0 | 0.092 | 0.084 | 0.103 | 0.100 | 0.134 | 0.151 | 0.091 | - |
| Concentration of supernatant (supernatant concentration, µg/mL) | 0.845 | 4.446 | 2.779 | 0.474 | 0.162 | 0.137 | 3.370 | - |
| EC50 (ng/mL) | 5.608 | 0.189 | 1.225 | 5.204 | 3.674 | 4.634 | 0.567 | - |

**Table 2. EC₅₀ of the 23 positive monoclonal antibodies**

| Affinity Ranking | Clone Name (monoclonal antibody name) | EC50 (ng/mL) |
|---|---|---|
| 1 | 94C4 | 0.189 |
| 2 | 39B4 | 0.567 |
| 3 | 65G6 | 0.981 |
| 4 | 8D1 | 1.036 |
| 5 | 98G10 | 1.225 |
| 6 | 85G3 | 1.921 |
| 7 | 59A2 | 2.013 |
| 8 | 61E10 | 2.253 |
| 9 | 67A10 | 2.474 |
| 10 | 9F4 | 2.691 |
| 11 | 45D5 | 2.744 |
| 12 | 31H11 | 3.674 |
| 13 | 47G12 | 3.938 |
| 14 | 15G10 | 4.582 |
| 15 | 33F10 | 4.634 |
| 16 | 43D2 | 4.855 |
| 17 | 99F5 | 5.204 |
| 18 | 90D7 | 5.608 |
| 19 | 30B4 | 6.079 |
| 20 | 76B10 | 6.620 |
| 21 | 21D10 | 10.815 |
| 22 | 11B12 | 12.990 |
| 23 | 88F2 | N/A |

**Table 3. Results of the relative affinity of 8 positive monoclonal antibodies determined by the concentration-response relationship curve**

| Dilution (Dilution ratio) | Antibody Name | | | | | | | |
|---|---|---|---|---|---|---|---|---|
| | B-1 | B-2 | B-4 | B-8 | 71F3-3 | 66G10-2 | 240B10-1 | 295D9-1 |
| no-dilution (stock solution) | 2.941 | 2.843 | 2.019 | 2.962 | 2.958 | 2.991 | 2.967 | 2.978 |
| 1/3 | 2.992 | 2.966 | 1.010 | 2.901 | 2.832 | 2.967 | 2.931 | 2.930 |
| 1/9 | 2.955 | 2.861 | 0.426 | 2.877 | 2.800 | 2.843 | 2.893 | 2.923 |
| 1/27 | 3.002 | 2.920 | 0.190 | 2.845 | 2.890 | 2.858 | 2.915 | 2.931 |
| 1/81 | 2.959 | 2.858 | 0.100 | 2.795 | 2.916 | 2.872 | 2.814 | 2.881 |
| 1/243 | 2.974 | 2.801 | 0.071 | 2.683 | 2.775 | 2.665 | 2.741 | 2.841 |
| 1/729 | 2.558 | 2.532 | 0.069 | 2.478 | 2.547 | 2.238 | 2.349 | 2.563 |
| 1/2187 | 2.059 | 2.159 | 0.063 | 1.931 | 2.040 | 1.558 | 1.678 | 2.055 |
| 1/6561 | 1.108 | 1.410 | 0.064 | 1.275 | 1.210 | 0.793 | 0.890 | 1.174 |
| 1/19683 | 0.501 | 0.681 | 0.072 | 0.526 | 0.552 | 0.360 | 0.390 | 0.552 |
| 1/59049 | 0.225 | 0.310 | 0.062 | 0.232 | 0.254 | 0.169 | 0.184 | 0.251 |
| 0 | 0.063 | 0.076 | 0.062 | 0.074 | 0.070 | 0.068 | 0.059 | 0.062 |
| Concentration of supernatant (supernatant concentration, µg/mL) | 12.256 | 16.397 | 0.397 | 12.838 | 13.005 | 5.754 | 7.209 | 10.197 |
| EC50 (ng/mL) | 3.135 | 2.715 | 397.380 | 2.506 | 2.860 | 2.471 | 2.601 | 2.376 |
| R² | 0.9983 | 0.9984 | 1.0000 | 0.9985 | 0.9981 | 0.9988 | 0.9996 | 0.9996 |

**Table 4. EC₅₀ of the 8 positive monoclonal antibodies**

| Affinity Ranking | Clone Name (monoclonal antibody name) | EC₅₀ (ng/mL) |
|---|---|---|
| 1 | 295D9-1 | 2.376 |
| 2 | 66G10-2 | 2.471 |
| 3 | B-8 | 2.506 |
| 4 | 240B10-1 | 2.601 |
| 5 | B-2 | 2.715 |
| 6 | 71F3-3 | 2.860 |
| 7 | B-1 | 3.135 |
| 8 | B-4 | 397.380 |

**Table 5. Graph of binding epitope analysis results of monoclonal antibodies of 94C4, 39B4, 8D1, 65G6, 295D9-1, 98G10, B-8, 240B10-1, B-2, and 71F3-3**

| Epitope 1 (Epitope 1) | Epitope 2 (Epitope 2) | | Epitope 3 (Epitope 3) | Epitope 4 (Epitope 4) | Epitope 5 (Epitope 5) | Undetermined (unknown) |
|---|---|---|---|---|---|---|
| 94C4 | 39B4 | 8D1 | 98G10 | B-8 | 240B10-1 | 71F3-3 |
| / | 65G6 | 295D9-1 | / | / | B-2 | / |

Finally, 94C4, B-8, 240B10-1, 98G10, and 39B4 (epitopes are different from each other) were selected to determine the sequences of their heavy chain variable regions and light chain variable regions, and the results were as follows:
The nucleotide sequence of the heavy chain variable region of 94C4 (containing the signal peptide) was: *ATGGAGACTGGGCTGCGCTGGCTTCTCCTGGTCGCTGTGCTCAAAGGTGTCCAGTGT*CAGTCGTTGGAGG AGTCCGGGGGAGGCCTGGTCCAGCCTGAGGGATCCCTGACACTCACCTGCAAAGCCTCTGGATTCGA CTTCAGTAGCAATGCAATGTGCTGGGTCCGCCAGGCTCCAGGGAAGGGGCTGGAGTGGATCGCATGC ATTAGTAGTGCTAGTAGTGGTAGTAGTGGTAGCACTTACTACGCGAGCTGGGCGAAAGGCCGATTCAC CATCTCCAAAACCTCGTCGACCACGGTGACTCTGCAAATGACCAGTCTGACAGCCGCGGACATGGCC ACCTATTTCTGTGCGAGAGATGGTTACGACTTGTGGGGCCCAGGCACCCTGGTCACCGTCTCCTCA (SEQ ID NO: 1), where the italicized portion was the signal peptide sequence and the underlined portion was the nucleotide sequence of the heavy chain variable region (SEQ ID NO: 2); the corresponding amino acid sequence was: *METGLRWLLLVAVLKGVQC*QSLEESGGGLVQPEGSLTLTCKASGFDFSSNAMCWVRQAPGKGLEWIACISS ASSGSSGSTYYASWAKGRFTISKTSSTTVTLQMTSLTAADMATYFCARDGYDLWGPGTLVTVSS (SEQ ID NO: 3), where the italicized portion was the signal peptide sequence and the underlined portion was the amino acid sequence of the heavy chain variable region (SEQ ID NO: 4); CDR-H1, CDR-H2, and CDR-H3 in the heavy chain variable region defined by different CDR Definition Schemes were as shown in Table 6.

**Table 6. CDR-H1, CDR-H2, and CDR-H3 in the heavy chain variable region defined by different CDR Definition Schemes**

| CDR Definition Scheme | CDR-H1 | CDR-H2 | CDR-H3 |
|---|---|---|---|
| Kabat | SNAMC (SEQ ID NO: 5) | CISSASSGSSGSTYYASWAKG (SEQ ID NO: 6) | DGYDL (SEQ ID NO: 7) |
| IMGT | GFDFSSNA (SEQ ID NO: 8) | ISSASSGSSGST (SEQ ID NO: 9) | ARDGYDL (SEQ ID NO: 10) |
| Chothia | GFDFSSN (SEQ ID NO: 11) | SSASSGSSGS (SEQ ID NO: 12) | DGYDL (SEQ ID NO: 7) |
| Contact | SSNAMC (SEQ ID NO: 13) | WIACISSASSGSSGSTY (SEQ ID NO: 14) | ARDGYD (SEQ ID NO: 15) |
| Abm | GFDFSSNAMC (SEQ ID NO: 16) | CISSASSGSSGSTY (SEQ ID NO: 17) | DGYDL (SEQ ID NO: 7) |

The nucleotide sequence of the light chain variable region of 94C4 (containing the signal peptide) was: 
ATGGACACGAGGGCCCCCACTCAGCTGCTGGGGCTCCTGCTGCTCTGGCTCCCAGGTGCCACATTTGCCC
AAGTGCTGACCCAGACTCCATCCTCCGTGTCTGCAGCTGTGGGAGGCACAGTCACCATCAGTTGTCA
GTCCAGTCCGAGTGTTTATAATAACTACTTATCCTGGTTTCAGCAGAAACCAGGGCAGTCTCCCAAGC
TCCTGATCTACAGGGTATCCACTCTGGCATCTGGGGTCCCATCGCGGTTCAGCGGCAGTGGATCTGGG
ACACAGTTCACTCTCACCATCAGCGGCGTGCAGTGTGACGATGCTGCCACTTATTACTGTGCAGGCGG
TTATGATAGCGCTAGTGATACATATGTTTTCGGCGGAGGGACCGAGGTGGTGGTCAAA (SEQ ID NO: 18),
 where the italicized portion was the signal peptide sequence and the underlined portion was the nucleotide sequence of the light chain variable region (SEQ ID NO: 19); the corresponding amino acid sequence was: *MDTRAPTQLLGLLLLWLPGATFA*QVLTQTPSSVSAAVGGTVTISCQSSPSVYNNYLSWFQQKPGQSPKLLIY RVSTLASGVPSRFSGSGSGTQFTLTISGVQCDDAATYYCAGGYDSASDTYVFGGGTEVVVK (SEQ ID NO: 20), where the italicized portion was the signal peptide sequence and the underlined portion was the amino acid sequence of the light chain variable region (SEQ ID NO: 21); CDR-L1, CDR-L2, and CDR-L3 in the light chain variable region defined by different CDR Definition Schemes were as shown in Table 7.

**Table 7. CDR-L1, CDR-L2, and CDR-L3 in the light chain variable region defined by different CDR Definition Schemes**

| CDR Definition Scheme | CDR-L1 | CDR-L2 | CDR-L3 |
|---|---|---|---|
| Kabat | QSSPSVYNNYLS (SEQ ID NO: 22) | RVSTLAS (SEQ ID NO: 23) | AGGYDSASDTYV (SEQ ID NO: 24) |
| IMGT | PSVYNNY (SEQ ID NO: 25) | RVS | AGGYDSASDTYV (SEQ ID NO: 24) |
| Chothia | QSSPSVYNNYLS (SEQ ID NO: 22) | RVSTLAS (SEQ ID NO: 23) | AGGYDSASDTYV (SEQ ID NO: 24) |
| Contact | YNNYLSWF (SEQ ID NO: 26) | LLIYRVSTLA (SEQ ID NO: 27) | AGGYDSASDTY (SEQ ID NO: 28) |
| Abm | QSSPSVYNNYLS (SEQ ID NO: 22) | RVSTLAS (SEQ ID NO: 23) | AGGYDSASDTYV (SEQ ID NO: 24) |

The nucleotide sequence of the heavy chain variable region of B-8 (containing the signal peptide) was: *ATGGAGACTGGGCTGCGCTGGCTTCTCCTGGTCGCTGTGCTCAAAGGTGTCCAGTGT*CAGTCGTTGGAGG AGTCCGGGGGAGACCTGGTCAAGCCTGGGGCATCCCTGACACTCACCTGCACAGCCTCTGGATTCTC CTTTAGTAGCAGCTACTACATGTGCTGGGTCCGCCAGGCTCCAGGGAAGGGGCTGGAGTGGATCGCA TGCATTTATGCTGGTAGTAGTGGTAGCACTTACTACGCGAGCTGGGCGAAAGGCCGATTCACCATCTC CAAAACCTCGTCGACCACGGTGACTCTGCAGATGACCAGTCTGACAGCCGCGGACACGGCCACTTAT TTCTGTGCGAGGGAGTATAGCTACGATGACTATGGTGATTTCTGGGGTTTTAACTTGTGGGGCCCAGG CACCCTGGTCACCGTCTCCTCA (SEQ ID NO: 29), where the italicized portion was the signal peptide sequence and the underlined portion was the nucleotide sequence of the heavy chain variable region (SEQ ID NO: 30); the corresponding amino acid sequence was: *METGLRWLLLVAVLKGVQC*QSLEESGGDLVKPGASLTLTCTASGFSFSSSYYMCWVRQAPGKGLEWIACIY AGSSGSTYYASWAKGRFTISKTSSTTVTLQMTSLTAADTATYFCAREYSYDDYGDFWGFNLWGPGTLVTV SS (SEQ ID NO: 31), where the italicized portion was the signal peptide sequence and the underlined portion was the amino acid sequence of the heavy chain variable region (SEQ ID NO: 32); CDR-H1, CDR-H2, and CDR-H3 in the heavy chain variable region defined by different CDR Definition Schemes were as shown in Table 8.

**Table 8. CDR-H1, CDR-H2, and CDR-H3 in the heavy chain variable region defined by different CDR Definition Schemes**

| CDR Definition Scheme | CDR-H1 | CDR-H2 | CDR-H3 |
|---|---|---|---|
| Kabat | SSYYMC (SEQ ID NO: 33) | CIYAGSSGSTYYASWAKG (SEQ ID NO: 34) | EYSYDDYGDFWGFNL (SEQ ID NO: 35) |
| IMGT | GFSFSSSYY (SEQ ID NO: 36) | IYAGSSGST (SEQ ID NO: 37) | AREYSYDDYGDFWGFNL (SEQ ID NO: 38) |
| Chothia | GFSFSSSY (SEQ ID NO: 39) | YAGSSGS (SEQ ID NO: 40) | EYSYDDYGDFWGFNL (SEQ ID NO: 35) |
| Contact | SSSYYMC (SEQ ID NO: 41) | WIACIYAGSSGSTY (SEQ ID NO: 42) | AREYSYDDYGDFWGFN (SEQ ID NO: 43) |
| Abm | GFSFSSSYYMC (SEQ ID NO: 44) | CIYAGSSGSTY (SEQ ID NO: 45) | EYSYDDYGDFWGFNL (SEQ ID NO: 35) |

The nucleotide sequence of the light chain variable region of B-8 (containing the signal peptide) was: *ATGGACACGAGGGCCCCCACTCAGCTGCTGGGGCTCCTGCTGCTCTGGCTCCCAGGTGCCAGATGT*GCCC TTGTGATGACCCAGACTCCATCCTCCGTGTCTGCAGCTGTGGGAGGCACAGTCACCATCAATTGCCAG GCCAGTCAGAACATTTACACCAATTTAGCCTGGTATCAGCAGAAACCAGGGCAGCCTCCCAAGCTCCT GATCTATGGTGCATCCAATCTGGAATCTGGGGTCCCATCGCGGTTCAAAGGCAGTGGATCTGGGACAG AATACACTCTCACCATCAGCGACCTGGAGTGTGACGATGCTGCCACTTATTACTGTCAAAGTTCTTTGT ATAGTCGTATTGCTGACCGGGCTAATGCTTTCGGCGGAGGGACCGAGGTGGTGGTCAAA (SEQ ID NO: 46), where the italicized portion was the signal peptide sequence and the underlined portion was the nucleotide sequence of the light chain variable region (SEQ ID NO: 47); the corresponding amino acid sequence was: *MDTRAPTQLLGLLLLWLPGARC*ALVMTQTPSSVSAAVGGTVTINCQASQNIYTNLAWYQQKPGQPPKLLIY GASNLESGVPSRFKGSGSGTEYTLTISDLECDDAATYYCQSSLYSRIADRANAFGGGTEVVVK (SEQ ID NO: 48), where the italicized portion was the signal peptide sequence and the underlined portion was the amino acid sequence of the light chain variable region (SEQ ID NO: 49); CDR-L1, CDR-L2, and CDR-L3 in the light chain variable region defined by different CDR Definition Schemes were as shown in Table 9.

**Table 9. CDR-L1, CDR-L2, and CDR-L3 in the light chain variable region defined by different CDR Definition Schemes**

| CDR Definition Scheme | CDR-L1 | CDR-L2 | CDR-L3 |
|---|---|---|---|
| Kabat | QASQNIYTNLA (SEQ ID NO: 50) | GASNLES (SEQ ID NO: 51) | QSSLYSRIADRANA (SEQ ID NO: 52) |
| IMGT | QNIYTN (SEQ ID NO: 53) | GAS | QSSLYSRIADRANA (SEQ ID NO: 52) |
| Chothia | QASQNIYTNLA (SEQ ID NO: 50) | GASNLES (SEQ ID NO: 51) | QSSLYSRIADRANA (SEQ ID NO: 52) |
| Contact | YTNLAWY (SEQ ID NO: 54) | LLIYGASNLE (SEQ ID NO: 55) | QSSLYSRIADRAN (SEQ ID NO: 56) |
| Abm | QASQNIYTNLA (SEQ ID NO: 50) | GASNLES (SEQ ID NO: 51) | QSSLYSRIADRANA (SEQ ID NO: 52) |

The nucleotide sequence of the heavy chain variable region (containing the signal peptide) of 240B10-1 was: *ATGGAGACTGGGCTGCGCTGGCTTCTCCTGGTCGCTGTGCTCAGAGGTGTCCAGTGT*CAGTCGCTGGAG GAGTCCGGGGGTCGCCTGGTCACGCCTGGAGGATCCCTGACACTCACCTGCACAGTCTCTGGAATCG ACCTCAGTAGCTATACAATGGGCTGGGTCCGCCAGGCTCCAGGGAAGGGACTTGAATACATCGGAATC ATTAGTGGTGGTGGTAGGACATACTACGCGAGCTGGGCGAAAGGCCGATTCACCATCTCCAAAACCTC GTCGACCACGGTGGATCTGAAAGTGACCAGTCTGACAACCGAGGACACGGCCACCTATTTCTGTGCC AGAATATATACCTTGTGGGGCCAAGGCACCCTGGTCACCGTCTCCTCG (SEQ ID NO: 57), where the italicized portion was the signal peptide sequence and the underlined portion was the nucleotide sequence of the heavy chain variable region (SEQ ID NO: 58); the corresponding amino acid sequence was: 
METGLRWLLLVAVLRGVQCQSLEESGGRLVTPGGSLTLTCTVSGIDLSSYTMGWVRQAPGKGLEYIGIISGG
GRTYYASWAKGRFTISKTSSTTVDLKVTSLTTEDTATYFCARIYTLWGQGTLVTVSS (SEQ ID NO: 59),
 where the italicized portion was the signal peptide sequence and the underlined portion was the amino acid sequence of the heavy chain variable region (SEQ ID NO: 60); CDR-H1, CDR-H2, and CDR-H3 in the heavy chain variable region defined by different CDR Definition Schemes were as shown in Table 10.

**Table 10. CDR-H1, CDR-H2, and CDR-H3 in the heavy chain variable region defined by different CDR Definition Schemes**

| CDR Definition Scheme | CDR-H1 | CDR-H2 | CDR-H3 |
|---|---|---|---|
| Kabat | SYTMG (SEQ ID NO: 61) | IISGGGRTYYASWAKG (SEQ ID NO: 62) | IYTL (SEQ ID NO: 63) |
| IMGT | GIDLSSYT (SEQ ID NO: 64) | ISGGGRT (SEQ ID NO: 65) | ARIYTL (SEQ ID NO: 66) |
| Chothia | GIDLSSY (SEQ ID NO: 67) | SGGGR (SEQ ID NO: 68) | IYTL (SEQ ID NO: 63) |
| Contact | SSYTMG (SEQ ID NO: 69) | YIGIISGGGRTY (SEQ ID NO: 70) | ARIYT (SEQ ID NO: 71) |
| Abm | GIDLSSYTMG (SEQ ID NO: 72) | IISGGGRTY (SEQ ID NO: 73) | IYTL (SEQ ID NO: 63) |

The nucleotide sequence of the light chain variable region (containing the signal peptide) of 240B10-1 was: *ATGGACACGAGGGCCCCCACTCAGCTGCTGGGGCTCCTGCTGCTCTGGCTCCCAGGTGCCACATTTGCC*C AAGTGCTGACCCAGACTCTAGCCTCCGTGTCTGCGGCTGTTGGAGGCACAGTCACCATCAATTGCCA GTCCAGTCAGAGTGTTTATAATAACGACTACCTAGCCTGGTTTCAGCAGAAATCAGGGCAGCCTCCCA AGCAACTGATCCATTCTGCATCCAAACTGGCATCTGGGGTCCCATCGCGGTTCAAAGGCAGTGGATCT GGGACACAGTTCACTCTCACCATCAACGACGTGCAGTGTGACGATGCTGCCACTTACTACTGTCTAGG CGGTTATGATTGTTATAGTGCTGATTGTAATGTTTTCGGCGGAGGGACCGAGGTGGTGGTCAAA (SEQ ID NO: 74), where the italicized portion was the signal peptide sequence and the underlined portion was the nucleotide sequence of the light chain variable region (SEQ ID NO: 75); the corresponding amino acid sequence was: *MDTRAPTQLLGLLLLWLPGATFA*QVLTQTLASVSAAVGGTVTINCQSSQSVYNNDYLAWFQQKSGQPPKQ LIHSASKLASGVPSRFKGSGSGTQFTLTINDVQCDDAATYYCLGGYDCYSADCNVFGGGTEVVVK(SEQ ID NO: 76), where the italicized portion was the signal peptide sequence and the underlined portion was the amino acid sequence of the light chain variable region (SEQ ID NO: 77); CDR-L1, CDR-L2, and CDR-L3 in the light chain variable region defined by different CDR Definition Schemes were as shown in Table 11.

**Table 11. CDR-L1, CDR-L2, and CDR-L3 in the light chain variable region defined by different CDR Definition Schemes**

| CDR Definition Scheme | CDR-L1 | CDR-L2 | CDR-L3 |
|---|---|---|---|
| Kabat | QSSQSVYNNDYLA (SEQ ID NO: 78) | SASKLAS (SEQ ID NO: 79) | LGGYDCYSADCNV (SEQ ID NO: 80) |
| IMGT | QSVYNNDY (SEQ ID NO: 81) | SAS | LGGYDCYSADCNV (SEQ ID NO: 80) |
| Chothia | QSSQSVYNNDYLA (SEQ ID NO: 78) | SASKLAS (SEQ ID NO: 79) | LGGYDCYSADCNV (SEQ ID NO: 80) |
| Contact | YNNDYLAWF (SEQ ID NO: 82) | QLIHSASKLA (SEQ ID NO: 83) | LGGYDCYSADCN (SEQ ID NO: 84) |
| Abm | QSSQSVYNNDYLA (SEQ ID NO: 78) | SASKLAS (SEQ ID NO: 79) | LGGYDCYSADCNV (SEQ ID NO: 80) |

The nucleotide sequence of the heavy chain variable region of 98G10 (containing the signal peptide) was: *ATGGAGACTGGGCTGCGCTGGCTTCTCCTGGTCGCTGTGCTCAAAGGTGTCCAGTGT*CAGGAGCAGCTG GAGGAGTCCGGGGGAGACCTGGTCAAGCCTGAGGGATCCCTGACACTCACCTGCACAGCCTCTGGAT TCTCCTTCAGTGCCAGCTACTGGATATGCTGGGTCCGCCAGGCTCCAGGGAAGGGCCTGGAGTGGAT CGCATGCATTTATGGTGGTAGTAGTGGTAGCGCTTACTACGCGAGCTGGGCGAAGGGCCGATTCACCA TCTCCAAAACCTCGTCGACCACGGTGACTCTGCAAATGACCAGTCTGACAGCCGCGGACACGGCCAC TTATTTCTGTGCGAGAGCCGGTTACTATAATTATGGTAGTGCTGGTGATATTTATCCTAGTTCCTTTAAGT TGTGGGGCCCAGGCACCCTGGTCACCGTCTCCTCA (SEQ ID NO: 85), where the italicized portion was the signal peptide sequence and the underlined portion was the nucleotide sequence of the heavy chain variable region (SEQ ID NO: 86); the corresponding amino acid sequence was: *METGLRWLLLVAVLKGVQC*QEQLEESGGDLVKPEGSLTLTCTASGFSFSASYWICWVRQAPGKGLEWIACI YGGSSGSAYYASWAKGRFTISKTSSTTVTLQMTSLTAADTATYFCARAGYYNYGSAGDIYPSSFKLWGPG TLVTVSS (SEQ ID NO: 87), where the italicized portion was the signal peptide sequence and the underlined portion was the amino acid sequence of the heavy chain variable region (SEQ ID NO: 88); CDR-H1, CDR-H2, and CDR-H3 in the heavy chain variable region defined by different CDR Definition Schemes were as shown in Table 12.

**Table 12. CDR-H1, CDR-H2, and CDR-H3 in the heavy chain variable region defined by different CDR Definition Schemes**

| CDR Definition Scheme | CDR-H1 | CDR-H2 | CDR-H3 |
|---|---|---|---|
| Kabat | ASYWIC (SEQ ID NO: 89) | CIYGGSSGSAYYASWAKG (SEQ ID NO: 90) | AGYYNYGSAGDIYPSSFKL (SEQ ID NO: 91) |
| IMGT | GFSFSASYW (SEQ ID NO: 92) | IYGGSSGSA (SEQ ID NO: 93) | ARAGYYNYGSAGDIYPSSFKL (SEQ ID NO: 94) |
| Chothia | GFSFSASY (SEQ ID NO: 95) | YGGSSGS (SEQ ID NO: 96) | AGYYNYGSAGDIYPSSFKL (SEQ ID NO: 91) |
| Contact | SASYWIC (SEQ ID NO: 97) | WIACIYGGSSGSAY (SEQ ID NO: 98) | ARAGYYNYGSAGDIYPSSFK (SEQ ID NO: 99) |
| Abm | GFSFSASYWIC (SEQ ID NO: 100) | CIYGGSSGSAY (SEQ ID NO: 101) | AGYYNYGSAGDIYPSSFKL (SEQ ID NO: 91) |

The nucleotide sequence of the light chain variable region of 98G10 (containing the signal peptide) was: *ATGGACACGAGGGCCCCCACTCAGCTGCTGGGGCTCCTACTGCTCTGGCTCCCAGGTGCCAGATGT*GACA TTGTGATGACCCAGACTCCAGCCTCCGTGGAGGCAGCTGTGGGAGGCACAGTCACCATCAATTGCCA GGCCAGTCAGAGCATTAGCACTGCATTAGCCTGGTATCAGCAGAAACCAGGGCAGCCTCCCAAGCTC CTGATCTATACTGCATCCAAGGTGGCATCTGGGGTCCCATCGCGGTTCAGCGGCAGTGGATCTGGGAC ACAGTTCACTCTCACCATCAGCGACCTGGAGTGTGCCGATGCTGCCACTTACTACTGTCAAAACTATT ATTATAGTAGTAGTAGTATTACTTTCGGCGGAGGGACCGAGGTGGTGGTCAAA (SEQ ID NO: 102), where the italicized portion was the signal peptide sequence and the underlined portion was the nucleotide sequence of the light chain variable region (SEQ ID NO: 103); the corresponding amino acid sequence was: 
MDTRAPTQLLGLLLLWLPGARCDIVMTQTPASVEAAVGGTVTINCQASQSISTALAWYQQKPGQPPKLLIY
TASKVASGVPSRFSGSGSGTQFTLTISDLECADAATYYCQNYYYSSSSITFGGGTEVVVK (SEQ ID NO: 104),
 where the italicized portion was the signal peptide sequence and the underlined portion was the amino acid sequence of the light chain variable region (SEQ ID NO: 105); CDR-L1, CDR-L2, and CDR-L3 in the light chain variable region defined by different CDR Definition Schemes were as shown in Table 13.

**Table 13. CDR-L1, CDR-L2, and CDR-L3 in the light chain variable region defined by different CDR Definition Schemes**

| CDR Definition Scheme | CDR-L1 | CDR-L2 | CDR-L3 |
|---|---|---|---|
| Kabat | QASQSISTALA (SEQ ID NO: 106) | TASKVAS (SEQ ID NO: 107) | QNYYYSSSSIT (SEQ ID NO: 108) |
| IMGT | QSISTA (SEQ ID NO: 109) | TAS | QNYYYSSSSIT (SEQ ID NO: 108) |
| Chothia | QASQSISTALA (SEQ ID NO: 106) | TASKVAS (SEQ ID NO: 107) | QNYYYSSSSIT (SEQ ID NO: 108) |
| Contact | STALAWY (SEQ ID NO: 110) | LLIYTASKVA (SEQ ID NO: 111) | QNYYYSSSSI (SEQ ID NO: 112) |
| Abm | QASQSISTALA (SEQ ID NO: 106) | TASKVAS (SEQ ID NO: 107) | QNYYYSSSSIT (SEQ ID NO: 108) |

The nucleotide sequence of the heavy chain variable region of 39B4 (containing the signal peptide) was: *ATGGAGACTGGGCTGCGCTGGCTTCTCCTGGTCGCTGTGCTCAAAGGTGTCCAGTGT*CAGGAGCAGCTG GTGGAGTCCGGGGGAGGCCTGGTCCAGCCTGAGGGATCCCTGACACTCACCTGCACAGCTTCTGGAT TCTCCTTCAGTAGTAGTTATTGGATATGCTGGGTCCGCCAGGCTCCAGGGAAGGGGCTGGAGTGGATC GCATGCATTTATGCTGGTACTAGTGGTAGCACTTACTACGCGAGCTGGGCGAAAGGCCGATTCACCAT CTCCAAAACCTCGTCGACCACTGTGGATCTTCAAATGAACAGTCTGACAGCGGCGGACACGGCCACC TATTTCTGTGCGAGAGCCGGTTACTATACTTATGGTGCTGGTGTTTATATTTATCCTAGTTCCTTTAAGTT GTGGGGCCCAGGCACCCTGGTCACCGTCTCCTCA (SEQ ID NO: 113), where the italicized portion was the signal peptide sequence and the underlined portion was the nucleotide sequence of the heavy chain variable region (SEQ ID NO: 114); the corresponding amino acid sequence was: *METGLRWLLLVAVLKGVQC*QEQLVESGGGLVQPEGSLTLTCTASGFSFSSSYWICWVRQAPGKGLEWIACI YAGTSGSTYYASWAKGRFTISKTSSTTVDLQMNSLTAADTATYFCARAGYYTYGAGVYIYPSSFKLWGPG TLVTVSS (SEQ ID NO: 115), where the italicized portion was the signal peptide sequence and the underlined portion was the amino acid sequence of the heavy chain variable region (SEQ ID NO: 116); CDR-H1, CDR-H2, and CDR-H3 in the heavy chain variable region defined by different CDR Definition Schemes were as shown in Table 14.

**Table 14. CDR-H1, CDR-H2, and CDR-H3 in the heavy chain variable region defined by different CDR Definition Schemes**

| CDR Definition Scheme, | CDR-H1 | CDR-H2 | CDR-H3 |
|---|---|---|---|
| Kabat | SSYWIC (SEQ ID NO: 117) | CIYAGTSGSTYYASWAKG (SEQ ID NO: 118) | AGYYTYGAGVYIYPSSFKL (SEQ ID NO: 119) |
| IMGT | GFSFSSSYW (SEQ ID NO: 120) | IYAGTSGST (SEQ ID NO: 121) | ARAGYYTYGAGVYIYPSSFKL (SEQ ID NO: 122) |
| Chothia | GFSFSSSY (SEQ ID NO: 120) | YAGTSGS (SEQ ID NO: 123) | AGYYTYGAGVYIYPSSFKL (SEQ ID NO: 119) |
| Contact | SSSYWIC (SEQ ID NO: 117) | WIACIYAGTSGSTY (SEQ ID NO: 124) | ARAGYYTYGAGVYIYPSSFK (SEQ ID NO: 125) |
| Abm | GFSFSSSYWIC (SEQ ID NO: 126) | CIYAGTSGSTY (SEQ ID NO: 127) | AGYYTYGAGVYIYPSSFKL (SEQ ID NO: 128) |

The nucleotide sequence of the light chain variable region of 39B4 (containing the signal peptide) was: *ATGGACACGAGGGCCCCCACTCAGCTGCTGGGGCTCCTACTGCTCTGGCTCCCAGGTGCCAGATGT*GATG TCGTGATGACCCAGACTCCAGCCTCCGTGGAGGCAGCTGTGGGAGGCACAGTCACCATCAAGTGCCA GGCCAGTGAGAGCATTGGCAATGCATTAGCCTGGTATCAGCAGAAACCAGGACAGCCTCCCAAGCTC CTGATCTATTCTGCATCCAATCTGGCATCTGGGGTCCCATCGCGGTTCAGTGGCAGTGGATCTGGGACA CAGTTCACTCTCACCATCAGCGACCTGGAGTGTGCCGATGCTGCCACTTACTACTGTCAAAGCTATTAT TATAGTAGTAGTAGTATTACTTTCGGCGGAGGGACCGAGGTGGTGGTCAAA (SEQ ID NO: 129), where the italicized portion was the signal peptide sequence and the underlined portion was the nucleotide sequence of the light chain variable region (SEQ ID NO: 130); the corresponding amino acid sequence was: *MDTRAPTQLLGLLLLWLPGARC*DVVMTQTPASVEAAVGGTVTIKCQASESIGNALAWYQQKPGQPPKLLI YSASNLASGVPSRFSGSGSGTQFTLTISDLECADAATYYCQSYYYSSSSITFGGGTEVVVK (SEQ ID NO: 131), where the italicized portion was the signal peptide sequence and the underlined portion was the amino acid sequence of the light chain variable region (SEQ ID NO: 132); CDR-L1, CDR-L2, and CDR-L3 in the light chain variable region defined by different CDR Definition Schemes were as shown in Table 15.

**Table 15. CDR-L1, CDR-L2, and CDR-L3 in the light chain variable region defined by different CDR Definition Schemes**

| CDR Definition Scheme | CDR-L1 | CDR-L2 | CDR-L3 |
|---|---|---|---|
| Kabat | QASESIGNALA (SEQ ID NO: 133) | SASNLAS (SEQ ID NO: 134) | QSYYYSSSSIT (SEQ ID NO: 135) |
| IMGT | ESIGNA (SEQ ID NO: 136) | SAS | QSYYYSSSSIT (SEQ ID NO: 135) |
| Chothia | QASESIGNALA (SEQ ID NO: 133) | SASNLAS (SEQ ID NO: 134) | QSYYYSSSSIT (SEQ ID NO: 135) |
| Contact | GNALAWY (SEQ ID NO: 137) | LLIYSASNLA (SEQ ID NO: 138) | QSYYYSSSSI (SEQ ID NO: 139) |
| Abm | QASESIGNALA (SEQ ID NO: 133) | SASNLAS (SEQ ID NO: 134) | QSYYYSSSSIT (SEQ ID NO: 135) |

### Example 2. Validation of the effect of anti-NMI monoclonal antibody

### I. Preparation Method of Antibody was as follows:

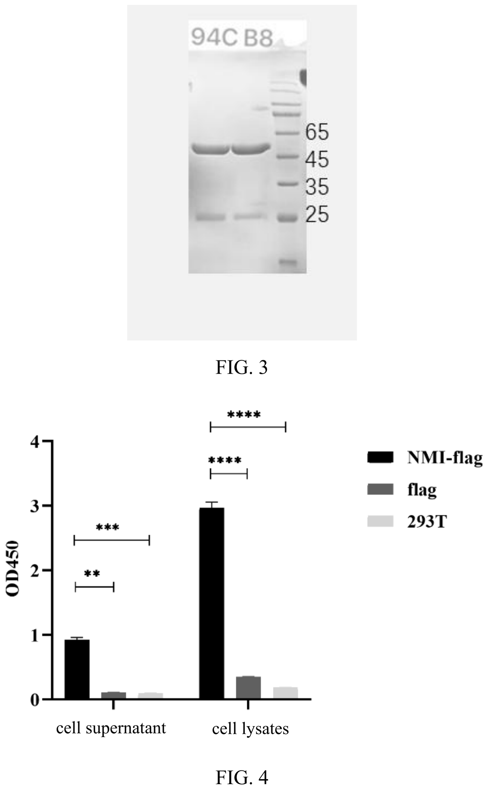

(1) Cell transfection: Transfection was performed when cell density reached 4.0×10⁶ cells/mL; prior to the transfection, original medium should be removed by centrifugation and supplemented with fresh medium. The following was an example of transfection of 100 mL cells:
   1) a total of 100 µg of antibody plasmid at a ratio of heavy chain: light chain of 1:2 was added into 5 mL of serum-free medium, and incubated at room temperature for 5 min;
   2) a total of 300 µg of PEI at a ratio of plasmid: PEI of 1:3 was added into 5 mL of serum-free medium, and incubated at room temperature for 5 min;
   3) the incubated plasmid and PEI were mixed thoroughly, incubated for 15 min, and added to the cells;
(2) Antibody purification: 6 days after transfection, the culture supernatant was collected based on cell culture status;
   1) centrifuged at 2000 g, 4 °C for 20 min to collect the cell culture supernatant;
   2) Protein G beads or Protein A beads pre-treated with binding buffer (20 mM sodium phosphate, pH 7.0) were added to the supernatant, and incubated with rotation at 4 °C for 2 h to ensure sufficient antibody binding to the beads;
   3) centrifuged at 3000 rpm, 4 °C to collect the beads, and washed for three times with binding buffer to remove impurity proteins;
   4) the antibodies were eluted from the beads by using elution buffer (0.1 M glycine-HCl, pH 2.9), and 100 µL of 1 M Tris-HCl (pH 9.0) for every 1 mL of the eluate was added to adjust the pH; the antibodies could be obtained (where SDS-PAGE results after preparation of the antibodies B8 and 94C4 were as shown in FIG. 3).

### II. Affinity test

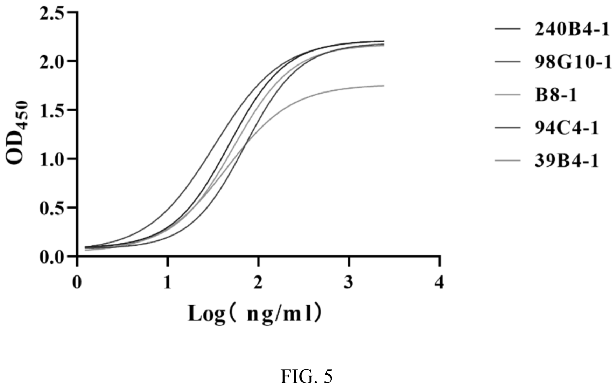

The affinities of 94C4, B-8, 240B10-1, 98G10, and 39B4 were measured using an I-ELISA assay as detailed below: 1) Antigen Coating: antigen (e.g., hNMI) was diluted with PBS to 1 µg/mL and added to ELISA plate for coating (100 µL/well) overnight at 4°C; 2) Blocking: the antigen solution was discarded, and the ELISA plate was washed for four times with PBST (250 µL/well) with 3 min of incubation per wash, residual solution was removed by plate tapping, 5% BSA formulated with PBS was added (200 µL/well), the ELISA plate was sealed with a sealing film and blocked at 37°C for 2 hours; 3) Incubation of Primary Antibody: the blocking solution was discarded, the ELISA plate was washed for four times with PBST (250 µL/well), primary antibody solution (100 µL/well, at an initial concentration of 2.5 µg/mL, followed by 2-fold gradient dilution with a total of 12 gradients and the dilution solution of PBS, 1× PBS served as the negative control) was added, the ELISA plate was sealed with a sealing film and incubated at 37°C for 1 hour; 4) Incubation of Secondary Antibody: the primary antibody solution was discarded, the ELISA plate was washed for four times with PBST (250 µL/well), 100 µL/well of the corresponding HRP-labeled secondary antibody dilution solution (anti-rabbit IgG HRP-Linked antibody, 1:3000) was added, the ELISA plate was sealed with a sealing film and incubated at 37°C for 1 hour; 5) Color Development: the secondary antibody dilution solution was discarded, the ELISA plate was washed for six times with PBST (250 µL/well), residual solution was removed by plate tapping, TMB (90 µL/well) was added, and incubated for 20 min under dark; 6) Termination and Reading: 2 mol/L of H₂SO₄ (50 µL/well) was added, and the OD450 value was read using a microplate reader. The results were as shown in Table 16. Nonlinear regression analysis was performed on the above data with the logarithm of antibody concentration as the abscissa and the measured OD₄₅₀ values at each dilution as the ordinate, and the results were as shown in FIG. 5 and Table 17: EC50 values of 94C4, B-8, 240B10-1, 98G10, and 39B4 were from 30 ng/mL to 70 ng/mL (The results here were largely different from those in Example 1, which may be attributed to antibody storage at -20°C for one year and the brand of the chromogenic reagent was different from that used in Example 1); notably, 94C4 exhibited the strongest antigen-binding affinity, with an EC50 value of 32.72 ng/mL.

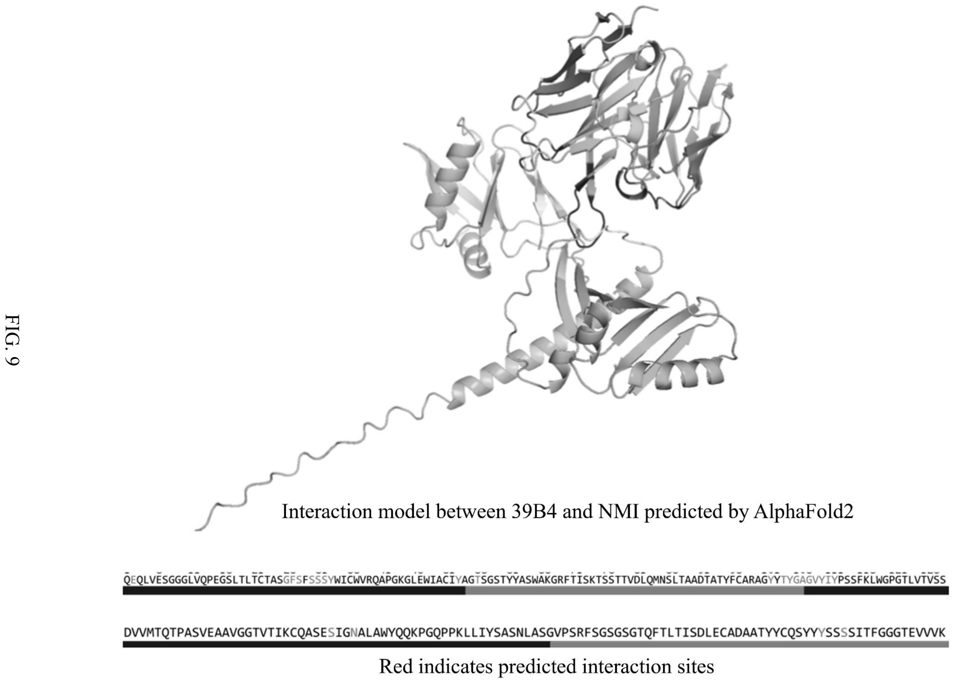

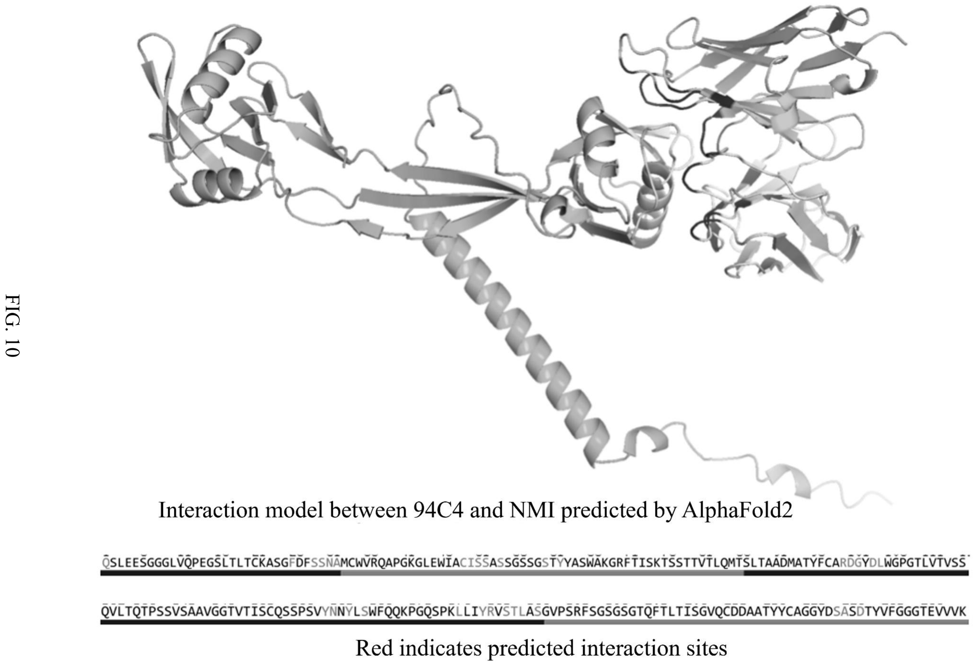

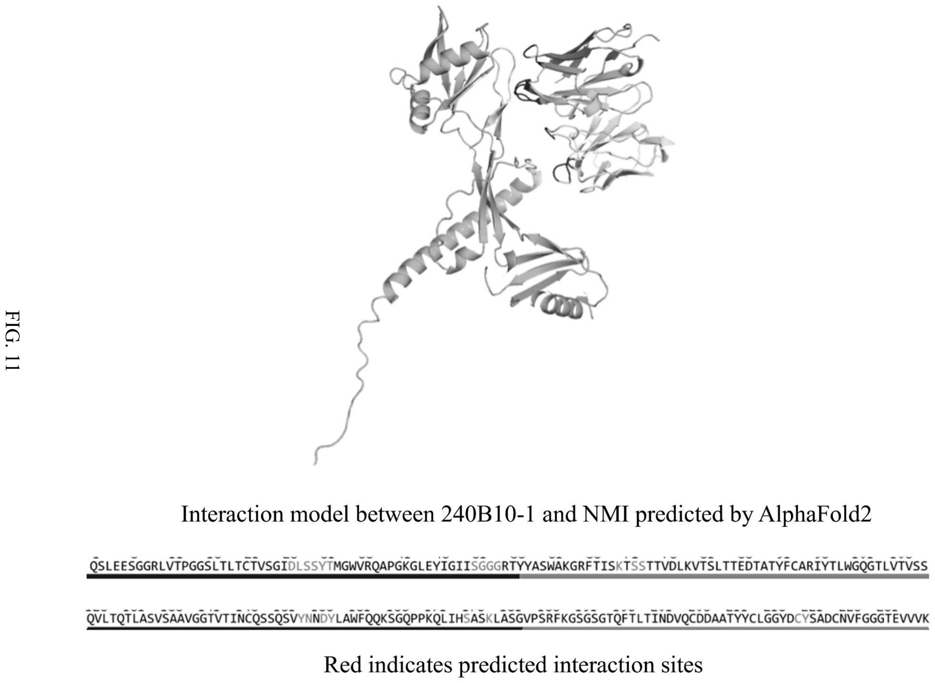

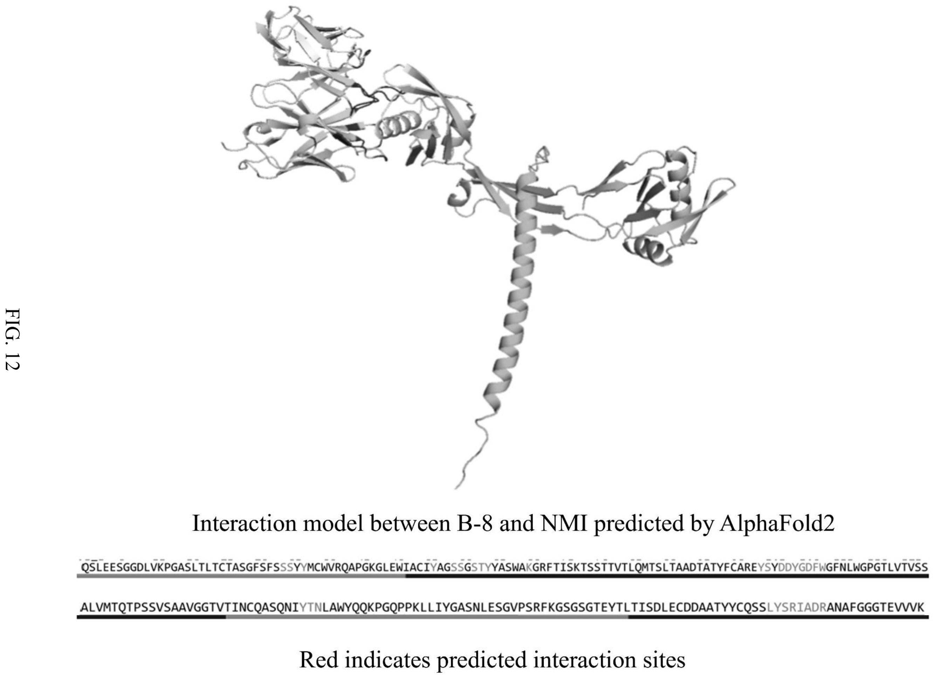

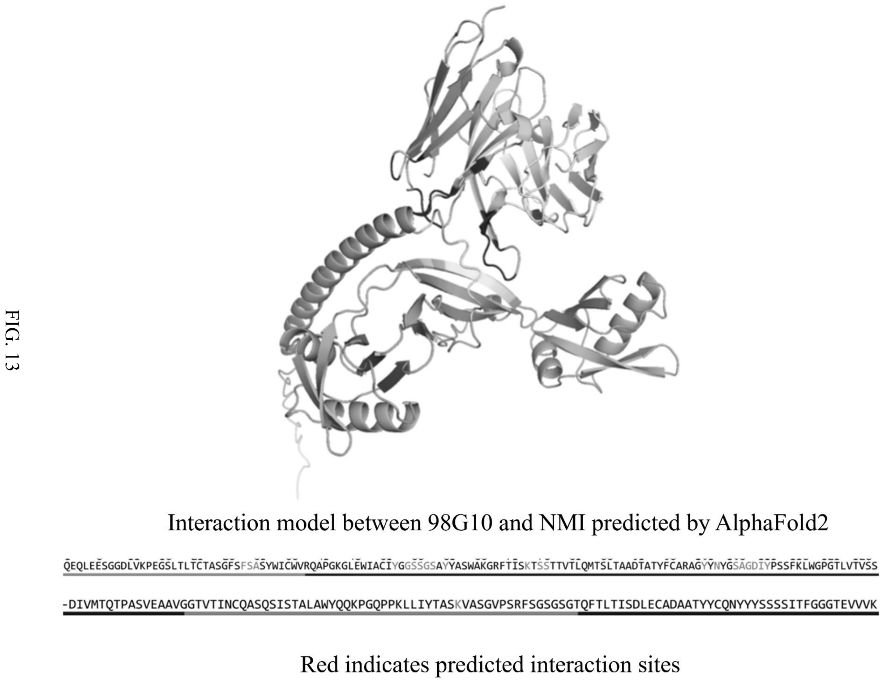

The inventors performed structural analysis with AlphaFold, and the results were as shown in FIGs. 9-13: it was found that the amino acids that bind to/recognize the antigen hNMI were some amino acids (hereinafter referred to as: key amino acids) of the above mentioned monoclonal antibodies (94C4, B-8, 240B10-1, 98G10, or 39B4): wherein, the critical sites of the monoclonal antibody 39B4 were: the following sites in the heavy chain variable region: E2 (E2 denotes the second amino acid E in the heavy chain variable region of monoclonal antibody 39B4, same applies below), G26, F27, S28, S30, S31, S32, Y33, Y53, T56, Y102, T104, Y105, G106, A107, G108, V109, Y110, I111, and Y112; and the following sites in the light chain variable region: S28, N31, Y93, and S96; the critical sites of the monoclonal antibody B-8 were: the following sites in the heavy chain variable region: S30, S31, Y33, Y52, S55, S56, S58, T59, Y60, K66, Y100, S101, D103, D104, Y105, G106, D107, F108, and W109, and the following sites in the light chain variable region: Y30, T31, N32, L92, Y93, S94, R95, 196, A97, D98, and R99; the critical sites of the monoclonal antibody 240B10-1 were: the following sites in the heavy chain variable region: D27, L28, S29, S30, Y31, T32, S51, G52, G53, G54, K70, S72, and S73, and the following sites in the light chain variable region: Y29, N30, D32, Y33, S51, K54, C75, and Y76; the critical sites of the monoclonal antibody 98G10 were: the following sites in the heavy chain variable region: F29, S30, A31, Y53, G55, S56, S57, G58, S59, Y61, K74, S76, S77, Y102, N104, S107, A108, G109, D110, I111, and Y112; the following sites in the light chain variable region: K53; the critical sites of the monoclonal antibody 94C4 were: the following sites in the heavy chain variable region: Q1, F26, S29, S30, N31, A32, C49, I50, S51, S52, S54, S60, Y62, R100, D101, G102, D104, and L105; the following sites in the light chain variable region: Y29, N30, Y32, S34, L46, Y49, R50, S52, T53, L54, S56, S94, A95, and D97. It is well known for those skilled in the art that the amino acids in a monoclonal antibody that binds to/recognizes the antigen hNMI is CDR of the monoclonal antibody. Therefore, in conjunction with the results of the AlphaFold structural analysis described above, it was further determined that the critical sites of the monoclonal antibody described above (94C4, B-8, 240B10-1, 98G10, or 39B4) were as follows: the critical sites of the monoclonal antibody 39B4 were: the following sites in the heavy chain variable region: G26 (G26 denotes the 26th amino acid G in the heavy chain variable region of monoclonal antibody 39B4, same applies below), F27, S28, S30, S31, S32, Y33, Y53, T56, Y102, T104, Y105, G106, A107, G108, V109, Y110, I111, and Y112; and the following sites in the light chain variable region: S28, N31, Y93, and S96; the critical sites of the monoclonal antibody B-8 were: the following sites in the heavy chain variable region: S30, S31, Y33, Y52, S55, S56, S58, T59, Y60, K66, Y100, S101, D103, D104, Y105, G106, D107, F108, and W109, and the following sites in the light chain variable region: Y30, T31, N32, L92, Y93, S94, R95, I96, A97, D98, and R99; the critical sites of the monoclonal antibody 240B10-1 were: the following sites in the heavy chain variable region: D27, L28, S29, S30, Y31, T32, S51, G52, G53, and G54, and the following sites in the light chain variable region: Y29, N30, D32, Y33, S51, K54, C75, and Y76; the critical sites of the monoclonal antibody 98G10 were: the following sites in the heavy chain variable region: F29, S30, A31, Y53, G55, S56, S57, G58, S59, Y61, Y102, N104, S107, A108, G109, D110, I111, and Y112; the following sites in the light chain variable region: K53; the critical sites of monoclonal antibody 94C4 were: the following sites in the heavy chain variable region: F26, S29, S30, N31, A32, C49, I50, S51, S52, S54, S60, Y62, R100, D101, G102, D104, and L105; the following sites in the light chain variable region: Y29, N30, Y32, S34, L46, Y49, R50, S52, T53, L54, S56, S94, A95, and D97.

To verify whether altering the critical sites of the monoclonal antibody 94C4 affects the function of this monoclonal antibody, the inventor performed point mutation on some critical sites (specific mutation information was shown in Table 17-1, other sequences were identical to the monoclonal antibody 94C4, taking mutation site of A32K as an example, only the 32nd amino acid in the heavy chain variable region was mutated relative to the monoclonal antibody 94C4, specifically: A at position 32 was mutated to K), and the results were as shown in Table 17-1: the function of the monoclonal antibody 94C4 was affected (exhibiting a significant decrease in binding affinity to the antigen NMI) upon the mutation of its critical sites (e.g., S29, A32, S52, and D101 in the heavy chain variable region, as well as D97 in the light chain variable region).

To verify whether altering the critical sites of the monoclonal antibody B-8 affects the function of this monoclonal antibody, the inventors performed point mutations on some critical sites (specific mutation information was shown in Table 17-2, other sequences were identical to the monoclonal antibody B-8), and the results were as shown in Table 17-2: the function of the monoclonal antibody B-8 was affected (exhibiting a significant decrease in binding affinity to the antigen NMI) upon the mutation of its critical sites (e.g., S101 and G106 in the heavy chain variable region, as well as T31 in the light chain variable region).

To verify whether altering the critical sites of the monoclonal antibody 240B10-1 affects the function of this monoclonal antibody, the inventors performed point mutations on some critical sites (specific mutation information was shown in Table 17-3, other sequences were identical to the monoclonal antibody 240B10-1), and the results were as shown in Table 17-3: the function of the monoclonal antibody 240B10-1 was affected (exhibiting a significant decrease in binding affinity to the antigen NMI) upon the mutation of its critical sites (e.g., D27, S29, S30, and G52 in the heavy chain variable region, as well as N30 in the light chain variable region).

To verify whether altering the critical sites of the monoclonal antibody 98G10 affects the function of this monoclonal antibody, the inventors performed point mutations on some critical sites (specific mutation information was shown in Table 17-4, other sequences were identical to the monoclonal antibody 98G10), and the results were as shown in Table 17-4: the function of the monoclonal antibody 98G10 was affected (exhibiting a significant decrease in binding affinity to the antigen NMI) upon the mutation of its critical sites (e.g., G55, S56, S57, and G58 in the heavy chain variable region).

To verify whether altering the critical sites of the monoclonal antibody 39B4 affects the function of this monoclonal antibody, the inventors performed point mutations on some critical sites (specific mutation information was shown in Table 17-5, other sequences were identical to the monoclonal antibody 39B4), and the results were as shown in Table 17-5: the function of the monoclonal antibody 39B4 was affected (exhibiting a significant decrease in binding affinity to the antigen NMI) upon the mutation of its critical sites (e.g., Y102, G106, A107, and V109 in the heavy chain variable region).

To verify whether altering (substituting, deleting and/or inserting) amino acids other than those at the critical sites of the monoclonal antibody 98G10 affects the function of this monoclonal antibody, the inventors altered amino acids other than those at the critical sites of the aforementioned monoclonal antibody 98G10 (specific mutation information was shown in Tables 17-6 (mutations occurred in the CDR regions) and 17-7 (mutations occurred in the non-CDR regions), and other sequences were identical to the monoclonal antibody 98G10, taking mutation site of W34H as an example, only the 34th amino acid in the heavy chain variable region was mutated relative to the monoclonal antibody 98G10, specifically: W at position 34 was mutated to H), and the results were as shown in Tables 17-6 and 17-7 (the differences in the results of 98G10 in Tables 17-6 and 17-7 were due to variations between experimental batches; similarly, in the present application, the differences in the results of the same antibody were also caused by experimental variations): it can be seen that mutations at these non-critical sites did not affect the function of the antibody, and the antibody still maintained strong binding affinity to the antigen hNMI.

To verify whether altering (substituting, deleting and/or inserting) amino acids other than those at the critical sites of the monoclonal antibody B8 affects the function of this monoclonal antibody, the inventors altered amino acids other than those at the critical sites of the aforementioned monoclonal antibody B8 (specific mutation information was shown in Table 17-8, and other sequences were identical to the monoclonal antibody B8, taking mutation site of Y93E as an example, only the 93rd amino acid in the heavy chain variable region was mutated relative to the monoclonal antibody B8, specifically: Y at position 93 was mutated to E), and the results were as shown in Table 17-8: it can be seen that mutations at these non-critical sites did not affect the function of the antibody, and the antibody still maintained strong binding affinity to the antigen hNMI.

**Table 16. Affinity of different antibodies to antigens by indirect Elisa assay**

| Antibody (ng/mL, OD450) | 2500 | 1250 | 625 | 312.5 | 156.2 5 | 78.12 5 | 39.06 25 | 19.53 125 | 9.765 625 | 4.882 8125 | 2.441 4063 | 1.220 7031 |
|---|---|---|---|---|---|---|---|---|---|---|---|---|
| 240B10-1 | 2.155 | 2.128 | 2.201 | 2.193 | 1.847 | 1.461 | 0.996 | 0.573 | 0.333 | 0.163 | 0.105 | 0.077 |
| 98G10-1 (98G10) | 2.174 | 2.155 | 2.079 | 1.969 | 1.714 | 1.18 | 0.67 | 0.41 | 0.217 | 0.115 | 0.081 | 0.065 |
| B8-1 (B-8) | 1.851 | 1.679 | 1.621 | 1.673 | 1.357 | 1.256 | 0.838 | 0.502 | 0.243 | 0.143 | 0.099 | 0.084 |
| 94C4-1 (94C4) | 2.171 | 2.185 | 2.185 | 2.096 | 1.93 | 1.644 | 1.22 | 0.833 | 0.482 | 0.262 | 0.142 | 0.092 |
| 39B4-1 (39B4) | 2.251 | 2.127 | 1.988 | 1.992 | 1.801 | 1.432 | 0.828 | 0.529 | 0.296 | 0.159 | 0.099 | 0.077 |
| PBS | 0.054 | 0.058 | 0.055 | 0.055 | 0.055 | 0.054 | 0.056 | 0.056 | 0.057 | 0.055 | 0.056 | 0.055 |

**Table 17. EC₅₀ values for the binding of different antibodies to antigens**

| Antigen | Antibody | EC₅₀ (ng/mL) | R² |
|---|---|---|---|
| | 240B10-1 | 47.73 | 0.9968 |
| | 98G10-1 (98G10) | 70.97 | 0.9992 |
| hNMI | B8-1 (B-8) | 44.44 | 0.9929 |
| | 94C4-1 (94C4) | 32.72 | 0.9995 |
| | 39B4-1 (39B4) | 53.53 | 0.9963 |

**Table 17-1. Effect of Mutations at Critical Sites of Monoclonal Antibody 94C4 on Its Function**

| CDR (the definition scheme is Abm) | Seq | Mutation site | Affinity EC₅₀ (µg/mL) | Affinity | Fold decrease in affinity |
|---|---|---|---|---|---|
| CDR-H1 | GFDFSSNAMC (SEQ ID NO: 16) | A32K | >500 | ↓ (decrease) | >500 |
| | | S29D | >500 | ↓ | >500 |
| CDR-H2 | CISSASSGSSGSTY (SEQ ID NO: 17) | S52K | >500 | ↓ | >500 |
| | DGYDL (SEQ ID NO: 7) | D101E | >500 | ↓ | >500 |
| | | D101K | >500 | ↓ | >500 |
| CDR-H3 | | D101M | >500 | ↓ | >500 |
| | | D101Q | >500 | ↓ | >500 |
| | | D101S | >500 | ↓ | >500 |
| CDR-L3 | AGGYDSASDTYV (SEQ ID NO: 24) | D97K | >500 | ↓ | >500 |

**Table 17-2. Effect of Mutations at Critical Sites of Monoclonal Antibody B-8 on Its Function**

| CDR (the definition scheme is Abm) | Seq | Mutation site | Affinity EC₅₀ (µg/mL) | Affinity | Fold decrease in affinity |
|---|---|---|---|---|---|
| | EYSYDDYGDFWGFN L (SEQ ID NO: 35) | S101K | >500 | ↓ | >500 |
| CDR-H3 | | S101Y | >500 | ↓ | >500 |
| | | G106K | >5 | ↓ | >100 |
| CDR-L1 | QASQNIYTNLA (SEQ ID NO: 50) | T31V | >5 | ↓ | >100 |

**Table 17-3. Effect of Mutations in the Critical Site of Monoclonal Antibody 240B 10-1 on Its Function**

| CDR (the definition scheme is Abm) | Seq | Mutation site | Affinity EC₅₀ (µg/mL) | Affinity | Fold decrease in affinity |
|---|---|---|---|---|---|
| | GIDLSSYTMG (SEQ ID NO: 72) | D27K | >500 | ↓ | >500 |
| | | D27P | >500 | ↓ | >500 |
| | | S29D | >500 | ↓ | >500 |
| | | S29E | >500 | ↓ | >500 |
| CDR-H1 | | S29Q | >500 | ↓ | >500 |
| | | S30A | >500 | ↓ | >500 |
| | | S30D | >500 | ↓ | >500 |
| | | S30E | >500 | ↓ | >500 |
| | | S30K | >500 | ↓ | >500 |
| | IISGGGRTY (SEQ ID NO: 73) | G52E | >500 | ↓ | >500 |
| CDR-H2 | | G52K | >500 | ↓ | >500 |
| | | G52Q | >500 | ↓ | >500 |
| | | G52R | >500 | ↓ | >500 |
| CDR-L1 | QSSQSVYNNDYLA (SEQ ID NO: 78) | N30E | >500 | ↓ | >500 |
| | | N30T | >500 | ↓ | >500 |

**Table 17-4. Effect of Mutations in the Critical Site of Monoclonal Antibody 98G10 on Its Function**

| CDR (the definitio n scheme is Abm) | Seq | Mutation site | Affinity EC₅₀ (µg/mL) | Affinity | Fold decrease in affinity |
|---|---|---|---|---|---|
| | | S57R | >500 | ↓ | >500 |
| | | S57Q | >500 | ↓ | >500 |
| | CIYGGSSGSAY (SEQ ID NO: 101) | S57K | >500 | ↓ | >500 |
| CDR-H2 | | S56P | >500 | ↓ | >500 |
| | | S56N | >500 | ↓ | >500 |
| | | G58V | >7 | ↓ | >100 |
| | | G55W | >7 | ↓ | >100 |
| | | G55H | >7 | ↓ | >100 |

**Table 17-5. Effect of Mutations in the Critical Site of Monoclonal Antibody 39B4 on Its Function**

| CDR (the definition scheme is Abm) | Seq | Mutation site | Affinity EC₅₀ (µg/mL) | Affinity | Fold decrease in affinity |
|---|---|---|---|---|---|
| | | Y102F | >500 | ↓ | >500 |
| | | G106R | >500 | ↓ | >500 |
| | AGYYTYGAGVYIYPSSFKL (SEQ ID NO: 128) | G106Y | >500 | ↓ | >500 |
| CDR-H3 | | A107Q | >500 | ↓ | >500 |
| | | A107R | >500 | ↓ | >500 |
| | | V109K | >500 | ↓ | >500 |
| | | V109Q | >500 | ↓ | >500 |

**Table 17-6. Effect of Mutations Other Than Those at the Critical Sites (CDR Region) of Monoclonal Antibody 98G10 on its Function**

| Mutated Antibody | Mutation site | | Binding to hNMI EC₅₀ (µg/mL) | Fold decrease in affinity |
|---|---|---|---|---|
| | Heavy chain (H) | Light chain (L) | | |
| 98G10 | - | - | 0.031 | - |
| 98G10-H-1 | W34H | - | 0.024 | 0.77 |
| 98G10-H-2 | W34Y | - | 0.507 | 16.35 |
| 98G10-H-3 | Y112R | - | 0.028 | 0.90 |
| 98G10-H-4 | Y112S | - | 0.082 | 2.65 |
| 98G10-H-5 | Y112E | - | 1.852 | 59.74 |
| 98G10-H-6 | Y112Q | - | 0.092 | 2.97 |
| 98G10-H-7 | W34R, Y112R | - | 1.561 | 50.35 |
| 98G10-H-8 | W34D, Y112R | - | 1.591 | 51.32 |
| 98G10-H-9 | W34H, Y112L | - | 0.311 | 10.03 |
| 98G10-H-10 | W34T, Y112R | - | 1.860 | 60.00 |
| 98G10-H-11 | W34H, Y112R | - | 0.026 | 0.84 |
| 98G10-H-12 | W34H, Y112S | - | 0.081 | 2.61 |
| 98G10-H-13 | W34K, Y112S | - | 1.676 | 54.06 |
| 98G10-H-14 | W34D, Y112S | - | 1.490 | 48.06 |
| 98G10-L-1 | - | Y93R | 0.087 | 2.81 |
| 98G10-L-2 | - | Y93S | 0.053 | 1.71 |
| 98G10-L-3 | - | Y93Q | 0.066 | 2.13 |

**Table 17-7. Effect of Mutations Other Than Those at the Critical Sites (Non-CDR Region) of Monoclonal Antibody 98G10 on Its Function**

| Mutated Antibody | Mutation site | | Binding to hNMI EC₅₀ (nmol/L) | Fold decrease in affinity |
|---|---|---|---|---|
| | Heavy chain (H) | Light chain (L) | | |
| 98G10 | - | - | 0.1652 | - |
| 98G10-1 | - | C80P | 0.1734 | 1.05 |
| 98G10-2 | T81Y | - | 0.04833 | 0.29 |
| 98G10-3 | T81Y | C80P | 0.1756 | 1.06 |
| 98G10-4 | T23K | C80P | 0.1766 | 1.07 |
| 98G10-5 | - | T18R/C80P | 0.238 | 1.44 |
| 98G10-6 | T81Y | T18R/C80P | 0.1677 | 1.02 |
| 98G10-7 | T23K/T81Y | T18R/C80P | 0.133 | 0.81 |
| 98G10-8 | T23K/T75D/T81Y | T18R/C80P | 0.1059 | 0.64 |
| 98G10-9 | T23K/T81Y | T18R/Q70D/C80P | 0.1194 | 0.72 |
| 98G10-10 | T23K/T75D/T81Y | T18R/Q70D/C80P | 0.1681 | 1.02 |
| 98G10-11 | - | Q70D | 0.0659 | 0.40 |
| 98G10-12 | T75D | - | 0.0746 | 0.45 |

**Table 17-8. Effect of Mutations Other Than Those at the Critical Sites of Monoclonal Antibody B-8 on its Function**

| Mutated Antibody | Mutation site | | Binding to hNMI | Fold decrease in |
|---|---|---|---|---|
| | Heavy chain (H) | Light chain (L) | EC₅₀ (nmol/L) | affinity |
| B-8 | - | - | 0.309 | - |
| B-8-1 | - | Y93E | 0.698 | 2.26 |
| B-8-2 | - | S91R | 1.446 | 4.68 |
| B-8-3 | Y33E | - | 0.1756 | 0.57 |
| B-8-4 | Y61E | - | 0.1766 | 0.57 |
| B-8-5 | F111A | - | 0.238 | 0.77 |

The Arabic numerals in the mutation sites in Tables 17-1 to 17-8 above represent the positions of the mutation site in the heavy chain variable region or light chain variable region, the letter before the Arabic numeral indicates the amino acid at that position before the mutation, the letter after the Arabic numeral indicates the amino acid at that position after the mutation, and the other sequences remain unchanged.

### II. Western blot verified whether recombinant proteins and 293T-transfected overexpressed proteins were recognized

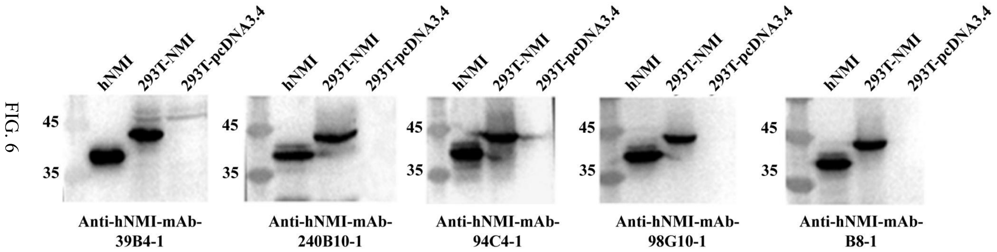

The present example provided a method for detecting the target protein by transferring proteins (recombinant protein (hNMI), lysates of 293T cells transfected with the hNMI plasmid (293T-NMI-flag, 293T-NMI), and lysates of 293T cells transfected with empty vector plasmid (293T-flag-pcDNA3.4, 293T-pcDNA3.4)) to an NC membrane after gel electrophoresis, followed by Western Blotting. The specific steps were as follows: The proteins (recombinant protein (hNMI), lysates of 293T cells transfected with the hNMI plasmid (293T-NMI-flag), and lysates of 293T cells transfected with the empty vector plasmid (293T-flag-pcDNA3.4)) were transferred to an NC membrane after polyacrylamide gel electrophoresis. The NC membrane was incubated with 5% skimmed milk at 37°C for 1 hour, washed for five times with 1× PBST solution, with 5 minutes per wash. The monoclonal antibodies of the present invention (94C4 (94C4-1), B-8 (B8-1), 240B10-1, 98G10 (98G10-1), and 39B4 (39B4-1)) were diluted to 1 µg/mL using 1% BSA solution as the primary antibody and incubated with the NC membrane at 4°C for 12 hours. The NC membrane was washed for five times with 1× PBST solution, with 5 minutes per wash. An HRP-labeled secondary antibody specific for rabbit-derived antibodies was incubated with the NC membrane at 37°C for 1 hour. The NC membrane was washed for three times with 1× PBST solution, with 5 minutes per wash. The HRP chromogenic substrate was added, developed using a gel imaging system, and the results were as shown in FIG. 6: Specific bands were observed at the positions of the recombinant protein (hNMI) and the lysates of 293T cells transfected with the NMI plasmid (293T-NMI-flag) on the membrane, while no specific band was detected at the position of the lysates of 293T cells transfected with the empty vector plasmid (293T-flag-pcDNA3.4).

### III. Pair-wise experiments of monoclonal antibody (94C4 (94C4-1), B-8 (B8-1), 240B10-1, 98G10 (98G10-1), and 39B4 (39B4-1))

The double antibody sandwich ELISA assay was employed to evaluate the efficacy of antigen detection by various antibody pairings, with detailed procedures as follows: 1) 2.5 µg/mL of monoclonal antibodies 94C4 (94C4-1), B-8 (B8-1), and 240B10-1 were used as coating antibody to coat microtiter plates, respectively (100 µL/well, coating overnight at 4°C); 2) The coating solution was discarded, and the plates were washed for 9 times using a plate washer with PBST (250 µL/well) as washing solution, residual solution was removed by tapping; 3) The plates were blocked with 5% BSA diluted with PBS (150 µL/well), and incubated at 37°C for 2 h; 4) The blocking solution was discarded, and the plates were washed for 9 times using a plate washer with PBST (250 µL/well); 5) The concentrations of the hNMI antigen were measured, and the initial concentration was set to 0.1 µg/mL, 3-fold gradient dilution was performed with a total of 6 gradients (as shown in Table 18), PBS was used as dilution solution (100 µL/well), and incubation was performed at 37°C for 1 h; 6) The antigen solution was discarded, and plates were washed for 9 times using a plate washer with PBST (250 µL/well); 7) Biotin-labeled 94C4 (94C4-1), B-8 (B8-1), 240B10-1, 98G10 (98G10-1), and 39B4 (39B4-1) were respectively added at 1 µg/mL (100 µL/well) as detection antibodies (the detection antibodies and the coating antibodies are different antibodies) and incubated at 37°C for 1 h; 8) The detection antibody solution was discarded, and plates were washed for 9 times using a plate washer with PBST (250 µL/well); 9) 100 µL/well of Streptavidin diluted at 1:5,000 in PBS was added and incubated at 37°C for 1 h; 10) The streptavidin solution was discarded, and plates were washed for 9 times using a plate washer with PBST (250 µL/well); 11) TMB chromogenic solution (90 µL/well) was added for color development, followed by incubation at 37°C for 15 min in the dark; 12) The reaction was terminated with 2 M H₂SO₄ (50 µL/well), and OD450 was measured. The results were as shown in Table 18: When 94C4 (94C4-1), B-8 (B8-1), or 240B10-1 was used as the coating antibody and the biotin-labeled B-8 (B8-1), 240B10-1, 98G10 (98G10-1), 94C4 (94C4-1), or 39B4 (39B4-1) was used as the detection antibody (the coating antibody and the detection antibody were different antibodies), better detection effect was achieved.

**Table 18. Effect of double-antibody sandwich Elisa assay for detection of each antibody pairing with antigens**

| Coating antibody 2.5µg/mL | Concentration of antigen (ng/mL) | | | | | | Detection Antibody 1µg/mL |
|---|---|---|---|---|---|---|---|
| | 100.000 | 33.333 | 11.111 | 3.704 | 1.235 | 0.000 | |
| B-8 | 3.074 | 2.428 | 1.247 | 0.563 | 0.291 | 0.125 | 39B4-1-biotin |
| | 3.252 | 2.858 | 1.917 | 0.915 | 0.419 | 0.118 | 94C4-1-biotin |
| | 3.019 | 2.484 | 1.378 | 0.607 | 0.307 | 0.126 | 98G10-1-biotin |
| | 3.048 | 2.426 | 1.212 | 0.527 | 0.272 | 0.130 | 240B10-1-biotin |
| 94C4-1 | 2.978 | 2.284 | 1.177 | 0.541 | 0.277 | 0.092 | B-8-biotin |
| | 3.222 | 2.754 | 1.670 | 0.735 | 0.372 | 0.122 | 39B4-1-biotin |
| | 3.102 | 2.512 | 1.361 | 0.627 | 0.292 | 0.109 | 98G10-1-biotin |
| | 3.231 | 2.680 | 1.669 | 0.744 | 0.394 | 0.147 | 240B10-1-biotin |
| 240B10-1 | 2.956 | 2.270 | 1.264 | 0.527 | 0.256 | 0.102 | B-8-biotin |
| | 3.119 | 2.666 | 1.705 | 0.785 | 0.350 | 0.111 | 39B4-1-biotin |
| | 3.282 | 3.027 | 2.300 | 1.277 | 0.590 | 0.162 | 94C4-1-biotin |
| | 3.139 | 2.559 | 1.696 | 0.777 | 0.362 | 0.121 | 98G10-1-biotin |

The double antibody sandwich ELISA assay was employed to evaluate the efficacy of antigen detection by various antibody pairings, the method was identical to the above procedures, with the differences only in that: 1) 2.5 µg/mL of monoclonal antibodies B-8 (B8-1), 240B10-1, 98G10 (98G10-1), and 39B4 (39B4-1) were used as coating antibodies to coat microtiter plates, respectively; 2) The concentrations of the antigen were measured, and the initial concentration was set to 50,000 pg/mL, 2-fold gradient dilution was performed with a total of 12 gradients (as shown in Table 19), PBS was used as dilution solution; 3) Biotin-labeled 94C4 (94C4-1) was used as the detection antibody at 1µg /mL and 0.5 µg/mL respectively, the results were as shown in Table 19: When biotin-labeled 94C4 (94C4-1) was used as the detection antibody at 0.5 µg/mL, and the monoclonal antibody B-8 (B8-1), 240B10-1, 98G10 (98G10-1), or 39B4 (39B4-1) was respectively used as the coating antibody at 2.5 µg/mL, the detection limits (sensitivities) were 60, 60, 60, and 40 pg/mL, respectively.

**Table 19. Detection results of using biotin-labeled 94C4 (94C4-1) as the detection antibody, and using B-8 (B8-1), 240B10-1, 98G10 (98G10-1), or 39B4 (39B4-1) as the coating antibody respectively**

| Concentration of antigen (antigen concentration, pg/mL) | Coating antibody (Concentration of coating antibody) 2.5µg/mL | | | | | | | |
|---|---|---|---|---|---|---|---|---|
| | B-8 | 39B4-1 | 98G10-1 | 240B10-1 | B-8 | 39B4-1 | 98G10-1 | 240B10-1 |
| 50,000.000 | 3.110 | 3.098 | 3.038 | 3.100 | 3.175 | 3.159 | 3.249 | 3.335 |
| 25,000.000 | 2.785 | 2.752 | 2.738 | 2.745 | 2.712 | 2.746 | 2.873 | 2.943 |
| 12,500.000 | 2.026 | 2.150 | 2.307 | 2.169 | 2.034 | 2.299 | 2.445 | 2.319 |
| 6,250.000 | 1.299 | 1.517 | 1.531 | 1.474 | 1.293 | 1.557 | 1.600 | 1.580 |
| 3,125.000 | 0.750 | 0.932 | 0.903 | 0.958 | 0.712 | 0.960 | 0.958 | 1.292 |
| 1,562.500 | 0.446 | 0.547 | 0.525 | 0.568 | 0.420 | 0.593 | 0.596 | 0.907 |
| 781.250 | 0.288 | 0.381 | 0.341 | 0.351 | 0.265 | 0.387 | 0.377 | 0.396 |
| 390.625 | 0.206 | 0.251 | 0.233 | 0.255 | 0.178 | 0.250 | 0.219 | 0.268 |
| 195.313 | 0.173 | 0.207 | 0.179 | 0.212 | 0.134 | 0.179 | 0.159 | 0.187 |
| 97.656 | 0.150 | 0.169 | 0.149 | 0.185 | 0.113 | 0.143 | 0.126 | 0.146 |
| 48.828 | 0.139 | 0.145 | 0.134 | 0.167 | 0.102 | 0.130 | 0.108 | 0.125 |
| 0.000 | 0.130 | 0.139 | 0.128 | 0.165 | 0.090 | 0.102 | 0.093 | 0.109 |
| Detection antibody | 94C4-1-biotin: 1µg/mL | | | | 94C4-1-biotin: 0.5µg/mL | | | |

### IV. Specificity of paired antibody detected by double-antibody sandwich ELISA assay

Preparation of Protein Samples-Overexpressing Cell: NMI-flag referred to 293T cells transfected with the hNMI-flag plasmid (NMI-flag), flag referred to 293T cells transfected with the flag tag plasmid (empty vector, flag), and 293T referred to synchronously cultured 293T cells (synchronization control, 293T). The screened paired antibodies (B-8 (B8-1) was used as the coating antibody and 0.5 µg/mL of 94C4-1-biotin was used as the detection antibody) were employed in a double-antibody sandwich ELISA assay to detect intracellularly overexpressed protein, with detailed procedures as follows: 1) The coating antibody was diluted to 2.5 µg/mL with PBS (100 µL/well) for coating overnight at 4°C; (2) The coating solution was discarded, and plates were washed for 9 times using a plate washer with PBST (250 µL/well) as washing solution, residual solution was removed by tapping; (3) The plates were blocked with 5% BSA diluted with PBS (150 µL/well), and incubated at 37°C for 2 h; (4) The blocking solution was discarded, and the plates were washed for 9 times using a plate washer with PBST (250 µL/well); 5) The antigen solution (cell supernatants or lysates (100 µL/well)) was added and incubated at 37°C for 1 h; 6) The antigen solution was discarded, and the plates were washed for 9 times using a plate washer with PBST (250 µL/well); 7) Biotin-labeled 94C4 (94C4-1) was added at 0.5 µg/mL (100 µL/well) as detection antibody and incubated at 37°C for 1 h; 8) The detection antibody solution was discarded, and the plates were washed for 9 times using a plate washer with PBST (250 µL/well); (9) 100 µL/well of Streptavidin diluted at 1:5,000 in PBS was added and incubated at 37°C for 1 h; (10) The streptavidin solution was discarded, and the plates were washed for 9 times using a plate washer with PBST (250 µL/well). (11) TMB chromogenic solution (90 µL/well) was added for color development, followed by incubation at 37°C for 15 min in the dark; (12) The reaction was terminated with 2 M H₂SO₄ (50 µL/well), and OD450 was measured. The results were as shown in FIG. 4 and Table 20: The NMI-flag overexpressed protein (NMI-flag) could be specifically identified in both supernatants and lysates of 293T cells. The OD₄₅₀ values detected in supernatants of empty vector and synchronization control of 293T cells were significantly below the detection limit. Additionally, the detection values in cell lysates of empty vector were higher than those in control supernatants, which was consistent with the phenomenon that NMI released from damaged or dead cells caused by some transfection during transfection process resulted in the increased detection values.

**Table 20. Graph of specificity results of paired antibodies detected by double-antibody sandwich Elisa assay**

| | NMI-flag | | flag | | 293T | |
|---|---|---|---|---|---|---|
| | 1 | 2 | 1 | 2 | 1 | 2 |
| cell supernatant | 0.897 | 0.95 | 0.107 | 0.101 | 0.096 | 0.093 |
| cell lysates | 3.028 | 2.903 | 0.349 | 0.354 | 0.184 | 0.181 |

### V. Natural protein testing of human body fluids

The double-antibody sandwich ELISA assay was employed to evaluate the efficacy of antibody pairings in detecting natural proteins in human body fluids, in which the method was the same as that described in Method III, with the differences only in that: 1) microtiter plates were coated with 2.5µg/mL of B-8 (B8-1) monoclonal antibody as the coating antibody; 5) The assay was performed with antigen derived from joint fluid sample of one arthritic patient(containing stock solution, 2-fold dilution of the stock solution (the dilution solution was PBS, 1/2), and PBS as a negative control (0)); (7) 0.5 µg/mL of biotin-labeled 94C4 (94C4-1) was used as the detection antibody, the results were as shown in Table 21: NMI in the joint fluid could be detected by using biotin-labeled 94C4 (94C4-1) as the detection antibody and B-8 (B8-1) as the coating antibody, i.e., the natural protein could be recognized.

**Table 21. Detection results of using biotin-labeled 94C4 (94C4-1) as the detection antibody and B-8 (B8-1) as the coating antibody**

| Concentration (pg/mL) | Coating antibody (2.5µg/mL) B8-1 |
|---|---|
| stock solution | 0.221 |
| 1/2 | 0.128 |
| 0 | 0.076 |
| Detection antibody | 94C4-1-biotin: 0.5µg/mL |

### Example 3. Optimization of a double-antibody sandwich enzyme-linked immunosorbent assay (ELISA) kit for the detection of NMI

Optimization of the blocking solution:
1) A double-antibody sandwich enzyme-linked immunosorbent assay (ELISA) kit for the detection of NMI, comprising: coating antibody B-8, polystyrene plastic plate (Corning Coster), dilution solution of the coating antibody (0.05 mol/L of phosphate buffer, pH 7.5), blocking solution (PBS solution containing 5 (w/v) % BSA (pH 7.5, 0.05 M)), washing solution (PBS solution containing 0.05% Tween-20 (pH 7.5, 0.05 M)), sample diluent (PBS solution containing 5% (w/v) BSA and 0.1% (v/v) Tween-20 (pH 7.5, 0.05 M)), biotin-labeled detection antibody 94C4, Streptavidin-Horseradish peroxidase conjugate (SA-HRP), 3,3',5,5'-tetramethylbenzidine (TMB) chromogenic solution and stop solution (2M sulfuric acid solution).
2) A double-antibody sandwich enzyme-linked immunosorbent assay (ELISA) kit for the detection of NMI, which was the same as 1), with differences only in that the blocking solution was 5 wt% skimmed milk powder.
3) A double-antibody sandwich enzyme-linked immunosorbent assay (ELISA) kit for the detection of NMI, which was the same as 1), with differences only in that the blocking solution was a PBS solution containing 5 (w/v) % FBS (pH 7.5, 0.05 M).

The kits 1), 2) and 3) were used to detect NMI as follows:
The coating antibody B-8 was diluted to 0.25 µg/mL with the dilution solution of the coating antibody (0.05 mol/L phosphate buffer, pH 7.5), the polystyrene plastic plate (Corning Coster) was coated with 100 µL per well at 4°C overnight.

The next day, the coating solution was aspirated, and then blocking was performed with the blocking solution in the kits 1), 2) and 3) respectively at 37°C for 2 hours.

After blocking, the plate was washed twice with the washing solution (PBS solution containing 0.05% Tween-20 (pH 7.5, 0.05 M)), detection antigen (hNMI) was added, the antigen was initiated at 25 ng/mL, and 2-fold serial dilution was performed by using the sample diluent (as shown in Table 22) with 100 µL per well.

The antigen was incubated for reaction at 37°C for 1 h. The plate was washed for four times with the washing solution, and 100 µL of 0.25 µg/mL biotin-labeled detection antibody 94C4 was added to each well.

The detection antibody was incubated for reaction at 37°C for 1 h. The plate was washed for four times with the washing solution, and 100 µL of the streptavidin-horseradish peroxidase conjugate (SA-HRP) at a concentration of 50 ng/mL was added to each well.

The SA-HRP was incubated for reaction at 37°C for 15 min, the plate was washed for four times with the washing solution, and the 3,3',5,5'-tetramethylbenzidine (TMB) chromogenic solution was added at 100 µL/well.

The chromogenic solution was incubated for reaction at 37°C for 15 min, the reaction was terminated by adding 50 µL/well of the stop solution (2M sulfuric acid solution), and the plate was read at 450 nm with a microplate reader.

The results were as shown in Table 22: the test results of different kits (blocking solution) were basically the same, and based on the OD value of the blank well, PBS solution containing 5 (w/v)% BSA (pH 7.5, 0.05 M) was selected as the blocking solution, i.e., the kit 1) was selected for the subsequent experiments.

**Table 22. Detection effects of different blocking solutions**

| Antigen concentration, pg/mL | OD450 | | |
|---|---|---|---|
| 25000 | 2.142 | 2.021 | 2.01 |
| 12500 | 1.279 | 1.105 | 1.115 |
| 6250 | 0.748 | 0.586 | 0.637 |
| 3125 | 0.433 | 0.36 | 0.391 |
| 1562.5 | 0.284 | 0.223 | 0.235 |
| 781.25 | 0.206 | 0.171 | 0.174 |
| 390.625 | 0.147 | 0.148 | 0.131 |
| 0.00 | 0.084 | 0.129 | 0.083 |
| Blocking solution | BSA | skimmed milk powder | FBS |

### Example 4. Optimization of the using method of the double-antibody sandwich enzyme-linked immunosorbent assay (ELISA) kit for the detection of NMI

### 1) Optimization of the working concentration of the coating antibody

The kit 1) in Example 3 was used for the optimization of the working concentration of the coating antibody, the method was performed in the same way as that in Example 3 for the detection of NMI, with the differences only in that: the coating antibody B-8 was diluted with the dilution solution of the coating antibody (0.05 mol/L phosphate buffer, pH 7.5) to 0.25, 0.5, 1, 2.5, and 5 µg/mL, respectively, the polystyrene plastic plate (Corning Coster) was coated with 100 µL per well at 4°C overnight.

The results were as shown in Table 23: The concentration of the coating antibody was optimal at 0.25 µg/mL, with high overall signal values and low background values.

**Table 23. Detection effects of different concentrations of coating antibody**

| Antigen concentration, pg/mL | OD450 | | | | |
|---|---|---|---|---|---|
| 25000 | 2.691 | 2.714 | 2.724 | 2.173 | 2.193 |
| 12500 | 2.125 | 2.212 | 2.157 | 1.62 | 1.738 |
| 6250 | 1.446 | 1.529 | 1.406 | 1.176 | 1.127 |
| 3125 | 0.821 | 0.882 | 0.858 | 0.726 | 0.733 |
| 1562.5 | 0.495 | 0.506 | 0.514 | 0.443 | 0.474 |
| 781.25 | 0.36 | 0.344 | 0.34 | 0.328 | 0.303 |
| 390.625 | 0.202 | 0.284 | 0.225 | 0.208 | 0.225 |
| 0.00 | 0.089 | 0.102 | 0.118 | 0.131 | 0.132 |
| Coating concentration, µg/mL | 0.25 | 0.5 | 1 | 2.5 | 5 |

### 2) Optimization of the concentration of the detection antibody

The kit 1) in Example 3 was used for the optimization of the working concentration of the detection antibody, the method was performed in the same way as that in Example 3 for detecting NMI, with the differences only in that: the initial concentration of the antigen was 12.5 ng/mL; the antigen was incubated for reaction at 37°C for 1 h, the plate was washed for four times with the washing solution, and 100 µL of biotin-labeled detection antibody 94C4 at concentrations of 0.25 µg/mL, 0.5 µg/mL, and 1.0 µg/mL was added to each well, respectively.

The results were as shown in Table 24: by comparing different concentrations of the detection antibody, a relatively good linear relationship could be achieved with 0.25 µg/mL.

**Table 24. Detection effects of different concentrations of detection antibody**

| Antigen concentration, pg/mL | OD450 | | |
|---|---|---|---|
| 12500 | 2.148 | 2.516 | 2.686 |
| 6250 | 1.299 | 1.389 | 1.446 |
| 3125 | 0.73 | 0.824 | 0.828 |
| 1562.5 | 0.438 | 0.482 | 0.479 |
| 781.25 | 0.276 | 0.275 | 0.337 |
| 390.625 | 0.176 | 0.198 | 0.24 |
| 195.313 | 0.128 | 0.148 | 0.19 |
| 0.00 | 0.083 | 0.083 | 0.135 |
| Concentration of detection antibody, µg/mL | 0.25 | 0.5 | 1 |

### 3) Optimization of detection concentration of SA-HRP

The kit 1) in Example 3 was used for the optimization of the working concentration of the coating antibody, the method was performed in the same way as that in Example 3 for detecting NMI, with the differences only in that: the initial concentration of the antigen was 12.5 ng/mL; the detection antibody was incubated for reaction at 37°C for 1 h, the plate was washed for four times with the washing solution, and 100 µL of the streptavidin-horseradish peroxidase conjugate (SA-HRP) at concentrations of 50 µg/mL, 100 µg/mL, and 200 ng/mL was added to each well, respectively.

The results were as shown in Table 25: by comparing different SA-HRP concentrations, the SA-HRP concentration of 50 ng/mL was the most optimal in terms of detection signal.

**Table 25. Detection effects of different concentrations of detection antibody**

| Antigen concentration, pg/mL | OD450 | | |
|---|---|---|---|
| 12500 | 2.312 | 2.349 | 2.644 |
| 6250 | 1.283 | 1.295 | 1.446 |
| 3125 | 0.666 | 0.707 | 0.812 |
| 1562.5 | 0.367 | 0.389 | 0.45 |
| 781.25 | 0.232 | 0.235 | 0.283 |
| 390.625 | 0.154 | 0.161 | 0.192 |
| 195.313 | 0.121 | 0.112 | 0.131 |
| 0.00 | 0.075 | 0.082 | 0.102 |
| SA-HRP, ng/mL | 50 | 100 | 200 |

### 4) Optimization of reaction time for the antigen

The kit 1) in Example 3 was used for the optimization of the working concentration of the coating antibody, the method was performed in the same way as that in Example 3 for detecting NMI, with the differences only in that: the antigen was incubated for reaction at 37°C for 45 minutes, 1 hour, and 1.5 hours, respectively, the plate was washed for four times using the washing solution, and 100 µL of biotin-labeled detection antibody 94C4 at a concentration of 0.25 µg/mL was added to each well.

The results were as shown in Table 26: by comparing different reaction times for the antigen, 1h was optimal by comprehensive analysis.

**Table 26. Detection effects of detection antibody with different reaction times for the antigen**

| Antigen concentration, pg/mL | OD450 | | |
|---|---|---|---|
| 12500 | 1.901 | 2.196 | 2.431 |
| 6250 | 0.958 | 1.141 | 1.219 |
| 3125 | 0.506 | 0.598 | 0.614 |
| 1562.5 | 0.281 | 0.33 | 0.348 |
| 781.25 | 0.188 | 0.197 | 0.209 |
| 390.625 | 0.126 | 0.143 | 0.135 |
| 195.313 | 0.097 | 0.103 | 0.102 |
| 0.00 | 0.072 | 0.078 | 0.069 |
| Reaction time for the antigen | 45min | 1h | 1.5h |

### Example 5. Linear range of the double-antibody sandwich enzyme-linked immunosorbent assay (ELISA) kit for the detection of NMI

The kit 1) in Example 3 was used for the preparation of a standard curve, the method was performed in the same way as that in Example 3 for detecting NMI, with the differences only in that: the antigen was initiated at 12 ng/mL and a 2-fold serial dilution was performed by using the sample diluent (as shown in Table 27).

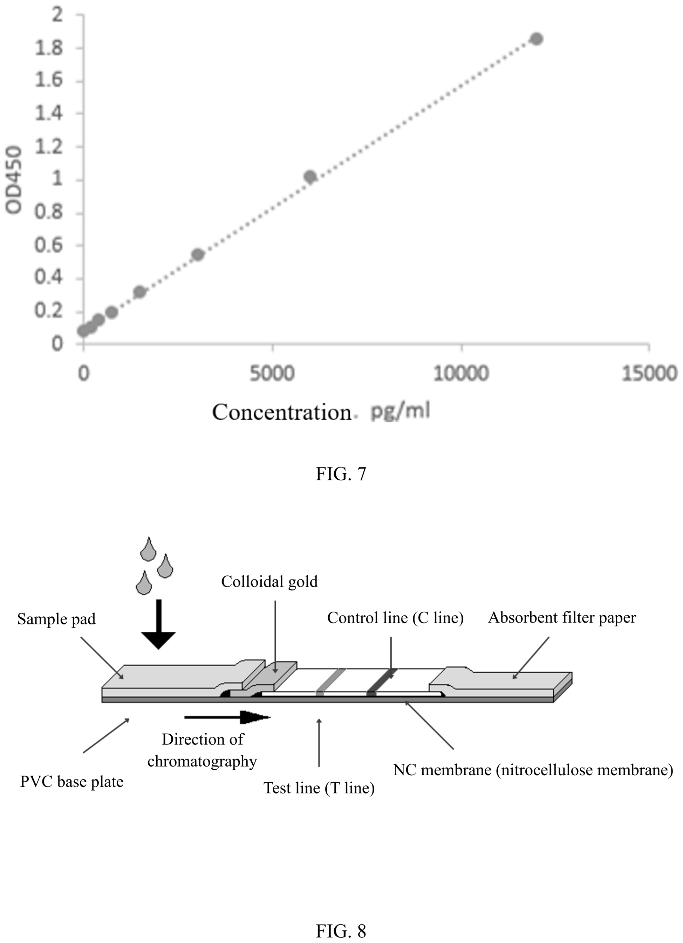

The results were as shown in Table 27 and FIG. 7: the fitted curve was y = 0.0001x + 0.0914 (R² = 0.9992), with good linearity and a linear range between 187.5 pg/mL and 12000 pg/mL.

**Table 27. Results of detection of different concentrations of antigen using the kit 1) in Example 3**

| Antigen concentration, pg/mL | OD450 | | Mean value |
|---|---|---|---|
| 12000 | 1.915 | 1.802 | 1.859 |
| 6000 | 1.058 | 0.982 | 1.02 |
| 3000 | 0.588 | 0.507 | 0.548 |
| 1500 | 0.318 | 0.317 | 0.318 |
| 750 | 0.183 | 0.201 | 0.192 |
| 375 | 0.149 | 0.148 | 0.149 |
| 187.5 | 0.107 | 0.111 | 0.109 |
| 0.00 | 0.082 | 0.085 | 0.084 |

### Example 6. Detection of serum NMI levels in healthy individuals and septic patients

The kit 1) in Example 3 was used to detect the samples collected from healthy individuals and septic patients, and the NMI protein content was calculated from the equation fitted to the standard curve of Example 5 by reading the OD value by microplate reader.

The difference in serum NMI (antigen 1) protein content between healthy individuals and septic patients was compared by using Graphpad prism 8 plotting, and by using Mann-Whitney U test analysis. The serum antigen 1 content of the septic patients was significantly higher than that of the healthy group (P<0.005). Therefore, the kit prepared according to the present invention could be used for rapid detection of serum antigen 1 content.

Meanwhile, ROC curve was plotted and analyzed for the diagnostic value of serum antigen 1 in sepsis, and the serum antigen 1 could well diagnose sepsis (with a high AUC value).

### Example 7. Analytical sensitivity of the double-antibody sandwich enzyme-linked immunosorbent assay (ELISA) kit for the detection of NMI

Twenty negative samples were tested using the kit 1) in Example 3 from the same batch, and the results were as shown in Table 27. The mean value (X) and standard deviation (SD) of the negative wells were calculated, and the critical value was determined by the formula (Critical Value = X + 2SD). The calculated critical value was substituted into the standard curve in Example 5, and the calculated concentration was the analytical sensitivity of the assay kit, the critical value of this kit was seen to be 0.1207 and the analytical sensitivity was 196.864 pg/mL.

**Table 27. OD450 assay values of 20 negative samples**

| | | | | | | | | | | |
|---|---|---|---|---|---|---|---|---|---|---|
| Number | 1 | 2 | 3 | 4 | 5 | 6 | 7 | 8 | 9 | 10 |
| OD450 | 0.117 | 0.116 | 0.115 | 0.123 | 0.115 | 0.112 | 0.117 | 0.113 | 0.115 | 0.12 |
| Number | 11 | 12 | 13 | 14 | 15 | 16 | 17 | 18 | 19 | 20 |
| OD450 | 0.113 | 0.117 | 0.115 | 0.114 | 0.11 | 0.114 | 0.111 | 0.114 | 0.113 | 0.115 |

### Example 8. A magnetic-bead chemiluminescent assay kit for the detection of NMI

### Example 8-1

A magnetic-bead chemiluminescent assay kit for the detection of NMI, comprising: 98G10-coated magnetic beads, acridinium ester labeled B-8, reaction buffer and hNMI calibrator;

Wherein, the 98G10-coated magnetic beads were prepared as follows:
1) Magnetic microsphere (magnetic bead) activation: 20mg of carboxyl-modified magnetic microspheres of about 1µm in diameter were pipetted into a 2mL centrifuge tube, the magnetic microspheres were magnetically separated from the liquid with a magnetic stand, and the magnetic microspheres were washed for four times with 1ml of 0.1M pH6.0 sodium 2-(N-morpholino)ethanesulfonate (MES);
   1 mL of 0.1 M pH 6.0 MES was added to the above washed magnetic microspheres, 10 µL of 20 mg/mL of 1-(3-dimethylaminopropyl)-3-ethylcarbodiimide and 50 µL of 20 mg/ml of N-hydroxy succinimide were added, and the mixture was vibrated at room temperature for 1 hour for reaction;
2) Coupling: the above activated magnetic microspheres were placed on a magnetic stand to magnetically separate the magnetic microspheres from the liquid, the magnetic microspheres were washed for 4 times with 1mL of 0.1M pH6.0 sodium 2-(N-morpholino)ethanesulfonate (MES), 0.5mg of the monoclonal antibody 98G10 was added, and 0.1M pH6.0 sodium 2-(N-morpholino)ethanesulfonate (MES) was replenished to make a volume of the magnetic microsphere suspension containing antibody to 1mL; the magnetic microsphere suspension was incubated for 15 hours with continuous rotation at room temperature;
3) Blocking: 1.5% (v/v) ethanolamine was added to the above magnetic microsphere suspension containing antibody and incubated for 30 minutes with continuous rotation at room temperature;

The above magnetic microsphere suspension containing antibody was placed on a magnetic stand for magnetic separation, washed for four times with 1 ml of 0.1 M pH 6.0 sodium 2-(N-morpholino)ethanesulfonate (MES), added with 1 mL of 0.05 M Tris-HCl buffer containing 2% (w/v) BSA and 1% (w/v) alginate, pH 7.5, and incubated with continuous rotation for 2 hours at room temperature;

The above magnetic microsphere suspension containing antibody was placed on a magnetic stand for magnetic separation, washed for four times with 1 mL of 0.05 M Tris-HCl buffer containing 2% (w/v) BSA and 1% (w/v) alginate, pH 7.5, and added with 1 mL of 0.05 M Tris-HCl buffer containing 2% (w/v) BSA and 1% (w/v) alginate, pH 7.5, to obtain magnetic microspheres coated with monoclonal antibody 98G10;

The above magnetic microspheres coated with monoclonal antibody 98G10 was diluted at 1:100 with 0.05 M Tris-HCl buffer containing 2% (w/v) BSA and 1% (w/v) alginate, pH 7.5, dispensed and stored in a medical refrigerator at 2-8°C;

The hNMI calibrator was a lyophilized powder, preparation method was as follows: recombinant human hNMI protein was diluted to 0.00 pg/mL, 200.00pg/mL, and 2000.00pg/mL with 0.05 M Tris-HCl buffer containing 5% (w/v) BSA, 1% (w/v) alginate, 0.1% (v/v) Tween-20, and 0.1% (v/v) Proclin300, pH 7.8, dispensed in 1mL/vial and lyophilized;

Acridinium ester-labeled B-8 was prepared as follows:
1) Washing: 1.0 mg of monoclonal antibody B-8 was taken, added to a 50KD ultrafiltration centrifuge tube, and concentrated at 4°C, 8000 rmp for 5 minutes using a high-speed freezing centrifuge;
   the waste solution was discarded and PB buffer containing 0.02 M disodium hydrogen phosphate and 0.002 M potassium dihydrogen phosphate, pH 6.5, was added to the ultrafiltration centrifuge tube to make the volume in the ultrafiltration centrifuge tube to approximately 400 µL;
   centrifugated at 4°C, 8000 rpm for 5 min using a high-speed freezing centrifuge, and the waste solution was discarded, the operations were repeated for four times;
   the ultrafiltration centrifuge tube was inverted and centrifuged with a high-speed freezing centrifuge at 4°C, 3000 rmp for 1 min, then 100 µL of PB buffer was added to the ultrafiltration tube and stood for 5 min, then the ultrafiltration centrifuge tube was inverted again and centrifuged with a high-speed freezing centrifuge at 4°C, 3000 rmp for 1 min, and the antibody from the two inverted centrifugations were collected and combined;
2) Coupling: 0.05mg acridinium salt was added to the above antibody and incubated in a thermostat at 26°C for 19±1 hours with shaking in the dark;
3) Purification: acridinium ester-labeled monoclonal antibody B-8 was purified using a SepHadex^{™}G50 gel column paired with a high performance protein purification system, and the eluent was the above mentioned PB buffer at pH 6.5; the cleanliness of the gel column was checked by the UV detection function of the purification system, and the purification was started after each baseline being stable, and the solution obtained by collecting the first protein peak segment was the target antibody solution;
4) Storage: the acridinium ester-labeled monoclonal antibody B-8 collected from the above purification was filtered through a 0.22 µm needle filter, and the protein concentration was determined by Nanodrop, and finally 1% of the total volume of protein stabilizer containing 10% (v/v) glycerol and 0.1% (v/v) Procline300 was added;

The above acridinium salt-labeled monoclonal antibody B-8 was diluted at 1:400 with 0.05M Tris-HCl buffer containing 1% (w/v) alginate, 0.1% (v/v) Tween-20, and 1 mM disodium ethylenediaminetetraacetate, pH 7.2, dispensed and stored in a medical refrigerator at 2°C to 8°C;

The reaction buffer was prepared by formulating 0.025 M Tris-HCl buffer containing 0.1% (v/v) Tween-20 and 0.1% (v/v) Proclin300, pH 7.2.

### Example 8-2

A magnetic-bead chemiluminescent assay kit for the detection of NMI, the same as that of Example 8-1, with differences only in that 1 mg of monoclonal antibody 98G10 was added during the coupling process in the preparation method of the 98G10-coated magnetic beads.

### Example 8-3

A magnetic-bead chemiluminescent assay kit for the detection of NMI, the same as that of Example 8-1, with differences only in that 2.0 mg of monoclonal antibody B-8 was taken during the washing process in the preparation method of the acridinium ester-labeled B-8.

### 1) Optimization of the amount of 98G10 in the preparation method of 98G10-coated magnetic beads

Different concentrations of hNMI were detected with the kits of Examples 8-1 and 8-2, respectively, and the results were as shown in Table 28: In the preparation method of 98G10-coated magnetic beads, when the amount of 98G10 was 0.5 mg (Example 8-1), the background interference was lower.

**Table 28. Results of detection of different concentrations of hNMI with the kits of Examples 8-1 and 8-2**

| Antigen concentration, pg/ml | Signal value | |
|---|---|---|
| 2000 | 204316 | 396838 |
| 1000 | 97595 | 200773 |
| 400 | 39897 | 86784 |
| 100 | 9492 | 20441 |
| 50 | 4561 | 11078 |
| 0.00 | 246 | 2993 |
| Antibody amount | 0.5 mg (Example 8-1) | 1.0 mg (Example 8-2) |

### 2) Optimization of the amount of B-8 in the preparation method of acridinium ester-labeled B-8

Different concentrations of hNMI were detected with the kits of Examples 8-1 and 8-3, respectively, in the same way as in 1), and the results were as shown in Table 29: In the preparation method of acridinium ester-labeled B-8, when the amount of B-8 was 1.0 mg (Example 8-1), the background interference was lower.

**Table 29. Results of detection of different concentrations of hNMI with the kits of Examples 8-1 and 8-3**

| Antigen concentration, pg/mL | Signal value | |
|---|---|---|
| 2000 | 204316 | 233545 |
| 1000 | 97595 | 115914 |
| 400 | 39897 | 44554 |
| 100 | 9492 | 11858 |
| 50 | 4561 | 6219 |
| 0.00 | 246 | 1089 |
| Antibody amount | 1.0 mg (Example 8-1) | 2.0 mg (Example 8-3) |

### 3) Determination of linear range

The antigen hNMI in different concentration ranges was detected with the kit of Example 8-1, in the same way as in 1), and the results were as shown in Table 30: After comprehensive analysis, the final linear range interval was selected to be from 50 pg/mL to 10,000 pg/mL, where the concentration points contained 0, 50, 200, 1,000, 4,000, and 10,000 pg/mL.

**Table 30. Results of detection of different concentration ranges of antigen hNMI using the kit 1 of Example 8-1**

| Antigen concentration, pg/mL | Signal value | Antigen concentration, pg/mL | Signal value | Antigen concentration, pg/mL | Signal value |
|---|---|---|---|---|---|
| 16000 | 1205693 | 10000 | 1193323 | | |
| 14000 | 1053074 | 7500 | 900134 | | |
| 12000 | 893693 | 5000 | 559556 | | |
| 8000 | 587482 | 3600 | 407916 | 10000 | 1475285 |
| 6000 | 414367 | 1800 | 181193 | 4000 | 572451 |
| 4000 | 258160 | 900 | 98456 | 1000 | 131486 |
| 1000 | 57648 | 150 | 15548 | 200 | 25727 |
| 50 | 2006 | 50 | 6177 | 50 | 6121 |
| 0.00 | 180 | 0.00 | 227 | 0.00 | 267 |

### 4) Kit Repeatability Verification

Repeatability verification was carried out using the kit of Example 8-1 for detecting different concentrations of antigen hNMI in the same way as in 1), and the results were as shown in Table 31: the CV% of detecting different concentrations of antigen hNMI using the kit of Example 8-1 were all ≤5%.

**Table 31. Results of detection of different concentrations of antigen hNMI using the kit of Example 8-1**

| Concentration pg/mL | Number of measurements | Detection concentration pg/mL | CV% |
|---|---|---|---|
| 140 | 1 | 136.76 | 2.82% |
| | 2 | 138.92 | |
| | 3 | 150.30 | |
| | 4 | 137.85 | |
| | 5 | 142.36 | |
| | 6 | 136.98 | |
| | 7 | 138.42 | |
| | 8 | 135.04 | |
| | 9 | 138.42 | |
| | 10 | 145.50 | |
| 700 | 1 | 722.80 | 3.34% |
| | 2 | 719.24 | |
| | 3 | 722.91 | |
| | 4 | 738.63 | |
| | 5 | 750.42 | |
| | 6 | 692.86 | |
| | 7 | 741.03 | |
| | 8 | 701.79 | |
| | 9 | 708.54 | |
| | 10 | 692.03 | |
| 3000 | 1 | 3053.23 | 2.62% |
| | 2 | 2978.65 | |
| | 3 | 2877.11 | |
| | 4 | 3018.27 | |
| | 5 | 2938.86 | |
| | 6 | 3136.08 | |
| | 7 | 3037.74 | |
| | 8 | 2927.27 | |
| | 9 | 2960.50 | |
| | 10 | 2908.75 | |

### 5) Accuracy verification

The same low-value sample was taken, divided into 3 equal volumes, follow the requirement that the added volume was less than 10% of the original volume, and added with the standard solution to each low-value sample (10 microliters of 2000 pg/mL standard solution was used for Sample 1 to be recovered for analysis; 20 microliters of 20000 pg/mL standard solution was used for Sample 2 to be recovered for analysis; 40 microliters of 20000 pg/mL standard solution was used for Sample 3 to be recovered for analysis), prepared into three different concentrations of samples to be recovered for analysis, and the recovery rate was calculated by taking the mean value of the repeated tests using the kit of Example 8-1, the results of which were as shown in Table 32: the recovery rates were within 85%~115%.

**Table 32. Results of samples to be recovered for analysis detected using the kit of Example 8-1**

| Sample Number | Detection concentration pg/ml | Mean concentration pg/ml | Recovery rate R |
|---|---|---|---|
| Sample 1 to be recovered for analysis | 101.72 | 98.09 | 93.11% |
| | 105.07 | | |
| | 87.48 | | |
| Sample 2 to be recovered for analysis | 1101.9 | 1064.44 | 92.28% |
| | 1040.62 | | |
| | 1050.8 | | |
| Sample 3 to be recovered for analysis | 2088.54 | 2008.78 | 91.67% |
| | 2004.39 | | |
| | 1933.42 | | |

### 6) Blank limit and analytical sensitivity

25 Health Check-up serum samples were detected with the kit of Example 8-1 in the same way as in 1), the mean value (X) and standard deviation (SD) of each concentration were calculated, and the critical value was determined by the formula (LOD=X+2SD). The results were as shown in Table 33: the analytical sensitivity was calculated to be 16.64 pg/mL.

**Table 33. Results of detecting 25 health check-up serum samples with the kit of Example 8-1**

| Health check-up | Concentration pg/mL |
|---|---|
| 1 | 1.73 |
| 2 | 2.37 |
| 3 | 4.07 |
| 4 | 0.90 |
| 5 | 0.21 |
| 6 | 2.26 |
| 7 | 5.15 |
| 8 | 1.59 |
| 9 | 5.72 |
| 10 | 0.53 |
| 11 | 3.77 |
| 12 | 8.58 |
| 13 | 17.74 |
| 14 | 12.54 |
| 15 | 14.74 |
| 16 | 5.08 |
| 17 | 11.90 |
| 18 | 16.39 |
| 19 | 1.01 |
| 20 | 6.48 |
| 21 | 2.00 |
| 22 | 1.04 |
| 23 | 12.69 |
| 24 | 2.07 |
| 25 | 5.98 |
| analytical sensitivity | 16.64 |

### 7) Detection of serum NMI levels in healthy individuals and septic patients

The collected 30 health check-up and 15 septic patients were detected with the kit of Example 8-1, and the results were as shown in Table 34: the signal values of the serum specimens of the measured septic patients and the health check-up were significantly different (p<0.05), and the kit of Example 8-1 had a better detection effect.

**Table 34. Results of detecting collected 30 health check-up and 15 septic patients with the kit of Example 8-1**

| | Serial number | Signal value |
|---|---|---|
| Septic patient | 1 | 34502 |
| | 2 | 33302 |
| | 3 | 39077 |
| | 4 | 46537 |
| | 5 | 17392 |
| | 6 | 50687 |
| | 7 | 84005 |
| | 8 | 85992 |
| | 9 | 53232 |
| | 10 | 137290 |
| | 11 | 586500 |
| | 12 | 65272 |
| | 13 | 243700 |
| | 14 | 140872 |
| | 15 | 33372 |
| Health check-up | 1 | 1920 |
| | 2 | 1730 |
| | 3 | 1980 |
| | 4 | 2140 |
| | 5 | 1842 |
| | 6 | 1777 |
| | 7 | 1970 |
| | 8 | 2242 |
| | 9 | 1907 |
| | 10 | 1657 |
| | 11 | 2295 |
| | 12 | 1807 |
| | 13 | 1747 |
| | 14 | 2112 |
| | 15 | 2565 |
| | 16 | 3427 |
| | 17 | 2937 |
| | 18 | 1477 |
| | 19 | 3145 |
| | 20 | 1607 |
| | 21 | 1715 |
| | 22 | 2235 |
| | 23 | 2877 |
| | 24 | 3300 |
| | 25 | 1852 |
| | 26 | 2367 |
| | 27 | 1945 |
| | 28 | 1855 |
| | 29 | 2952 |
| | 30 | 1952 |

### Example 9. A Colloidal gold test strip for the detection of NMI

A colloidal gold test strip for the detection of NMI, as shown in FIG. 8, comprising: a PVC base plate; a sample pad, a colloidal gold pad, a nitrocellulose membrane, and an absorbent filter paper partially overlapping and adhered to the PVC base plate; wherein: the colloidal gold pad was coated with colloidal gold-labeled B-8; the nitrocellulose membrane was provided with: a test line coated with 94C4, and a control line coated with goat anti-rabbit IgG antibody.

### Example 10. A biological material for adsorption of NMI

A biological material for adsorption of NMI, comprising: a Protein G chromatography column coupled with B-8.

The above examples are better embodiments of the present invention, but the embodiments of the present invention are not limited by the above examples, and any other changes, modifications, substitutions, combinations, and simplifications made without departing from the spirit and principles of the present invention shall be equivalent replacements and are included in the scope of protection of the present invention.

## Claims

1. An anti-NMI antibody or antigen-binding fragment thereof, wherein the antibody or antigen-binding fragment thereof is 94C4, B-8, 240B10-1, 98G10, or 39B4; the antibody or antigen-binding fragment thereof comprises a heavy chain variable region and a light chain variable region:
the heavy chain variable region of the 94C4 is any one of a1) to a2):
a1) which comprises CDR-H1, CDR-H2, and CDR-H3 of the heavy chain variable region with an amino acid sequence as shown in SEQ ID NO: 4;
a2) which comprises CDR-H1, CDR-H2, and CDR-H3 of the heavy chain variable region with an amino acid sequence derived by substituting 1, 2, 3, 4, or 5 amino acids of SEQ ID NO: 4 while having the same function as the protein as shown in SEQ ID NO: 4;
the amino acid that is substituted is an amino acid in CDR, and the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the light chain variable region of the 94C4 is any one of b1) to b2):
b1) which comprises CDR-L1, CDR-L2, and CDR-L3 of the light chain variable region with an amino acid sequence as shown in SEQ ID NO: 21;
b2) which comprises CDR-L1, CDR-L2, and CDR-L3 of the light chain variable region with an amino acid sequence derived by substituting 1, 2, 3, 4, or 5 amino acids of SEQ ID NO: 21 while having the same function as the protein as shown in SEQ ID NO: 21;
the amino acid that is substituted is an amino acid in CDR, and the amino acid that is substituted is selected from: an amino acid other than Y29, N30, Y32, S34, L46, Y49, R50, S52, T53, L54, S56, S94, A95, and D97 in SEQ ID NO: 21;
the heavy chain variable region of the B-8 is any one of c1) to c2):
c1) which comprises CDR-H1, CDR-H2, and CDR-H3 of the heavy chain variable region with an amino acid sequence as shown in SEQ ID NO: 32;
c2) which comprises CDR-H1, CDR-H2, and CDR-H3 of the heavy chain variable region with an amino acid sequence derived by substituting 1, 2, 3, 4, or 5 amino acids of SEQ ID NO: 32 while having the same function as the protein as shown in SEQ ID NO: 32;
the amino acid that is substituted is an amino acid in CDR, and the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the light chain variable region of the B-8 is any one of d1) to d2):
d1) which comprises CDR-L1, CDR-L2, and CDR-L3 of the light chain variable region with an amino acid sequence as shown in SEQ ID NO: 49;
d2) which comprises CDR-L1, CDR-L2, and CDR-L3 of the light chain variable region with an amino acid sequence derived by substituting 1, 2, 3, 4, or 5 amino acids of SEQ ID NO: 49 while having the same function as the protein as shown in SEQ ID NO: 49;
the amino acid that is substituted is an amino acid in CDR, and the amino acid that is substituted is selected from: an amino acid other than Y30, T31, N32, L92, Y93, S94, R95, 196, A97, D98, and R99 in SEQ ID NO: 49;
the heavy chain variable region of the 240B10-1 is any one of e1) to e2):
e1) which comprises CDR-H1, CDR-H2, and CDR-H3 of the heavy chain variable region with an amino acid sequence as shown in SEQ ID NO: 60;
e2) which comprises CDR-H1, CDR-H2, and CDR-H3 of the heavy chain variable region with an amino acid sequence derived by substituting 1, 2, 3, 4, or 5 amino acids of SEQ ID NO: 60 while having the same function as the protein as shown in SEQ ID NO: 60;
the amino acid that is substituted is an amino acid in CDR, and the amino acid that is substituted is selected from: an amino acid other than D27, L28, S29, S30, Y31, T32, S51, G52, G53, and G54 in SEQ ID NO: 60;
the light chain variable region of the 240B10-1 is any one of f1) to f2):
f1) which comprises CDR-L1, CDR-L2, and CDR-L3 of the light chain variable region with an amino acid sequence as shown in SEQ ID NO: 77;
f2) which comprises CDR-L1, CDR-L2, and CDR-L3 of the light chain variable region with an amino acid sequence derived by substituting 1, 2, 3, 4, or 5 amino acids of SEQ ID NO: 77 while having the same function as the protein as shown in SEQ ID NO: 77;
the amino acid that is substituted is an amino acid in CDR, and the amino acid that is substituted is selected from: an amino acid other than Y29, N30, D32, Y33, S51, K54, C75, and Y76 in SEQ ID NO: 77;
the heavy chain variable region of the 98G10 is any one of g1) to g2):
g1) which comprises CDR-H1, CDR-H2, and CDR-H3 of the heavy chain variable region with an amino acid sequence as shown in SEQ ID NO: 88;
g2) which comprises CDR-H1, CDR-H2, and CDR-H3 of the heavy chain variable region with an amino acid sequence derived by substituting 1, 2, 3, 4, or 5 amino acids of SEQ ID NO: 88 while having the same function as the protein as shown in SEQ ID NO: 88;
the amino acid that is substituted is an amino acid in CDR, and the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the light chain variable region of the 98G10 is any one of h1) to h2):
h1) which comprises CDR-L1, CDR-L2, and CDR-L3 of the light chain variable region with an amino acid sequence as shown in SEQ ID NO: 105;
h2) which comprises CDR-L1, CDR-L2, and CDR-L3 of the light chain variable region with an amino acid sequence derived by substituting 1, 2, 3, 4, or 5 amino acids of SEQ ID NO: 105 while having the same function as the protein as shown in SEQ ID NO: 105;
the amino acid that is substituted is an amino acid in CDR, and the amino acid that is substituted is selected from: an amino acid other than K53 in SEQ ID NO: 105;
the heavy chain variable region of the 39B4 is any one of i1) to i2):
i1) which comprises CDR-H1, CDR-H2, and CDR-H3 of the heavy chain variable region with an amino acid sequence as shown in SEQ ID NO: 116;
i2) which comprises CDR-H1, CDR-H2, and CDR-H3 of the heavy chain variable region with an amino acid sequence derived by substituting 1, 2, 3, 4, or 5 amino acids of SEQ ID NO: 116 while having the same function as the protein as shown in SEQ ID NO: 116;
the amino acid that is substituted is an amino acid in CDR, and the amino acid that is substituted is selected from: an amino acid other than F27, S28, S30, S31, S32, Y33, Y53, T56, Y102, T104, Y105, G106, A107, G108, V109, Y110, I111, and Y112 in SEQ ID NO: 116;
the light chain variable region of the 39B4 is any one of j1) to j2):
j1) which comprises CDR-L1, CDR-L2, and CDR-L3 of the light chain variable region with an amino acid sequence as shown in SEQ ID NO: 132;
j2) which comprises CDR-L1, CDR-L2, and CDR-L3 of the light chain variable region with an amino acid sequence derived by substituting 1, 2, 3, 4, or 5 amino acids of SEQ ID NO: 132 while having the same function as the protein as shown in SEQ ID NO: 132;
the amino acid that is substituted is an amino acid in CDR, and the amino acid that is substituted is selected from: an amino acid other than S28, N31, Y93, and S96 in SEQ ID NO: 132.

2. An anti-NMI antibody or antigen-binding fragment thereof, wherein the antibody or antigen-binding fragment thereof is 94C4, B-8, 240B10-1, 98G10, or 39B4; the antibody or antigen-binding fragment thereof comprises a heavy chain variable region and a light chain variable region:
the 94C4 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
a111) SEQ ID NO: 5;
a112) an amino acid sequence derived by substituting 1, 2, or 3 amino acids of SEQ ID NO: 5 while having the same function as the protein as shown in SEQ ID NO: 5, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
a211) SEQ ID NO: 6;
a212) an amino acid sequence derived by substituting 1, 2, or 3 amino acids of SEQ ID NO: 6 while having the same function as the protein as shown in SEQ ID NO: 6, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
a311) SEQ ID NO: 7;
a312) an amino acid sequence derived by substituting 1, 2, or 3 amino acids of SEQ ID NO: 7 while having the same function as the protein as shown in SEQ ID NO: 7, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
a411) SEQ ID NO: 22;
a412) an amino acid sequence derived by substituting 1, 2, or 3 amino acids of SEQ ID NO: 22 while having the same function as the protein as shown in SEQ ID NO: 22, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
a511) SEQ ID NO: 23;
a512) an amino acid sequence derived by substituting 1, 2, or 3 amino acids of SEQ ID NO: 23 while having the same function as the protein as shown in SEQ ID NO: 23, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
a611) SEQ ID NO: 24;
a612) an amino acid sequence derived by substituting 1, 2, or 3 amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the IMGT Definition Scheme,
the CDR-H1 is any one of a111) to a112):
a111) SEQ ID NO: 8;
a112) an amino acid sequence derived by substituting 1, 2, or 3 amino acids of SEQ ID NO: 8 while having the same function as the protein as shown in SEQ ID NO: 8, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
a211) SEQ ID NO: 9;
a212) an amino acid sequence derived by substituting 1, 2, or 3 amino acids of SEQ ID NO: 9 while having the same function as the protein as shown in SEQ ID NO: 9, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
a311) SEQ ID NO: 10;
a312) an amino acid sequence derived by substituting 1, 2, or 3 amino acids of SEQ ID NO: 10 while having the same function as the protein as shown in SEQ ID NO: 10, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
a411) SEQ ID NO: 25;
a412) an amino acid sequence derived by substituting 1, 2, or 3 amino acids of SEQ ID NO: 25 while having the same function as the protein as shown in SEQ ID NO: 25, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
a511) RVS;
a512) an amino acid sequence derived by substituting 1 or 2 amino acids of RVS while having the same function as RVS, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
a611) SEQ ID NO: 24;
a612) an amino acid sequence derived by substituting 1, 2, or 3 amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the Chothia Definition Scheme,
the CDR-H1 is any one of a111) to a112):
a111) SEQ ID NO: 11;
a112) an amino acid sequence derived by substituting 1, 2, or 3 amino acids of SEQ ID NO: 11 while having the same function as the protein as shown in SEQ ID NO: 11, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
a211) SEQ ID NO: 12;
a212) an amino acid sequence derived by substituting 1, 2, or 3 amino acids of SEQ ID NO: 12 while having the same function as the protein as shown in SEQ ID NO: 12, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
a311) SEQ ID NO: 7;
a312) an amino acid sequence derived by substituting 1, 2, or 3 amino acids of SEQ ID NO: 7 while having the same function as the protein as shown in SEQ ID NO: 7, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
a411) SEQ ID NO: 22;
a412) an amino acid sequence derived by substituting 1, 2, or 3 amino acids of SEQ ID NO: 22 while having the same function as the protein as shown in SEQ ID NO: 22, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
a511) SEQ ID NO: 23;
a512) an amino acid sequence derived by substituting 1, 2, or 3 amino acids of SEQ ID NO: 23 while having the same function as the protein as shown in SEQ ID NO: 23, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
a611) SEQ ID NO: 24;
a612) an amino acid sequence derived by substituting 1, 2, or 3 amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the Contact Definition Scheme,
the CDR-H1 is any one of a111) to a112):
a111) SEQ ID NO: 13;
a112) an amino acid sequence derived by substituting 1, 2, or 3 amino acids of SEQ ID NO: 13 while having the same function as the protein as shown in SEQ ID NO: 13, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
a211) SEQ ID NO: 14;
a212) an amino acid sequence derived by substituting 1, 2, or 3 amino acids of SEQ ID NO: 14 while having the same function as the protein as shown in SEQ ID NO: 14, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
a311) SEQ ID NO: 15;
a312) an amino acid sequence derived by substituting 1, 2, or 3 amino acids of SEQ ID NO: 15 while having the same function as the protein as shown in SEQ ID NO: 15, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
a411) SEQ ID NO: 26;
a412) an amino acid sequence derived by substituting 1, 2, or 3 amino acids of SEQ ID NO: 26 while having the same function as the protein as shown in SEQ ID NO: 26, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
a511) SEQ ID NO: 27;
a512) an amino acid sequence derived by substituting 1, 2, or 3 amino acids of SEQ ID NO: 27 while having the same function as the protein as shown in SEQ ID NO: 27, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
a611) SEQ ID NO: 28;
a612) an amino acid sequence derived by substituting 1, 2, or 3 amino acids of SEQ ID NO: 28 while having the same function as the protein as shown in SEQ ID NO: 28, wherein the amino acid that is substituted is selected from: an amino acid other than Y29, N30, Y32, S34, L46, Y49, R50, S52, T53, L54, S56, S94, A95, and D97 in SEQ ID NO: 21; or
as defined by the Abm Definition Scheme,
the CDR-H1 is any one of a111) to a112):
a111) SEQ ID NO: 16;
a112) an amino acid sequence derived by substituting 1, 2, or 3 amino acids of SEQ ID NO: 16 while having the same function as the protein as shown in SEQ ID NO: 16, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H2 is any one of a211) to a212):
a211) SEQ ID NO: 17;
a212) an amino acid sequence derived by substituting 1, 2, or 3 amino acids of SEQ ID NO: 17 while having the same function as the protein as shown in SEQ ID NO: 17, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-H3 is any one of a311) to a312):
a311) SEQ ID NO: 7;
a312) an amino acid sequence derived by substituting 1, 2, or 3 amino acids of SEQ ID NO: 7 while having the same function as the protein as shown in SEQ ID NO: 7, wherein the amino acid that is substituted is selected from: an amino acid other than F26, S29, S30, N31, A32, C49, I50, S51, S52, S54, S60, Y62, R100, D101, G102, D104, and L105 in SEQ ID NO: 4;
the CDR-L1 is any one of a411) to a412):
a411) SEQ ID NO: 22;
a412) an amino acid sequence derived by substituting 1, 2, or 3 amino acids of SEQ ID NO: 22 while having the same function as the protein as shown in SEQ ID NO: 22, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L2 is any one of a511) to a512):
a511) SEQ ID NO: 23;
a512) an amino acid sequence derived by substituting 1, 2, or 3 amino acids of SEQ ID NO: 23 while having the same function as the protein as shown in SEQ ID NO: 23, wherein the amino acid that is substituted is selected from: an amino acid other than Y29, N30, Y32, S34, L46, Y49, R50, S52, T53, L54, S56, S94, A95, and D97 in SEQ ID NO: 21;
the CDR-L3 is any one of a611) to a612):
a611) SEQ ID NO: 24;
a612) an amino acid sequence derived by substituting 1, 2, or 3 amino acids of SEQ ID NO: 24 while having the same function as the protein as shown in SEQ ID NO: 24, wherein the amino acid that is substituted is selected from: an amino acid other than Y29, N30, Y32, S34, L46, Y49, R50, S52, T53, L54, S56, S94, A95, and D97 in SEQ ID NO: 21; or
the B-8 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
a111) SEQ ID NO: 33;
a112) an amino acid sequence derived by substituting 1, 2, or 3 amino acids of SEQ ID NO: 33 while having the same function as the protein as shown in SEQ ID NO: 33, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H2 is any one of a211) to a212):
a211) SEQ ID NO: 34;
a212) an amino acid sequence derived by substituting 1, 2, or 3 amino acids of SEQ ID NO: 34 while having the same function as the protein as shown in SEQ ID NO: 34, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
a311) SEQ ID NO: 35;
a312) an amino acid sequence derived by substituting 1, 2, or 3 amino acids of SEQ ID NO: 35 while having the same function as the protein as shown in SEQ ID NO: 35, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-L1 is any one of a411) to a412):
a411) SEQ ID NO: 50;
a412) an amino acid sequence derived by substituting 1, 2, or 3 amino acids of SEQ ID NO: 50 while having the same function as the protein as shown in SEQ ID NO: 50, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
a511) SEQ ID NO: 51;
a512) an amino acid sequence derived by substituting 1, 2, or 3 amino acids of SEQ ID NO: 51 while having the same function as the protein as shown in SEQ ID NO: 51, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
a611) SEQ ID NO: 52;
a612) an amino acid sequence derived by substituting 1, 2, or 3 amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49; or
as defined by the IMGT Definition Scheme,
the CDR-H1 is any one of a111) to a112):
a111) SEQ ID NO: 36;
a112) an amino acid sequence derived by substituting 1, 2, or 3 (preferably 1) amino acids of SEQ ID NO: 36 while having the same function as the protein as shown in SEQ ID NO: 36, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32 (the amino acid that is substituted is preferably selected from Y33 in SEQ ID NO: 32);
the CDR-H2 is any one of a211) to a212):
a211) SEQ ID NO: 37;
a212) an amino acid sequence derived by substituting 1, 2, or 3 amino acids of SEQ ID NO: 37 while having the same function as the protein as shown in SEQ ID NO: 37, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
a311) SEQ ID NO: 38;
a312) an amino acid sequence derived by substituting 1, 2, or 3 amino acids of SEQ ID NO: 38 while having the same function as the protein as shown in SEQ ID NO: 38, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-L1 is any one of a411) to a412):
a411) SEQ ID NO: 53;
a412) an amino acid sequence derived by substituting 1, 2, or 3 amino acids of SEQ ID NO: 53 while having the same function as the protein as shown in SEQ ID NO: 53, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
a511) GAS;
a512) an amino acid sequence derived by substituting 1 or 2 amino acids of GAS while having the same function as GAS, wherein the amino acid that is substituted is selected from: an amino acid other than Y30, T31, N32, L92, Y93, S94, R95, I96, A97, D98, and R99 in SEQ ID NO: 49;
the CDR-L3 is any one of a611) to a612):
a611) SEQ ID NO: 52;
a612) an amino acid sequence derived by substituting 1, 2, or 3 amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49; or
as defined by the Chothia Definition Scheme,
the CDR-H1 is any one of a111) to a112):
a111) SEQ ID NO: 39;
a112) an amino acid sequence derived by substituting 1, 2, or 3 amino acids of SEQ ID NO: 39 while having the same function as the protein as shown in SEQ ID NO: 39, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H2 is any one of a211) to a212):
a211) SEQ ID NO: 40;
a212) an amino acid sequence derived by substituting 1, 2, or 3 amino acids of SEQ ID NO: 40 while having the same function as the protein as shown in SEQ ID NO: 40, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
a311) SEQ ID NO: 35;
a312) an amino acid sequence derived by substituting 1, 2, or 3 amino acids of SEQ ID NO: 35 while having the same function as the protein as shown in SEQ ID NO: 35, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-L1 is any one of a411) to a412):
a411) SEQ ID NO: 50;
a412) an amino acid sequence derived by substituting 1, 2, or 3 amino acids of SEQ ID NO: 50 while having the same function as the protein as shown in SEQ ID NO: 50, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
a511) SEQ ID NO: 51;
a512) an amino acid sequence derived by substituting 1, 2, or 3 amino acids of SEQ ID NO: 51 while having the same function as the protein as shown in SEQ ID NO: 51, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
a611) SEQ ID NO: 52;
a612) an amino acid sequence derived by substituting 1, 2, or 3 amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49; or
as defined by the Contact Definition Scheme,
the CDR-H1 is any one of a111) to a112):
a111) SEQ ID NO: 41;
a112) an amino acid sequence derived by substituting 1, 2, or 3 amino acids of SEQ ID NO: 41 while having the same function as the protein as shown in SEQ ID NO: 41, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H2 is any one of a211) to a212):
a211) SEQ ID NO: 42;
a212) an amino acid sequence derived by substituting 1, 2, or 3 amino acids of SEQ ID NO: 42 while having the same function as the protein as shown in SEQ ID NO: 42, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
a311) SEQ ID NO: 43;
a312) an amino acid sequence derived by substituting 1, 2, or 3 amino acids of SEQ ID NO: 43 while having the same function as the protein as shown in SEQ ID NO: 43, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-L1 is any one of a411) to a412):
a411) SEQ ID NO: 54;
a412) an amino acid sequence derived by substituting 1, 2, or 3 amino acids of SEQ ID NO: 54 while having the same function as the protein as shown in SEQ ID NO: 54, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
a511) SEQ ID NO: 55;
a512) an amino acid sequence derived by substituting 1, 2, or 3 amino acids of SEQ ID NO: 55 while having the same function as the protein as shown in SEQ ID NO: 55, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
a611) SEQ ID NO: 56;
a612) an amino acid sequence derived by substituting 1, 2, or 3 amino acids of SEQ ID NO: 56 while having the same function as the protein as shown in SEQ ID NO: 56, wherein the amino acid that is substituted is selected from: an amino acid other than Y30, T31, N32, L92, Y93, S94, R95, 196, A97, D98, and R99 in SEQ ID NO: 49; or
as defined by the Abm Definition Scheme,
the CDR-H1 is any one of a111) to a112):
a111) SEQ ID NO: 44;
a112) an amino acid sequence derived by substituting 1, 2, or 3 amino acids of SEQ ID NO: 44 while having the same function as the protein as shown in SEQ ID NO: 44, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H2 is any one of a211) to a212):
a211) SEQ ID NO: 45;
a212) an amino acid sequence derived by substituting 1, 2, or 3 amino acids of SEQ ID NO: 45 while having the same function as the protein as shown in SEQ ID NO: 45, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-H3 is any one of a311) to a312):
a311) SEQ ID NO: 35;
a312) an amino acid sequence derived by substituting 1, 2, or 3 amino acids of SEQ ID NO: 35 while having the same function as the protein as shown in SEQ ID NO: 35, wherein the amino acid that is substituted is selected from: an amino acid other than S30, S31, Y33, Y52, S55, S56, S58, T59, Y60, K66, Y100, S101, D103, D104, Y105, G106, D107, F108, and W109 in SEQ ID NO: 32;
the CDR-L1 is any one of a411) to a412):
a411) SEQ ID NO: 50;
a412) an amino acid sequence derived by substituting 1, 2, or 3 amino acids of SEQ ID NO: 50 while having the same function as the protein as shown in SEQ ID NO: 50, wherein the amino acid that is substituted is selected from: an amino acid other than Y30, T31, N32, L92, Y93, S94, R95, 196, A97, D98, and R99 in SEQ ID NO: 49;
the CDR-L2 is any one of a511) to a512):
a511) SEQ ID NO: 51;
a512) an amino acid sequence derived by substituting 1, 2, or 3 amino acids of SEQ ID NO: 51 while having the same function as the protein as shown in SEQ ID NO: 51, wherein the amino acid that is substituted is selected from: an amino acid other than Y30, T31, N32, L92, Y93, S94, R95, 196, A97, D98, and R99 in SEQ ID NO: 49;
the CDR-L3 is any one of a611) to a612):
a611) SEQ ID NO: 52;
a612) an amino acid sequence derived by substituting 1, 2, or 3 amino acids of SEQ ID NO: 52 while having the same function as the protein as shown in SEQ ID NO: 52, wherein the amino acid that is substituted is selected from: an amino acid other than Y30, T31, N32, L92, Y93, S94, R95, 196, A97, D98, and R99 in SEQ ID NO: 49; or
the 240B10-1 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
a111) SEQ ID NO: 61;
a112) an amino acid sequence derived by substituting 1, 2, or 3 amino acids of SEQ ID NO: 61 while having the same function as the protein as shown in SEQ ID NO: 61, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
a211) SEQ ID NO: 62;
a212) an amino acid sequence derived by substituting 1, 2, or 3 amino acids of SEQ ID NO: 62 while having the same function as the protein as shown in SEQ ID NO: 62, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
a311) SEQ ID NO: 63;
a312) an amino acid sequence derived by substituting 1, 2, or 3 amino acids of SEQ ID NO: 63 while having the same function as the protein as shown in SEQ ID NO: 63, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
a411) SEQ ID NO: 78;
a412) an amino acid sequence derived by substituting 1, 2, or 3 amino acids of SEQ ID NO: 78 while having the same function as the protein as shown in SEQ ID NO: 78, wherein the amino acid that is substituted is selected from: an amino acid other than Y29, N30, D32, Y33, S51, K54, C75, and Y76 in SEQ ID NO: 77;
the CDR-L2 is any one of a511) to a512):
a511) SEQ ID NO: 79;
a512) an amino acid sequence derived by substituting 1, 2, or 3 amino acids of SEQ ID NO: 79 while having the same function as the protein as shown in SEQ ID NO: 79, wherein the amino acid that is substituted is selected from: an amino acid other than Y29, N30, D32, Y33, S51, K54, C75, and Y76 in SEQ ID NO: 77;
the CDR-L3 is any one of a611) to a612):
a611) SEQ ID NO: 80;
a612) an amino acid sequence derived by substituting 1, 2, or 3 amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
as defined by the IMGT Definition Scheme,
the CDR-H1 is any one of a111) to a112):
a111) SEQ ID NO: 64;
a112) an amino acid sequence derived by substituting 1, 2, or 3 amino acids of SEQ ID NO: 64 while having the same function as the protein as shown in SEQ ID NO: 64, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
a211) SEQ ID NO: 65;
a212) an amino acid sequence derived by substituting 1, 2, or 3 amino acids of SEQ ID NO: 65 while having the same function as the protein as shown in SEQ ID NO: 65, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
a311) SEQ ID NO: 66;
a312) an amino acid sequence derived by substituting 1, 2, or 3 amino acids of SEQ ID NO: 66 while having the same function as the protein as shown in SEQ ID NO: 66, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
a411) SEQ ID NO: 81;
a412) an amino acid sequence derived by substituting 1, 2, or 3 amino acids of SEQ ID NO: 81 while having the same function as the protein as shown in SEQ ID NO: 81, wherein the amino acid that is substituted is selected from: an amino acid other than Y29, N30, D32, Y33, S51, K54, C75, and Y76 in SEQ ID NO: 77;
the CDR-L2 is any one of a511) to a512):
a511) SAS;
a512) an amino acid sequence derived by substituting 1 or 2 amino acids of SAS while having the same function as SAS, wherein the amino acid that is substituted is selected from: an amino acid other than Y29, N30, D32, Y33, S51, K54, C75, and Y76 in SEQ ID NO: 77;
the CDR-L3 is any one of a611) to a612):
a611) SEQ ID NO: 80;
a612) an amino acid sequence derived by substituting 1, 2, or 3 amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
as defined by the Chothia Definition Scheme,
the CDR-H1 is any one of a111) to a112):
a111) SEQ ID NO: 67;
a112) an amino acid sequence derived by substituting 1, 2, or 3 amino acids of SEQ ID NO: 67 while having the same function as the protein as shown in SEQ ID NO: 67, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
a211) SEQ ID NO: 68;
a212) an amino acid sequence derived by substituting 1, 2, or 3 amino acids of SEQ ID NO: 68 while having the same function as the protein as shown in SEQ ID NO: 68, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
a311) SEQ ID NO: 63;
a312) an amino acid sequence derived by substituting 1, 2, or 3 amino acids of SEQ ID NO: 63 while having the same function as the protein as shown in SEQ ID NO: 63, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
a411) SEQ ID NO: 78;
a412) an amino acid sequence derived by substituting 1, 2, or 3 amino acids of SEQ ID NO: 78 while having the same function as the protein as shown in SEQ ID NO: 78, wherein the amino acid that is substituted is selected from: an amino acid other than Y29, N30, D32, Y33, S51, K54, C75, and Y76 in SEQ ID NO: 77;
the CDR-L2 is any one of a511) to a512):
a511) SEQ ID NO: 79;
a512) an amino acid sequence derived by substituting 1, 2, or 3 amino acids of SEQ ID NO: 79 while having the same function as the protein as shown in SEQ ID NO: 79, wherein the amino acid that is substituted is selected from: an amino acid other than Y29, N30, D32, Y33, S51, K54, C75, and Y76 in SEQ ID NO: 77;
the CDR-L3 is any one of a611) to a612):
a611) SEQ ID NO: 80;
a612) an amino acid sequence derived by substituting 1, 2, or 3 amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
as defined by the Contact Definition Scheme,
the CDR-H1 is any one of a111) to a112):
a111) SEQ ID NO: 69;
a112) an amino acid sequence derived by substituting 1, 2, or 3 amino acids of SEQ ID NO: 69 while having the same function as the protein as shown in SEQ ID NO: 69, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
a211) SEQ ID NO: 70;
a212) an amino acid sequence derived by substituting 1, 2, or 3 amino acids of SEQ ID NO: 70 while having the same function as the protein as shown in SEQ ID NO: 70, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
a311) SEQ ID NO: 71;
a312) an amino acid sequence derived by substituting 1, 2, or 3 amino acids of SEQ ID NO: 71 while having the same function as the protein as shown in SEQ ID NO: 71, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
a411) SEQ ID NO: 82;
a412) an amino acid sequence derived by substituting 1, 2, or 3 amino acids of SEQ ID NO: 82 while having the same function as the protein as shown in SEQ ID NO: 82, wherein the amino acid that is substituted is selected from: an amino acid other than Y29, N30, D32, Y33, S51, K54, C75, and Y76 in SEQ ID NO: 77;
the CDR-L2 is any one of a511) to a512):
a511) SEQ ID NO: 83;
a512) an amino acid sequence derived by substituting 1, 2, or 3 amino acids of SEQ ID NO: 83 while having the same function as the protein as shown in SEQ ID NO: 83, wherein the amino acid that is substituted is selected from: an amino acid other than Y29, N30, D32, Y33, S51, K54, C75, and Y76 in SEQ ID NO: 77;
the CDR-L3 is any one of a611) to a612):
a611) SEQ ID NO: 84;
a612) an amino acid sequence derived by substituting 1, 2, or 3 amino acids of SEQ ID NO: 84 while having the same function as the protein as shown in SEQ ID NO: 84, wherein the amino acid that is substituted is selected from: an amino acid other than Y29, N30, D32, Y33, S51, K54, C75, and Y76 in SEQ ID NO: 77; or
as defined by the Abm Definition Scheme,
the CDR-H1 is any one of a111) to a112):
a111) SEQ ID NO: 72;
a112) an amino acid sequence derived by substituting 1, 2, or 3 amino acids of SEQ ID NO: 72 while having the same function as the protein as shown in SEQ ID NO: 72, wherein the amino acid that is substituted is selected from: an amino acid other than D27, L28, S29, S30, Y31, T32, S51, G52, G53, and G54 in SEQ ID NO: 60;
the CDR-H2 is any one of a211) to a212):
a211) SEQ ID NO: 73;
a212) an amino acid sequence derived by substituting 1, 2, or 3 amino acids of SEQ ID NO: 73 while having the same function as the protein as shown in SEQ ID NO: 73, wherein the amino acid that is substituted is selected from: an amino acid other than D27, L28, S29, S30, Y31, T32, S51, G52, G53, and G54 in SEQ ID NO: 60;
the CDR-H3 is any one of a311) to a312):
a311) SEQ ID NO: 63;
a312) an amino acid sequence derived by substituting 1, 2, or 3 amino acids of SEQ ID NO: 63 while having the same function as the protein as shown in SEQ ID NO: 63, wherein the amino acid that is substituted is selected from: an amino acid other than D27, L28, S29, S30, Y31, T32, S51, G52, G53, and G54 in SEQ ID NO: 60;
the CDR-L1 is any one of a411) to a412):
a411) SEQ ID NO: 78;
a412) an amino acid sequence derived by substituting 1, 2, or 3 amino acids of SEQ ID NO: 78 while having the same function as the protein as shown in SEQ ID NO: 78, wherein the amino acid that is substituted is selected from: an amino acid other than Y29, N30, D32, Y33, S51, K54, C75, and Y76 in SEQ ID NO: 77;
the CDR-L2 is any one of a511) to a512):
a511) SEQ ID NO: 79;
a512) an amino acid sequence derived by substituting 1, 2, or 3 amino acids of SEQ ID NO: 79 while having the same function as the protein as shown in SEQ ID NO: 79, wherein the amino acid that is substituted is selected from: an amino acid other than Y29, N30, D32, Y33, S51, K54, C75, and Y76 in SEQ ID NO: 77;
the CDR-L3 is any one of a611) to a612):
a611) SEQ ID NO: 80;
a612) an amino acid sequence derived by substituting 1, 2, or 3 amino acids of SEQ ID NO: 80 while having the same function as the protein as shown in SEQ ID NO: 80, wherein the amino acid that is substituted is selected from: an amino acid other than Y29, N30, D32, Y33, S51, K54, C75, and Y76 in SEQ ID NO: 77; or
the 98G10 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
a111) SEQ ID NO: 89;
a112) an amino acid sequence derived by substituting 1, 2, or 3 amino acids of SEQ ID NO: 89 while having the same function as the protein as shown in SEQ ID NO: 89, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H2 is any one of a211) to a212):
a211) SEQ ID NO: 90;
a212) an amino acid sequence derived by substituting 1, 2, or 3 amino acids of SEQ ID NO: 90 while having the same function as the protein as shown in SEQ ID NO: 90, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H3 is any one of a311) to a312):
a311) SEQ ID NO: 91;
a312) an amino acid sequence derived by substituting 1, 2, or 3 amino acids of SEQ ID NO: 91 while having the same function as the protein as shown in SEQ ID NO: 91, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-L1 is any one of a411) to a412):
a411) SEQ ID NO: 106;
a412) an amino acid sequence derived by substituting 1, 2, or 3 amino acids of SEQ ID NO: 106 while having the same function as the protein as shown in SEQ ID NO: 106, wherein the amino acid that is substituted is selected from: an amino acid other than K53 in SEQ ID NO: 105;
the CDR-L2 is any one of a511) to a512):
a511) SEQ ID NO: 107;
a512) an amino acid sequence derived by substituting 1, 2, or 3 amino acids of SEQ ID NO: 107 while having the same function as the protein as shown in SEQ ID NO: 107, wherein the amino acid that is substituted is selected from: an amino acid other than K53 in SEQ ID NO: 105;
the CDR-L3 is any one of a611) to a612):
a611) SEQ ID NO: 108;
a612) an amino acid sequence derived by substituting 1, 2, or 3 amino acids of SEQ ID NO: 108 while having the same function as the protein as shown in SEQ ID NO: 108, wherein the amino acid that is substituted is selected from: an amino acid other than K53 in SEQ ID NO: 105; or
as defined by the IMGT Definition Scheme,
the CDR-H1 is any one of a111) to a112):
a111) SEQ ID NO: 92;
a112) an amino acid sequence derived by substituting 1, 2, or 3 amino acids of SEQ ID NO: 92 while having the same function as the protein as shown in SEQ ID NO: 92, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H2 is any one of a211) to a212):
a211) SEQ ID NO: 93;
a212) an amino acid sequence derived by substituting 1, 2, or 3 amino acids of SEQ ID NO: 93 while having the same function as the protein as shown in SEQ ID NO: 93, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H3 is any one of a311) to a312):
a311) SEQ ID NO: 94;
a312) an amino acid sequence derived by substituting 1, 2, or 3 amino acids of SEQ ID NO: 94 while having the same function as the protein as shown in SEQ ID NO: 94, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-L1 is any one of a411) to a412):
a411) SEQ ID NO: 109;
a412) an amino acid sequence derived by substituting 1, 2, or 3 amino acids of SEQ ID NO: 109 while having the same function as the protein as shown in SEQ ID NO: 109, wherein the amino acid that is substituted is selected from: an amino acid other than K53 in SEQ ID NO: 105;
the CDR-L2 is any one of a511) to a512):
a511) TAS;
a512) an amino acid sequence derived by substituting 1 or 2 amino acids of TAS while having the same function as TAS, wherein the amino acid that is substituted is selected from: an amino acid other than K53 in SEQ ID NO: 105;
the CDR-L3 is any one of a611) to a612):
a611) SEQ ID NO: 108;
a612) an amino acid sequence derived by substituting 1, 2, or 3 amino acids of SEQ ID NO: 108 while having the same function as the protein as shown in SEQ ID NO: 108, wherein the amino acid that is substituted is selected from: an amino acid other than K53 in SEQ ID NO: 105; or
as defined by the Chothia Definition Scheme,
the CDR-H1 is any one of a111) to a112):
a111) SEQ ID NO: 95;
a112) an amino acid sequence derived by substituting 1, 2, or 3 amino acids of SEQ ID NO: 95 while having the same function as the protein as shown in SEQ ID NO: 95, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H2 is any one of a211) to a212):
a211) SEQ ID NO: 96;
a212) an amino acid sequence derived by substituting 1, 2, or 3 amino acids of SEQ ID NO: 96 while having the same function as the protein as shown in SEQ ID NO: 96, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H3 is any one of a311) to a312):
a311) SEQ ID NO: 91;
a312) an amino acid sequence derived by substituting 1, 2, or 3 amino acids of SEQ ID NO: 91 while having the same function as the protein as shown in SEQ ID NO: 91, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-L1 is any one of a411) to a412):
a411) SEQ ID NO: 106;
a412) an amino acid sequence derived by substituting 1, 2, or 3 amino acids of SEQ ID NO: 106 while having the same function as the protein as shown in SEQ ID NO: 106, wherein the amino acid that is substituted is selected from: an amino acid other than K53 in SEQ ID NO: 105;
the CDR-L2 is any one of a511) to a512):
a511) SEQ ID NO: 107;
a512) an amino acid sequence derived by substituting 1, 2, or 3 amino acids of SEQ ID NO: 107 while having the same function as the protein as shown in SEQ ID NO: 107, wherein the amino acid that is substituted is selected from: an amino acid other than K53 in SEQ ID NO: 105;
the CDR-L3 is any one of a611) to a612):
a611) SEQ ID NO: 108;
a612) an amino acid sequence derived by substituting 1, 2, or 3 amino acids of SEQ ID NO: 108 while having the same function as the protein as shown in SEQ ID NO: 108, wherein the amino acid that is substituted is selected from: an amino acid other than K53 in SEQ ID NO: 105; or
as defined by the Contact Definition Scheme,
the CDR-H1 is any one of a111) to a112):
a111) SEQ ID NO: 97;
a112) an amino acid sequence derived by substituting 1, 2, or 3 amino acids of SEQ ID NO: 97 while having the same function as the protein as shown in SEQ ID NO: 97, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H2 is any one of a211) to a212):
a211) SEQ ID NO: 98;
a212) an amino acid sequence derived by substituting 1, 2, or 3 amino acids of SEQ ID NO: 98 while having the same function as the protein as shown in SEQ ID NO: 98, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H3 is any one of a311) to a312):
a311) SEQ ID NO: 99;
a312) an amino acid sequence derived by substituting 1, 2, or 3 amino acids of SEQ ID NO: 99 while having the same function as the protein as shown in SEQ ID NO: 99, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-L1 is any one of a411) to a412):
a411) SEQ ID NO: 110;
a412) an amino acid sequence derived by substituting 1, 2, or 3 amino acids of SEQ ID NO: 110 while having the same function as the protein as shown in SEQ ID NO: 110, wherein the amino acid that is substituted is selected from: an amino acid other than K53 in SEQ ID NO: 105;
the CDR-L2 is any one of a511) to a512):
a511) SEQ ID NO: 111;
a512) an amino acid sequence derived by substituting 1, 2, or 3 amino acids of SEQ ID NO: 111 while having the same function as the protein as shown in SEQ ID NO: 111, wherein the amino acid that is substituted is selected from: an amino acid other than K53 in SEQ ID NO: 105;
the CDR-L3 is any one of a611) to a612):
a611) SEQ ID NO: 112;
a612) an amino acid sequence derived by substituting 1, 2, or 3 amino acids of SEQ ID NO: 112 while having the same function as the protein as shown in SEQ ID NO: 112, wherein the amino acid that is substituted is selected from: an amino acid other than K53 in SEQ ID NO: 105; or
as defined by the Abm Definition Scheme,
the CDR-H1 is any one of a111) to a112):
a111) SEQ ID NO: 100;
a112) an amino acid sequence derived by substituting 1, 2, or 3 amino acids of SEQ ID NO: 100 while having the same function as the protein as shown in SEQ ID NO: 100, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H2 is any one of a211) to a212):
a211) SEQ ID NO: 101;
a212) an amino acid sequence derived by substituting 1, 2, or 3 amino acids of SEQ ID NO: 101 while having the same function as the protein as shown in SEQ ID NO: 101, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-H3 is any one of a311) to a312):
a311) SEQ ID NO: 91;
a312) an amino acid sequence derived by substituting 1, 2, or 3 amino acids of SEQ ID NO: 91 while having the same function as the protein as shown in SEQ ID NO: 91, wherein the amino acid that is substituted is selected from: an amino acid other than F29, S30, A31, Y53, G55, S56, S57, G58, S59, Y61, Y102, N104, S107, A108, G109, D110, I111, and Y112 in SEQ ID NO: 88;
the CDR-L1 is any one of a411) to a412):
a411) SEQ ID NO: 106;
a412) an amino acid sequence derived by substituting 1, 2, or 3 amino acids of SEQ ID NO: 106 while having the same function as the protein as shown in SEQ ID NO: 106, wherein the amino acid that is substituted is selected from: an amino acid other than K53 in SEQ ID NO: 105;
the CDR-L2 is any one of a511) to a512):
a511) SEQ ID NO: 107;
a512) an amino acid sequence derived by substituting 1, 2, or 3 amino acids of SEQ ID NO: 107 while having the same function as the protein as shown in SEQ ID NO: 107, wherein the amino acid that is substituted is selected from: an amino acid other than K53 in SEQ ID NO: 105;
the CDR-L3 is any one of a611) to a612):
a611) SEQ ID NO: 108;
a612) an amino acid sequence derived by substituting 1, 2, or 3 amino acids of SEQ ID NO: 108 while having the same function as the protein as shown in SEQ ID NO: 108, wherein the amino acid that is substituted is selected from: an amino acid other than K53 in SEQ ID NO: 105; or
the 39B4 comprises a heavy chain variable region and a light chain variable region, the heavy chain variable region comprises CDR-H1, CDR-H2 and CDR-H3, and the light chain variable region comprises CDR-L1, CDR-L2 and CDR-L3;
as defined by the Kabat Definition Scheme,
the CDR-H1 is any one of a111) to a112):
a111) SEQ ID NO: 117;
a112) an amino acid sequence derived by substituting 1, 2, or 3 amino acids of SEQ ID NO: 117 while having the same function as the protein as shown in SEQ ID NO: 117, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
a211) SEQ ID NO: 118;
a212) an amino acid sequence derived by substituting 1, 2, or 3 amino acids of SEQ ID NO: 118 while having the same function as the protein as shown in SEQ ID NO: 118, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
a311) SEQ ID NO: 119;
a312) an amino acid sequence derived by substituting 1, 2, or 3 amino acids of SEQ ID NO: 119 while having the same function as the protein as shown in SEQ ID NO: 119, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
a411) SEQ ID NO: 133;
a412) an amino acid sequence derived by substituting 1, 2, or 3 amino acids of SEQ ID NO: 133 while having the same function as the protein as shown in SEQ ID NO: 133, wherein the amino acid that is substituted is selected from: an amino acid other than S28, N31, Y93, and S96 in SEQ ID NO: 132;
the CDR-L2 is any one of a511) to a512):
a511) SEQ ID NO: 134;
a512) an amino acid sequence derived by substituting 1, 2, or 3 amino acids of SEQ ID NO: 134 while having the same function as the protein as shown in SEQ ID NO: 134, wherein the amino acid that is substituted is selected from: an amino acid other than S28, N31, Y93, and S96 in SEQ ID NO: 132;
the CDR-L3 is any one of a611) to a612):
a611) SEQ ID NO: 135;
a612) an amino acid sequence derived by substituting 1, 2, or 3 amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132; or
as defined by the IMGT Definition Scheme,
the CDR-H1 is any one of a111) to a112):
a111) SEQ ID NO: 120;
a112) an amino acid sequence derived by substituting 1, 2, or 3 amino acids of SEQ ID NO: 120 while having the same function as the protein as shown in SEQ ID NO: 120, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
a211) SEQ ID NO: 121;
a212) an amino acid sequence derived by substituting 1, 2, or 3 amino acids of SEQ ID NO: 121 while having the same function as the protein as shown in SEQ ID NO: 121, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
a311) SEQ ID NO: 122;
a312) an amino acid sequence derived by substituting 1, 2, or 3 amino acids of SEQ ID NO: 122 while having the same function as the protein as shown in SEQ ID NO: 122, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
a411) SEQ ID NO: 136;
a412) an amino acid sequence derived by substituting 1, 2, or 3 amino acids of SEQ ID NO: 136 while having the same function as the protein as shown in SEQ ID NO: 136, wherein the amino acid that is substituted is selected from: an amino acid other than S28, N31, Y93, and S96 in SEQ ID NO: 132;
the CDR-L2 is any one of a511) to a512):
a511) SAS;
a512) an amino acid sequence derived by substituting 1 or 2 amino acids of SAS while having the same function as SAS, wherein the amino acid that is substituted is selected from: an amino acid other than S28, N31, Y93, and S96 in SEQ ID NO: 132;
the CDR-L3 is any one of a611) to a612):
a611) SEQ ID NO: 135;
a612) an amino acid sequence derived by substituting 1, 2, or 3 amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132; or
as defined by the Chothia Definition Scheme,
the CDR-H1 is any one of a111) to a112):
a111) SEQ ID NO: 120;
a112) an amino acid sequence derived by substituting 1, 2, or 3 amino acids of SEQ ID NO: 120 while having the same function as the protein as shown in SEQ ID NO: 120, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
a211) SEQ ID NO: 123;
a212) an amino acid sequence derived by substituting 1, 2, or 3 amino acids of SEQ ID NO: 123 while having the same function as the protein as shown in SEQ ID NO: 123, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
a311) SEQ ID NO: 119;
a312) an amino acid sequence derived by substituting 1, 2, or 3 amino acids of SEQ ID NO: 119 while having the same function as the protein as shown in SEQ ID NO: 119, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
a411) SEQ ID NO: 133;
a412) an amino acid sequence derived by substituting 1, 2, or 3 amino acids of SEQ ID NO: 133 while having the same function as the protein as shown in SEQ ID NO: 133, wherein the amino acid that is substituted is selected from: an amino acid other than S28, N31, Y93, and S96 in SEQ ID NO: 132;
the CDR-L2 is any one of a511) to a512):
a511) SEQ ID NO: 134;
a512) an amino acid sequence derived by substituting 1, 2, or 3 amino acids of SEQ ID NO: 134 while having the same function as the protein as shown in SEQ ID NO: 134, wherein the amino acid that is substituted is selected from: an amino acid other than S28, N31, Y93, and S96 in SEQ ID NO: 132;
the CDR-L3 is any one of a611) to a612):
a611) SEQ ID NO: 135;
a612) an amino acid sequence derived by substituting 1, 2, or 3 amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132; or
as defined by the Contact Definition Scheme,
the CDR-H1 is any one of a111) to a112):
a111) SEQ ID NO: 117;
a112) an amino acid sequence derived by substituting 1, 2, or 3 amino acids of SEQ ID NO: 117 while having the same function as the protein as shown in SEQ ID NO: 117, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
a211) SEQ ID NO: 124;
a212) an amino acid sequence derived by substituting 1, 2, or 3 amino acids of SEQ ID NO: 124 while having the same function as the protein as shown in SEQ ID NO: 124, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
a311) SEQ ID NO: 125;
a312) an amino acid sequence derived by substituting 1, 2, or 3 amino acids of SEQ ID NO: 125 while having the same function as the protein as shown in SEQ ID NO: 125, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
a411) SEQ ID NO: 137;
a412) an amino acid sequence derived by substituting 1, 2, or 3 amino acids of SEQ ID NO: 137 while having the same function as the protein as shown in SEQ ID NO: 137, wherein the amino acid that is substituted is selected from: an amino acid other than S28, N31, Y93, and S96 in SEQ ID NO: 132;
the CDR-L2 is any one of a511) to a512):
a511) SEQ ID NO: 138;
a512) an amino acid sequence derived by substituting 1, 2, or 3 amino acids of SEQ ID NO: 138 while having the same function as the protein as shown in SEQ ID NO: 138, wherein the amino acid that is substituted is selected from: an amino acid other than S28, N31, Y93, and S96 in SEQ ID NO: 132;
the CDR-L3 is any one of a611) to a612):
a611) SEQ ID NO: 139;
a612) an amino acid sequence derived by substituting 1, 2, or 3 amino acids of SEQ ID NO: 139 while having the same function as the protein as shown in SEQ ID NO: 139, wherein the amino acid that is substituted is selected from: an amino acid other than S28, N31, Y93, and S96 in SEQ ID NO: 132; or
as defined by the Abm Definition Scheme,
the CDR-H1 is any one of a111) to a112):
a111) SEQ ID NO: 126;
a112) an amino acid sequence derived by substituting 1, 2, or 3 amino acids of SEQ ID NO: 126 while having the same function as the protein as shown in SEQ ID NO: 126, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H2 is any one of a211) to a212):
a211) SEQ ID NO: 127;
a212) an amino acid sequence derived by substituting 1, 2, or 3 amino acids of SEQ ID NO: 127 while having the same function as the protein as shown in SEQ ID NO: 127, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-H3 is any one of a311) to a312):
a311) SEQ ID NO: 128;
a312) an amino acid sequence derived by substituting 1, 2, or 3 amino acids of SEQ ID NO: 128 while having the same function as the protein as shown in SEQ ID NO: 128, wherein the amino acid that is substituted is selected from: an amino acid other than F27, S28, S30, S31, S32, Y33, Y53, T56, Y102, T104, Y105, G106, A107, G108, V109, Y110, 1111, and Y112 in SEQ ID NO: 116;
the CDR-L1 is any one of a411) to a412):
a411) SEQ ID NO: 133;
a412) an amino acid sequence derived by substituting 1, 2, or 3 amino acids of SEQ ID NO: 133 while having the same function as the protein as shown in SEQ ID NO: 133, wherein the amino acid that is substituted is selected from: an amino acid other than S28, N31, Y93, and S96 in SEQ ID NO: 132;
the CDR-L2 is any one of a511) to a512):
a511) SEQ ID NO: 134;
a512) an amino acid sequence derived by substituting 1, 2, or 3 amino acids of SEQ ID NO: 134 while having the same function as the protein as shown in SEQ ID NO: 134, wherein the amino acid that is substituted is selected from: an amino acid other than S28, N31, Y93, and S96 in SEQ ID NO: 132;
the CDR-L3 is any one of a611) to a612):
a611) SEQ ID NO: 135;
a612) an amino acid sequence derived by substituting 1, 2, or 3 amino acids of SEQ ID NO: 135 while having the same function as the protein as shown in SEQ ID NO: 135, wherein the amino acid that is substituted is selected from: an amino acid other than S28, N31, Y93, and S96 in SEQ ID NO: 132.

3. The antibody or antigen-binding fragment thereof according to claim 1 or 2, **characterized in that**:
preferably, the amino acid sequences of CDR-H1, CDR-H2, CDR-H3, CDR-L1, CDR-L2, and CDR-L3 of the 94C4 are as shown in SEQ ID NOs: 5, 6, 7, 22, 23, and 24 in order, and the CDRs are defined by the Kabat Definition Scheme; or
the amino acid sequences of CDR-H1, CDR-H2, CDR-H3, CDR-L1, and CDR-L3 of the 94C4 are as shown in SEQ ID NOs: 8, 9, 10, 25, and 24 in order and the amino acid sequence of CDR-L2 is RVS, and the CDRs are defined by the IMGT Definition Scheme; or
the amino acid sequences of CDR-H1, CDR-H2, CDR-H3, CDR-L1, CDR-L2, and CDR-L3 of the 94C4 are as shown in SEQ ID NOs: 11, 12, 7, 22, 23, and 24 in order, and the CDRs are defined by the Chothia Definition Scheme; or
the amino acid sequences of CDR-H1, CDR-H2, CDR-H3, CDR-L1, CDR-L2, and CDR-L3 of the 94C4 are as shown in SEQ ID NOs: 13, 14, 15, 26, 27, and 28 in order, and the CDRs are defined by the Contact Definition Scheme; or
the amino acid sequences of CDR-H1, CDR-H2, CDR-H3, CDR-L1, CDR-L2, and CDR-L3 of the 94C4 are as shown in SEQ ID NOs: 16, 17, 7, 22, 23, and 24 in order, and the CDRs are defined by the Abm Definition Scheme;
preferably, the amino acid sequences of CDR-H1, CDR-H2, CDR-H3, CDR-L1, CDR-L2, and CDR-L3 of the 240B10-1 are as shown in SEQ ID NOs: 61, 62, 63, 78, 79, and 80 in order, and the CDRs are defined by the Kabat Definition Scheme; or
the amino acid sequences of CDR-H1, CDR-H2, CDR-H3, CDR-L1, and CDR-L3 of the 240B10-1 are as shown in SEQ ID NOs: 64, 65, 66, 81, and 80 in order and the amino acid sequence of CDR-L2 is SAS, and the CDRs are defined by the IMGT Definition Scheme; or
the amino acid sequences of CDR-H1, CDR-H2, CDR-H3, CDR-L1, CDR-L2, and CDR-L3 of the 240B10-1 are as shown in SEQ ID NOs: 67, 68, 63, 78, 79, and 80 in order, and the CDRs are defined by the Chothia Definition Scheme; or
the amino acid sequences of CDR-H1, CDR-H2, CDR-H3, CDR-L1, CDR-L2, and CDR-L3 of the 240B10-1 are as shown in SEQ ID NOs: 69, 70, 71, 82, 83, and 84 in order, and the CDRs are defined by the Contact Definition Scheme; or
the amino acid sequences of CDR-H1, CDR-H2, CDR-H3, CDR-L1, CDR-L2, and CDR-L3 of the 240B10-1 are as shown in SEQ ID NOs: 72, 73, 63, 78, 79, and 80 in order, and the CDRs are defined by the Abm Definition Scheme;
preferably, the amino acid sequences of CDR-H1, CDR-H2, CDR-H3, CDR-L1, CDR-L2, and CDR-L3 of the 39B4 are as shown in SEQ ID NOs: 117, 118, 119, 133, 134, and 135 in order, and the CDRs are defined by the Kabat Definition Scheme; or
the amino acid sequences of CDR-H1, CDR-H2, CDR-H3, CDR-L1, and CDR-L3 of the 39B4 are as shown in SEQ ID NOs: 120, 121, 122, 136, and 135 in order and the amino acid sequence of CDR-L2 is SAS, and the CDRs are defined by the IMGT Definition Scheme; or
the amino acid sequences of CDR-H1, CDR-H2, CDR-H3, CDR-L1, CDR-L2, and CDR-L3 of the 39B4 are as shown in SEQ ID NOs: 120, 123, 119, 133, 134, and 135 in order, and the CDRs are defined by the Chothia Definition Scheme; or
the amino acid sequences of CDR-H1, CDR-H2, CDR-H3, CDR-L1, CDR-L2, and CDR-L3 of the 39B4 are as shown in SEQ ID NOs: 117, 124, 125, 137, 138, and 139 in order, and the CDRs are defined by the Contact Definition Scheme; or
the amino acid sequences of CDR-H1, CDR-H2, CDR-H3, CDR-L1, CDR-L2, and CDR-L3 of the 39B4 are as shown in SEQ ID NOs: 126, 127, 128, 133, 134, and 135 in order, and the CDRs are defined by the Abm Definition Scheme;
preferably,
m1) the amino acid sequences of CDR-H1, CDR-H2, CDR-H3, CDR-L1, CDR-L2, and CDR-L3 of the B-8 are as shown in SEQ ID NOs: 33, 34, 35, 50, 51, and 52 in order, and the CDRs are defined by the Kabat Definition Scheme; or
m2) the amino acid sequences of CDR-H1, CDR-H2, CDR-H3, CDR-L1, CDR-L2, and CDR-L3 of the B-8 have the following mutations compared to m1): Y33E in SEQ ID NO: 32, and the CDRs are defined by the Kabat Definition Scheme; or
m3) the amino acid sequences of CDR-H1, CDR-H2, CDR-H3, CDR-L1, CDR-L2, and CDR-L3 of the B-8 have the following mutations compared to m1): Y61E in SEQ ID NO: 32, and the CDRs are defined by the Kabat Definition Scheme; or
m4) the amino acid sequences of CDR-H1, CDR-H2, CDR-H3, CDR-L1, CDR-L2, and CDR-L3 of the B-8 have the following mutations compared to m1): F111A in SEQ ID NO: 32, and the CDRs are defined by the Kabat Definition Scheme; or
m5) the amino acid sequences of CDR-H1, CDR-H2, CDR-H3, CDR-L1, CDR-L2, and CDR-L3 of the B-8 have the following mutations compared to m1): Y93E in SEQ ID NO: 49, and the CDRs are defined by the Kabat Definition Scheme; or
m6) the amino acid sequences of CDR-H1, CDR-H2, CDR-H3, CDR-L1, CDR-L2, and CDR-L3 of the B-8 have the following mutations compared to m1): S91R in SEQ ID NO: 49, and the CDRs are defined by the Kabat Definition Scheme; or
m7) the amino acid sequences of CDR-H1, CDR-H2, CDR-H3, CDR-L1, and CDR-L3 of the B-8 are as shown in SEQ ID NOs: 36, 37, 38, 53, and 52 in order, and the amino acid sequence of CDR-L2 is GAS, and the CDRs are defined by the IMGT Definition Scheme; or
m8) the amino acid sequences of CDR-H1, CDR-H2, CDR-H3, CDR-L1, CDR-L2, and CDR-L3 of the B-8 have the following mutations compared to m7): Y33E in SEQ ID NO: 32, and the CDRs are defined by the IMGT Definition Scheme; or
m9) the amino acid sequences of CDR-H1, CDR-H2, CDR-H3, CDR-L1, CDR-L2, and CDR-L3 of the B-8 have the following mutations compared to m7): F111A in SEQ ID NO: 32, and the CDRs are defined by the IMGT Definition Scheme; or
m10) the amino acid sequences of CDR-H1, CDR-H2, CDR-H3, CDR-L1, CDR-L2, and CDR-L3 of the B-8 have the following mutations compared to m7): Y93E in SEQ ID NO: 49, and the CDRs are defined by the IMGT Definition Scheme; or
m11) the amino acid sequences of CDR-H1, CDR-H2, CDR-H3, CDR-L1, CDR-L2, and CDR-L3 of the B-8 have the following mutations compared to m7): S91R in SEQ ID NO: 49, and the CDRs are defined by the IMGT Definition Scheme; or
m12) the amino acid sequences of CDR-H1, CDR-H2, CDR-H3, CDR-L1, CDR-L2, and CDR-L3 of the B-8 are as shown in SEQ ID NOs: 50, 51, 52, 67, 68, and 63 in order, and the CDRs are defined by the Chothia Definition Scheme; or
m13) the amino acid sequences of CDR-H1, CDR-H2, CDR-H3, CDR-L1, CDR-L2, and CDR-L3 of the B-8 have the following mutations compared to m12): F111A in SEQ ID NO: 32, and the CDRs are defined by the Chothia Definition Scheme; or
m14) the amino acid sequences of CDR-H1, CDR-H2, CDR-H3, CDR-L1, CDR-L2, and CDR-L3 of the B-8 have the following mutations compared to m12): Y93E in SEQ ID NO: 49, and the CDRs are defined by the Chothia Definition Scheme; or
m15) the amino acid sequences of CDR-H1, CDR-H2, CDR-H3, CDR-L1, CDR-L2, and CDR-L3 of the B-8 have the following mutations compared to m12): S91R in SEQ ID NO: 49, and the CDRs are defined by the Chothia Definition Scheme; or
m16) the amino acid sequences of CDR-H1, CDR-H2, CDR-H3, CDR-L1, CDR-L2, and CDR-L3 of the B-8 are as shown in SEQ ID NOs: 41, 42, 43, 54, 55, and 56 in order, and the CDRs are defined by the Contact Definition Scheme; or
m17) the amino acid sequences of CDR-H1, CDR-H2, CDR-H3, CDR-L1, CDR-L2, and CDR-L3 of the B-8 have the following mutations compared to m16): Y33E in SEQ ID NO: 32, and the CDRs are defined by the Contact Definition Scheme; or
m18) the amino acid sequences of CDR-H1, CDR-H2, CDR-H3, CDR-L1, CDR-L2, and CDR-L3 of the B-8 have the following mutations compared to m16): F111A in SEQ ID NO: 32, and the CDRs are defined by the Contact Definition Scheme; or
m19) the amino acid sequences of CDR-H1, CDR-H2, CDR-H3, CDR-L1, CDR-L2, and CDR-L3 of the B-8 have the following mutations compared to m16): Y93E in SEQ ID NO: 49, and the CDRs are defined by the Contact Definition Scheme; or
m20) the amino acid sequences of CDR-H1, CDR-H2, CDR-H3, CDR-L1, CDR-L2, and CDR-L3 of the B-8 have the following mutations compared to m16): S91R in SEQ ID NO: 49, and the CDRs are defined by the Contact Definition Scheme; or
m21) the amino acid sequences of CDR-H1, CDR-H2, CDR-H3, CDR-L1, CDR-L2, and CDR-L3 of the B-8 are as shown in SEQ ID NOs: 44, 45, 35, 50, 51, and 52 in order, and the CDRs are defined by the Abm Definition Scheme; or
m22) the amino acid sequences of CDR-H1, CDR-H2, CDR-H3, CDR-L1, CDR-L2, and CDR-L3 of the B-8 have the following mutations compared to m21): Y33E in SEQ ID NO: 32, and the CDRs are defined by the Abm Definition Scheme; or
m23) the amino acid sequences of CDR-H1, CDR-H2, CDR-H3, CDR-L1, CDR-L2, and CDR-L3 of the B-8 have the following mutations compared to m21): F111A in SEQ ID NO: 32, and the CDRs are defined by the Abm Definition Scheme; or
m24) the amino acid sequences of CDR-H1, CDR-H2, CDR-H3, CDR-L1, CDR-L2, and CDR-L3 of the B-8 have the following mutations compared to m21): Y93E in SEQ ID NO: 49, and the CDRs are defined by the Abm Definition Scheme; or
m25) the amino acid sequences of CDR-H1, CDR-H2, CDR-H3, CDR-L1, CDR-L2, and CDR-L3 of the B-8 have the following mutations compared to m21): S91R in SEQ ID NO: 49, and the CDRs are defined by the Abm Definition Scheme;
preferably,
n1) the amino acid sequences of CDR-H1, CDR-H2, CDR-H3, CDR-L1, CDR-L2, and CDR-L3 of the 98G10 are as shown in SEQ ID NOs: 89, 90, 91, 106, 107, and 108 in order, and the CDRs are defined by the Kabat Definition Scheme; or
n2) the amino acid sequences of CDR-H1, CDR-H2, CDR-H3, CDR-L1, CDR-L2, and CDR-L3 of the 98G10 have the following mutations compared to n1): W34H in SEQ ID NO: 88, and the CDRs are defined by the Kabat Definition Scheme; or
n3) the amino acid sequences of CDR-H1, CDR-H2, CDR-H3, CDR-L1, CDR-L2, and CDR-L3 of the 98G10 have the following mutations compared to n1): W34Y in SEQ ID NO: 88, and the CDRs are defined by the Kabat Definition Scheme; or
n4) the amino acid sequences of CDR-H1, CDR-H2, CDR-H3, CDR-L1, CDR-L2, and CDR-L3 of the 98G10 have the following mutations compared to n1): Y112R in SEQ ID NO: 88, and the CDRs are defined by the Kabat Definition Scheme; or
n5) the amino acid sequences of CDR-H1, CDR-H2, CDR-H3, CDR-L1, CDR-L2, and CDR-L3 of the 98G10 have the following mutations compared to n1): Y112S in SEQ ID NO: 88, and the CDRs are defined by the Kabat Definition Scheme; or
n6) the amino acid sequences of CDR-H1, CDR-H2, CDR-H3, CDR-L1, CDR-L2, and CDR-L3 of the 98G10 have the following mutations compared to n1): Y112E in SEQ ID NO: 88, and the CDRs are defined by the Kabat Definition Scheme; or
n7) the amino acid sequences of CDR-H1, CDR-H2, CDR-H3, CDR-L1, CDR-L2, and CDR-L3 of the 98G10 have the following mutations compared to n1): Y112Q in SEQ ID NO: 88, and the CDRs are defined by the Kabat Definition Scheme; or
n8) the amino acid sequences of CDR-H1, CDR-H2, CDR-H3, CDR-L1, CDR-L2, and CDR-L3 of the 98G10 have the following mutations compared to n1): W34R and Y112R in SEQ ID NO: 88, and the CDRs are defined by the Kabat Definition Scheme; or
n9) the amino acid sequences of CDR-H1, CDR-H2, CDR-H3, CDR-L1, CDR-L2, and CDR-L3 of the 98G10 have the following mutations compared to n1): W34D and Y112R in SEQ ID NO: 88, and the CDRs are defined by the Kabat Definition Scheme; or
n10) the amino acid sequences of CDR-H1, CDR-H2, CDR-H3, CDR-L1, CDR-L2, and CDR-L3 of the 98G10 have the following mutations compared to n1): W34H and Y112L in SEQ ID NO: 88, and the CDRs are defined by the Kabat Definition Scheme; or
n11) the amino acid sequences of CDR-H1, CDR-H2, CDR-H3, CDR-L1, CDR-L2, and CDR-L3 of the 98G10 have the following mutations compared to n1): W34T and Y112R in SEQ ID NO: 88, and the CDRs are defined by the Kabat Definition Scheme; or
n12) the amino acid sequences of CDR-H1, CDR-H2, CDR-H3, CDR-L1, CDR-L2, and CDR-L3 of the 98G10 have the following mutations compared to n1): W34H and Y112R in SEQ ID NO: 88, and the CDRs are defined by the Kabat Definition Scheme; or
n13) the amino acid sequences of CDR-H1, CDR-H2, CDR-H3, CDR-L1, CDR-L2, and CDR-L3 of the 98G10 have the following mutations compared to n1): W34H and Y112S in SEQ ID NO: 88, and the CDRs are defined by the Kabat Definition Scheme; or
n14) the amino acid sequences of CDR-H1, CDR-H2, CDR-H3, CDR-L1, CDR-L2, and CDR-L3 of the 98G10 have the following mutations compared to n1): W34K and Y112S in SEQ ID NO: 88, and the CDRs are defined by the Kabat Definition Scheme; or
n15) the amino acid sequences of CDR-H1, CDR-H2, CDR-H3, CDR-L1, CDR-L2, and CDR-L3 of the 98G10 have the following mutations compared to n1): W34D and Y112S in SEQ ID NO: 88, and the CDRs are defined by the Kabat Definition Scheme; or
n16) the amino acid sequences of CDR-H1, CDR-H2, CDR-H3, CDR-L1, CDR-L2, and CDR-L3 of the 98G10 have the following mutations compared to n1): Y93R in SEQ ID NO: 105, and the CDRs are defined by the Kabat Definition Scheme; or
n17) the amino acid sequences of CDR-H1, CDR-H2, CDR-H3, CDR-L1, CDR-L2, and CDR-L3 of the 98G10 have the following mutations compared to n1): Y93S in SEQ ID NO: 105, and the CDRs are defined by the Kabat Definition Scheme; or
n18) the amino acid sequences of CDR-H1, CDR-H2, CDR-H3, CDR-L1, CDR-L2, and CDR-L3 of the 98G10 have the following mutations compared to n1): Y93Q in SEQ ID NO: 105, and the CDRs are defined by the Kabat Definition Scheme; or
n20) the amino acid sequences of CDR-H1, CDR-H2, CDR-H3, CDR-L1, and CDR-L3 of the 98G10 are as shown in SEQ ID NOs: 92, 93, 94, 109, and 108 in order, and the amino acid sequence of CDR-L2 is TAS, and the CDRs are defined by the IMGT Definition Scheme; or
n21) the amino acid sequences of CDR-H1, CDR-H2, CDR-H3, CDR-L1, CDR-L2, and CDR-L3 of the 98G10 have the following mutations compared to n20): W34H in SEQ ID NO: 88, and the CDRs are defined by the IMGT Definition Scheme; or
n22) the amino acid sequences of CDR-H1, CDR-H2, CDR-H3, CDR-L1, CDR-L2, and CDR-L3 of the 98G10 have the following mutations compared to n20): W34Y in SEQ ID NO: 88, and the CDRs are defined by the IMGT Definition Scheme; or
n23) the amino acid sequences of CDR-H1, CDR-H2, CDR-H3, CDR-L1, CDR-L2, and CDR-L3 of the 98G10 have the following mutations compared to n20): Y112R in SEQ ID NO: 88, and the CDRs are defined by the IMGT Definition Scheme; or
n24) the amino acid sequences of CDR-H1, CDR-H2, CDR-H3, CDR-L1, CDR-L2, and CDR-L3 of the 98G10 have the following mutations compared to n20): Y112S in SEQ ID NO: 88, and the CDRs are defined by the IMGT Definition Scheme; or
n25) the amino acid sequences of CDR-H1, CDR-H2, CDR-H3, CDR-L1, CDR-L2, and CDR-L3 of the 98G10 have the following mutations compared to n20): Y112E in SEQ ID NO: 88, and the CDRs are defined by the IMGT Definition Scheme; or
n26) the amino acid sequences of CDR-H1, CDR-H2, CDR-H3, CDR-L1, CDR-L2, and CDR-L3 of the 98G10 have the following mutations compared to n20): Y112Q in SEQ ID NO: 88, and the CDRs are defined by the IMGT Definition Scheme; or
n27) the amino acid sequences of CDR-H1, CDR-H2, CDR-H3, CDR-L1, CDR-L2, and CDR-L3 of the 98G10 have the following mutations compared to n20): W34R and Y112R in SEQ ID NO: 88, and the CDRs are defined by the IMGT Definition Scheme; or
n28) the amino acid sequences of CDR-H1, CDR-H2, CDR-H3, CDR-L1, CDR-L2, and CDR-L3 of the 98G10 have the following mutations compared to n20): W34D and Y112R in SEQ ID NO: 88, and the CDRs are defined by the IMGT Definition Scheme; or
n29) the amino acid sequences of CDR-H1, CDR-H2, CDR-H3, CDR-L1, CDR-L2, and CDR-L3 of the 98G10 have the following mutations compared to n20): W34H and Y112L in SEQ ID NO: 88, and the CDRs are defined by the IMGT Definition Scheme; or
n30) the amino acid sequences of CDR-H1, CDR-H2, CDR-H3, CDR-L1, CDR-L2, and CDR-L3 of the 98G10 have the following mutations compared to n20): W34T and Y112R in SEQ ID NO: 88, and the CDRs are defined by the IMGT Definition Scheme; or
n31) the amino acid sequences of CDR-H1, CDR-H2, CDR-H3, CDR-L1, CDR-L2, and CDR-L3 of the 98G10 have the following mutations compared to n20): W34H and Y112R in SEQ ID NO: 88, and the CDRs are defined by the IMGT Definition Scheme; or
n32) the amino acid sequences of CDR-H1, CDR-H2, CDR-H3, CDR-L1, CDR-L2, and CDR-L3 of the 98G10 have the following mutations compared to n20): W34H and Y112S in SEQ ID NO: 88, and the CDRs are defined by the IMGT Definition Scheme; or
n33) the amino acid sequences of CDR-H1, CDR-H2, CDR-H3, CDR-L1, CDR-L2, and CDR-L3 of the 98G10 have the following mutations compared to n20): W34K and Y112S in SEQ ID NO: 88, and the CDRs are defined by the IMGT Definition Scheme; or
n34) the amino acid sequences of CDR-H1, CDR-H2, CDR-H3, CDR-L1, CDR-L2, and CDR-L3 of the 98G10 have the following mutations compared to n20): W34D and Y112S in SEQ ID NO: 88, and the CDRs are defined by the IMGT Definition Scheme; or
n35) the amino acid sequences of CDR-H1, CDR-H2, CDR-H3, CDR-L1, CDR-L2, and CDR-L3 of the 98G10 have the following mutations compared to n20): Y93R in SEQ ID NO: 105, and the CDRs are defined by the IMGT Definition Scheme; or
n36) the amino acid sequences of CDR-H1, CDR-H2, CDR-H3, CDR-L1, CDR-L2, and CDR-L3 of the 98G10 have the following mutations compared to n20): Y93S in SEQ ID NO: 105, and the CDRs are defined by the IMGT Definition Scheme; or
n37) the amino acid sequences of CDR-H1, CDR-H2, CDR-H3, CDR-L1, CDR-L2, and CDR-L3 of the 98G10 have the following mutations compared to n20): Y93Q in SEQ ID NO: 105, and the CDRs are defined by the IMGT Definition Scheme; or
n39) the amino acid sequences of CDR-H1, CDR-H2, CDR-H3, CDR-L1, CDR-L2, and CDR-L3 of the 98G10 are as shown in SEQ ID NOs: 95, 96, 91, 106, 107, and 108 in order, and the CDRs are defined by the Chothia Definition Scheme; or
n40) the amino acid sequences of CDR-H1, CDR-H2, CDR-H3, CDR-L1, CDR-L2, and CDR-L3 of the 98G10 have the following mutations compared to n39): Y112R in SEQ ID NO: 88, and the CDRs are defined by the Chothia Definition Scheme; or
n41) the amino acid sequences of CDR-H1, CDR-H2, CDR-H3, CDR-L1, CDR-L2, and CDR-L3 of the 98G10 have the following mutations compared to n39): Y112S in SEQ ID NO: 88, and the CDRs are defined by the Chothia Definition Scheme; or
n42) the amino acid sequences of CDR-H1, CDR-H2, CDR-H3, CDR-L1, CDR-L2, and CDR-L3 of the 98G10 have the following mutations compared to n39): Y112E in SEQ ID NO: 88, and the CDRs are defined by the Chothia Definition Scheme; or
n43) the amino acid sequences of CDR-H1, CDR-H2, CDR-H3, CDR-L1, CDR-L2, and CDR-L3 of the 98G10 have the following mutations compared to n39): Y112Q in SEQ ID NO: 88, and the CDRs are defined by the Chothia Definition Scheme; or
n44) the amino acid sequences of CDR-H1, CDR-H2, CDR-H3, CDR-L1, CDR-L2, and CDR-L3 of the 98G10 have the following mutations compared to n39): Y112L in SEQ ID NO: 88, and the CDRs are defined by the Chothia Definition Scheme; or
n45) the amino acid sequences of CDR-H1, CDR-H2, CDR-H3, CDR-L1, CDR-L2, and CDR-L3 of the 98G10 have the following mutations compared to n39): Y93R in SEQ ID NO: 105, and the CDRs are defined by the Chothia Definition Scheme; or
n46) the amino acid sequences of CDR-H1, CDR-H2, CDR-H3, CDR-L1, CDR-L2, and CDR-L3 of the 98G10 have the following mutations compared to n39): Y93S in SEQ ID NO: 105, and the CDRs are defined by the Chothia Definition Scheme; or
n47) the amino acid sequences of CDR-H1, CDR-H2, CDR-H3, CDR-L1, CDR-L2, and CDR-L3 of the 98G10 have the following mutations compared to n1): Y93Q in SEQ ID NO: 105, and the CDRs are defined by the Chothia Definition Scheme; or
n48) the amino acid sequences of CDR-H1, CDR-H2, CDR-H3, CDR-L1, CDR-L2, and CDR-L3 of the 98G10 are as shown in SEQ ID NOs: 97, 98, 99, 110, 111, and 112 in order, and the CDRs are defined by the Contact Definition Scheme; or
n49) the amino acid sequences of CDR-H1, CDR-H2, CDR-H3, CDR-L1, CDR-L2, and CDR-L3 of the 98G10 have the following mutations compared to n48): W34H in SEQ ID NO: 88, and the CDRs are defined by the Contact Definition Scheme; or
n50) the amino acid sequences of CDR-H1, CDR-H2, CDR-H3, CDR-L1, CDR-L2, and CDR-L3 of the 98G10 have the following mutations compared to n48): W34Y in SEQ ID NO: 88, and the CDRs are defined by the Contact Definition Scheme; or
n51) the amino acid sequences of CDR-H1, CDR-H2, CDR-H3, CDR-L1, CDR-L2, and CDR-L3 of the 98G10 have the following mutations compared to n48): Y112R in SEQ ID NO: 88, and the CDRs are defined by the Contact Definition Scheme; or
n52) the amino acid sequences of CDR-H1, CDR-H2, CDR-H3, CDR-L1, CDR-L2, and CDR-L3 of the 98G10 have the following mutations compared to n48): Y112S in SEQ ID NO: 88, and the CDRs are defined by the Contact Definition Scheme; or
n53) the amino acid sequences of CDR-H1, CDR-H2, CDR-H3, CDR-L1, CDR-L2, and CDR-L3 of the 98G10 have the following mutations compared to n48): Y112E in SEQ ID NO: 88, and the CDRs are defined by the Contact Definition Scheme; or
n54) the amino acid sequences of CDR-H1, CDR-H2, CDR-H3, CDR-L1, CDR-L2, and CDR-L3 of the 98G10 have the following mutations compared to n48): Y112Q in SEQ ID NO: 88, and the CDRs are defined by the Contact Definition Scheme; or
n55) the amino acid sequences of CDR-H1, CDR-H2, CDR-H3, CDR-L1, CDR-L2, and CDR-L3 of the 98G10 have the following mutations compared to n48): W34R and Y112R in SEQ ID NO: 88, and the CDRs are defined by the Contact Definition Scheme; or
n56) the amino acid sequences of CDR-H1, CDR-H2, CDR-H3, CDR-L1, CDR-L2, and CDR-L3 of the 98G10 have the following mutations compared to n48): W34D and Y112R in SEQ ID NO: 88, and the CDRs are defined by the Contact Definition Scheme; or
n57) the amino acid sequences of CDR-H1, CDR-H2, CDR-H3, CDR-L1, CDR-L2, and CDR-L3 of the 98G10 have the following mutations compared to n48): W34H and Y112L in SEQ ID NO: 88, and the CDRs are defined by the Contact Definition Scheme; or
n58) the amino acid sequences of CDR-H1, CDR-H2, CDR-H3, CDR-L1, CDR-L2, and CDR-L3 of the 98G10 have the following mutations compared to n48): W34T and Y112R in SEQ ID NO: 88, and the CDRs are defined by the Contact Definition Scheme; or
n59) the amino acid sequences of CDR-H1, CDR-H2, CDR-H3, CDR-L1, CDR-L2, and CDR-L3 of the 98G10 have the following mutations compared to n48): W34H and Y112R in SEQ ID NO: 88, and the CDRs are defined by the Contact Definition Scheme; or
n60) the amino acid sequences of CDR-H1, CDR-H2, CDR-H3, CDR-L1, CDR-L2, and CDR-L3 of the 98G10 have the following mutations compared to n48): W34H and Y112S in SEQ ID NO: 88, and the CDRs are defined by the Contact Definition Scheme; or
n61) the amino acid sequences of CDR-H1, CDR-H2, CDR-H3, CDR-L1, CDR-L2, and CDR-L3 of the 98G10 have the following mutations compared to n48): W34K and Y112S in SEQ ID NO: 88, and the CDRs are defined by the Contact Definition Scheme; or
n62) the amino acid sequences of CDR-H1, CDR-H2, CDR-H3, CDR-L1, CDR-L2, and CDR-L3 of the 98G10 have the following mutations compared to n48): W34D and Y112S in SEQ ID NO: 88, and the CDRs are defined by the Contact Definition Scheme; or
n63) the amino acid sequences of CDR-H1, CDR-H2, CDR-H3, CDR-L1, CDR-L2, and CDR-L3 of the 98G10 have the following mutations compared to n48): Y93R in SEQ ID NO: 105, and the CDRs are defined by the Contact Definition Scheme; or
n64) the amino acid sequences of CDR-H1, CDR-H2, CDR-H3, CDR-L1, CDR-L2, and CDR-L3 of the 98G10 have the following mutations compared to n48): Y93S in SEQ ID NO: 105, and the CDRs are defined by the Contact Definition Scheme; or
n65) the amino acid sequences of CDR-H1, CDR-H2, CDR-H3, CDR-L1, CDR-L2, and CDR-L3 of the 98G10 have the following mutations compared to n48): Y93Q in SEQ ID NO: 105, and the CDRs are defined by the Contact Definition Scheme; or
n66) the amino acid sequences of CDR-H1, CDR-H2, CDR-H3, CDR-L1, CDR-L2, and CDR-L3 of the 98G10 are as shown in SEQ ID NOs: 100, 101, 91, 106, 107, and 108 in order, and the CDRs are defined by the Abm Definition Scheme; or
n67) the amino acid sequences of CDR-H1, CDR-H2, CDR-H3, CDR-L1, CDR-L2, and CDR-L3 of the 98G10 have the following mutations compared to n66): W34H in SEQ ID NO: 88, and the CDRs are defined by the Abm Definition Scheme; or
n68) the amino acid sequences of CDR-H1, CDR-H2, CDR-H3, CDR-L1, CDR-L2, and CDR-L3 of the 98G10 have the following mutations compared to n66): W34Y in SEQ ID NO: 88, and the CDRs are defined by the Abm Definition Scheme; or
n69) the amino acid sequences of CDR-H1, CDR-H2, CDR-H3, CDR-L1, CDR-L2, and CDR-L3 of the 98G10 have the following mutations compared to n66): Y112R in SEQ ID NO: 88, and the CDRs are defined by the Abm Definition Scheme; or
n70) the amino acid sequences of CDR-H1, CDR-H2, CDR-H3, CDR-L1, CDR-L2, and CDR-L3 of the 98G10 have the following mutations compared to n66): Y112S in SEQ ID NO: 88, and the CDRs are defined by the Abm Definition Scheme; or
n71) the amino acid sequences of CDR-H1, CDR-H2, CDR-H3, CDR-L1, CDR-L2, and CDR-L3 of the 98G10 have the following mutations compared to n66): Y112E in SEQ ID NO: 88, and the CDRs are defined by the Abm Definition Scheme; or
n72) the amino acid sequences of CDR-H1, CDR-H2, CDR-H3, CDR-L1, CDR-L2, and CDR-L3 of the 98G10 have the following mutations compared to n66): Y112Q in SEQ ID NO: 88, and the CDRs are defined by the Abm Definition Scheme; or
n73) the amino acid sequences of CDR-H1, CDR-H2, CDR-H3, CDR-L1, CDR-L2, and CDR-L3 of the 98G10 have the following mutations compared to n66): W34R and Y112R in SEQ ID NO: 88, and the CDRs are defined by the Abm Definition Scheme; or
n74) the amino acid sequences of CDR-H1, CDR-H2, CDR-H3, CDR-L1, CDR-L2, and CDR-L3 of the 98G10 have the following mutations compared to n66): W34D and Y112R in SEQ ID NO: 88, and the CDRs are defined by the Abm Definition Scheme; or
n75) the amino acid sequences of CDR-H1, CDR-H2, CDR-H3, CDR-L1, CDR-L2, and CDR-L3 of the 98G10 have the following mutations compared to n66): W34H and Y112L in SEQ ID NO: 88, and the CDRs are defined by the Abm Definition Scheme; or
n76) the amino acid sequences of CDR-H1, CDR-H2, CDR-H3, CDR-L1, CDR-L2, and CDR-L3 of the 98G10 have the following mutations compared to n66): W34T and Y112R in SEQ ID NO: 88, and the CDRs are defined by the Abm Definition Scheme; or
n77) the amino acid sequences of CDR-H1, CDR-H2, CDR-H3, CDR-L1, CDR-L2, and CDR-L3 of the 98G10 have the following mutations compared to n66): W34H and Y112R in SEQ ID NO: 88, and the CDRs are defined by the Abm Definition Scheme; or
n78) the amino acid sequences of CDR-H1, CDR-H2, CDR-H3, CDR-L1, CDR-L2, and CDR-L3 of the 98G10 have the following mutations compared to n66): W34H and Y112S in SEQ ID NO: 88, and the CDRs are defined by the Abm Definition Scheme; or
n79) the amino acid sequences of CDR-H1, CDR-H2, CDR-H3, CDR-L1, CDR-L2, and CDR-L3 of the 98G10 have the following mutations compared to n66): W34K and Y112S in SEQ ID NO: 88, and the CDRs are defined by the Abm Definition Scheme; or
n80) the amino acid sequences of CDR-H1, CDR-H2, CDR-H3, CDR-L1, CDR-L2, and CDR-L3 of the 98G10 have the following mutations compared to n66): W34D and Y112S in SEQ ID NO: 88, and the CDRs are defined by the Abm Definition Scheme; or
n81) the amino acid sequences of CDR-H1, CDR-H2, CDR-H3, CDR-L1, CDR-L2, and CDR-L3 of the 98G10 have the following mutations compared to n66): Y93R in SEQ ID NO: 105, and the CDRs are defined by the Abm Definition Scheme; or
n82) the amino acid sequences of CDR-H1, CDR-H2, CDR-H3, CDR-L1, CDR-L2, and CDR-L3 of the 98G10 have the following mutations compared to n66): Y93S in SEQ ID NO: 105, and the CDRs are defined by the Abm Definition Scheme; or
n83) the amino acid sequences of CDR-H1, CDR-H2, CDR-H3, CDR-L1, CDR-L2, and CDR-L3 of the 98G10 have the following mutations compared to n66): Y93Q in SEQ ID NO: 105, and the CDRs are defined by the Abm Definition Scheme;
preferably, the monoclonal antibody or antigen-binding fragment thereof comprises at least one of a full-length antibody, Fab, Fab', F(ab')2, Fv, scFv, a bispecific antibody, and a multispecific antibody.

4. A recombinant protein, comprising: the antibody or antigen-binding fragment thereof according to any one of claims 1 to 3; and
optionally, a tag sequence for facilitating expression and/or purification.

5. A biological material related to the antibody or antigen-binding fragment thereof according to any one of claims 1 to 3, or the recombinant protein according to claim 4, the biological material comprises at least one of l1) to l16):
l1) a nucleic acid molecule encoding the antibody or antigen-binding fragment thereof according to any one of claims 1 to 3, or the recombinant protein according to claim 4;
l2) an expression cassette comprising the nucleic acid molecule of l1);
l3) a vector comprising the nucleic acid molecule of l1);
l4) a vector comprising the expression cassette of l2);
l5) a transgenic cell line comprising the nucleic acid molecule of l1);
l6) a transgenic cell line comprising the expression cassette of l2);
l7) a transgenic cell line comprising the vector of l3);
l8) a transgenic cell line comprising the vector of l4);
l9) a microorganism comprising the nucleic acid molecule of l1);
l10) a microorganism comprising the expression cassette of l2);
l11) a microorganism comprising the vector of l3);
l12) a microorganism comprising the vector of l4);
l13) a virus comprising the nucleic acid molecule of l1);
l14) a virus comprising the expression cassette of l2);
l15) a virus comprising the vector of l3);
l16) a virus comprising the vector of l4).

6. A conjugate, comprising: at least one of the antibody or antigen-binding fragment thereof according to any one of claims 1 to 3 and the recombinant protein according to claim 4;
and a conjugated moiety, the conjugated moiety comprises at least one of a detectable marker, a drug, a toxin, and a cytokine;
preferably, the detectable marker is selected from a microparticle, a fluorescent label, a redox molecular label, a chemiluminescent label, a radioactive label, an enzyme label, a ligand label, or any combination thereof.

7. Use of at least one of (1) to (4) in the preparation of a product;
(1) the antibody or antigen-binding fragment thereof according to any one of claims 1 to 3;
(2) the recombinant protein according to claim 4;
(3) the biological material according to claim 5;
(4) the conjugate according to claim 6;
the product comprises at least one of a drug, a reagent, a test strip, a test plate, a kit, a test chip, an adsorbent, and a medical device;
preferably, the drug may prevent and treat a disease or condition related to abnormal high level and/or activity of NMI;
preferably, the reagent, test strip, test plate, test chip or kit has at least one of the functions w1) to w3):
w1) detecting the presence or level of NMI protein in a sample;
w2) diagnosing, auxiliary diagnosing, or prognostic evaluating a diseases or condition related to abnormal high level and/or activity of NMI;
w3) drug screening, the drug is used for preventing and treating a disease or condition related to abnormal high level and/or activity of NMI;
preferably, the reagent, test strip, test plate, test chip or kit is used for one or more detection methods selected from the following groups: a radio-labeled immunoassay, enzyme immunoassay, fluorescence immunoassay, luminescence immunoassay, Western Blot method, physicochemical method, and biochip method;
preferably, a test sample of the reagent, test strip, test plate, test chip or kit is selected from at least one of body fluids, tissues, cells, and excreta of a subject to be tested;
preferably, the adsorbent or the medical device is used for removing NMI protein from body fluids;
preferably, the disease or condition related to abnormal high level and/or activity of NMI comprises at least one of inflammation, infection, septicemia, organ damage, and autoimmune disease.

8. A product, comprising at least one of x1) to x3):
x1) the antibody or antigen-binding fragment thereof according to any one of claims 1 to 3;
x2) the recombinant protein according to claim 4;
x3) the conjugate according to claim 6;
the product comprises at least one of a reagent, a test strip, a test plate, a kit, a test chip, an adsorbent, and a medical device;
preferably, the reagent, test strip, test plate, test chip or kit has at least one of the functions w1) to w3):
w1) detecting the presence or level of NMI protein in a sample;
w2) diagnosing, auxiliary diagnosing, or prognostic evaluating a diseases or condition related to abnormal high level and/or activity of NMI;
w3) drug screening, the drug is used for preventing and treating a disease or condition related to abnormal high level and/or activity of NMI;
preferably, the reagent, test strip, test plate, test chip or kit is used for one or more detection methods selected from the following groups: a radio-labeled immunoassay, enzyme immunoassay, fluorescence immunoassay, luminescence immunoassay, Western Blot method, physicochemical method, and biochip method;
preferably, a test sample of the reagent, test strip, test plate, test chip or kit is selected from at least one of body fluids, tissues, cells, and excreta of a subject to be tested;
preferably, the adsorbent or the medical device is used for removing NMI protein from body fluids;
preferably, the disease or condition related to abnormal high level and/or activity of NMI comprises at least one of inflammation, infection, septicemia, organ damage, and autoimmune disease.

9. A double-antibody sandwich ELISA kit, comprising: a coating antibody and a detection antibody, at least one of the coating antibody and the detection antibody comprises the antibody or antigen-binding fragment thereof according to any one of claims 1 to 3.

10. The double-antibody sandwich ELISA kit according to claim 9, **characterized in that**:
the coating antibody is bound to a solid phase carrier;
preferably, the solid phase carrier comprises at least one of magnetic particles, latex particles and a microplate;
preferably, the detection antibody is labeled with a detectable marker;
preferably, the detectable marker is selected from at least one of an enzyme label, and a ligand label;
preferably, the double-antibody sandwich ELISA kit further comprises an enzyme-labeled receptor when the detectable marker is selected from a ligand label;
preferably, the coating antibody and the detection antibody are selected from any two of the antibodies or antigen-binding fragments thereof according to any one of claims 1 to 3;
preferably, the coating antibody comprises B-8 and the detection antibody comprises 94C4; or the coating antibody comprises 94C4 and the detection antibody comprises B-8;
preferably, the double-antibody sandwich ELISA kit further comprises at least one of a sample diluent, a washing solution, a blocking solution, a chromogenic solution, a stop solution, and a calibrator.

11. A chemiluminescent assay kit, comprising a coating antibody and a detection antibody, at least one of the coating antibody and the detection antibody comprises the antibody or antigen-binding fragment thereof according to any one of claims 1 to 3.

12. The chemiluminescent assay kit according to claim 11, **characterized in that**:
the coating antibody is bound to a solid phase carrier;
preferably, the solid phase carrier comprises at least one of magnetic particles, latex particles and a microplate;
preferably, the detection antibody is labeled with a detectable marker;
preferably, the detectable marker is selected from a chemiluminescent label;
preferably, the coating antibody and the detection antibody are selected from any two of the antibodies or antigen-binding fragments thereof according to any one of claims 1 to 3;
preferably, the coating antibody comprises B-8 and the detection antibody comprises 98G10; or the coating antibody comprises 98G10 and the detection antibody comprises B-8;
preferably, the chemiluminescent assay kit further comprises at least one of a reaction buffer, an NMI calibrator and a luminescent substrate.

13. An immunochromatographic test strip, comprising a base plate; and a sample pad, a marker pad, a chromatographic membrane and an absorbent pad that are partially overlapping and adhered to the base plate; wherein the marker pad is coated with a marker-labeled anti-NMI antibody I, the chromatographic membrane is provided with a test line, the test line is coated with an anti-NMI antibody II, at least one of the anti-NMI antibody I and the anti-NMI antibody II comprises the antibody or antigen-binding fragment thereof according to any one of claims 1 to 3.

14. The immunochromatographic test strip according to claim 13, **characterized in that**:
the anti-NMI antibody I and the anti-NMI antibody II are selected from any two of the antibodies or antigen-binding fragments thereof according to any one of claims 1 to 3;
preferably, the anti-NMI antibody I comprises B-8 and the anti-NMI antibody II comprises 94C4; or the anti-NMI antibody I comprises 94C4 and the anti-NMI antibody II comprises B-8;
preferably, the marker is colloidal gold;
preferably, the chromatographic membrane is also provided with a quality control line.

15. An adsorbent, comprising a carrier matrix and the antibody or antigen-binding fragment thereof according to any one of claims 1 to 3.

16. The adsorbent according to claim 15, **characterized in that**:
the carrier matrix is selected from at least one of agarose gel particles, cellulose gel particles, dextran gel particles, magnetic particles, silica gel particles, activated carbon, resin particles, Protein A agarose particles, and Protein G agarose particles;
preferably, the antibody or antigen-binding fragment thereof is attached to the carrier matrix.

17. A medical device, comprising at least one of the antibody or antigen-binding fragment thereof according to any one of claims 1 to 3 and the adsorbent according to any one of claims 15 to 16.

18. A drug, comprising at least one of y1) to y4):
y1) the antibody or antigen-binding fragment thereof according to any one of claims 1 to 3;
y2) the recombinant protein according to claim 4;
y3) the biological material according to claim 5;
y4) the conjugate according to claim 6;
preferably, the drug further comprises a pharmaceutically acceptable carrier.
